(19) Europäisches Patentamt
European Patent Office
Office européen des brevets

(11) EP 1 083 228 A1

(12) **EUROPEAN PATENT APPLICATION**

(43) Date of publication:
**14.03.2001 Bulletin 2001/11**

(51) Int Cl.[7]: **C12N 15/86**, C12N 5/10, C12N 7/01, A61K 48/00

(21) Application number: **00203121.9**

(22) Date of filing: **11.09.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **10.09.1999 EP 99202966**
**13.09.1999 US 153641 P**

(71) Applicant: **Introgene B.V.**
**2333 AL Leiden (NL)**

(72) Inventors:
- **Schouten, Govert Johan**
  **2321 AR Leiden (NL)**
- **Vogels, Ronald**
  **3461 HW Linschoten (NL)**
- **Opstelten, Dirk Jan Elbertus**
  **2352 VC Leiderdorp (NL)**

(74) Representative: **Prins, Adrianus Willem et al**
**Vereenigde,**
**Nieuwe Parklaan 97**
**2587 BN Den Haag (NL)**

(54) **Modified adenoviral vectors for use in gene therapy**

(57) The invention provides a method for producing a recombinant adenovirus-like gene delivery vehicle having reduced expression of adenoviral E2B and/or E4 gene products in a target cell for gene therapy, comprising generating a recombinant adenoviral vector lacking E1A and preferably E1B sequences, but having at least the E2B and/or E4 sequences encoding products essential for adenoviral replication, wherein said E2B and/or E4 sequences have been modified to lead to a reduced expression and/or induced expression of at least one of said essential products. Said recombinant adenoviral vector and/or recombinant adenovirus-like gene delivery vehicle, which are also provided herein, are for instance suitable for *in vivo* production of a gene product in a cell, and for the preparation of a medicament or vaccine.

A vector and/or gene delivery vehicle of the invention has a diminished antigenicity and is safer than vectors/gene delivery vehicles of the prior art. Furthermore, expression of a nucleic acid of interest delivered to a host through a vector of the invention is prolonged compared to adenoviral vectors of the art.

EP 1 083 228 A1

## Description

The present invention relates to the field of human gene therapy, in particular to gene therapy vehicles with reduced expression of viral genes, more specifically with a reduced immunity. The invention provides novel expression vectors and complementing cell lines, including means and methods to produce such vectors and cell lines, and applications of such vectors, cell lines and methods in human gene therapy protocols.
The development of human gene therapy for the treatment of inherited and acquired disorders requires gene transfer vectors capable of safe and effective delivery and expression of therapeutic genes into target cells. The actual method to introduce and express the genetic material into the target cells of a patient is a key component in every gene therapy protocol. Several different gene transfer systems are currently being employed to introduce therapeutic genes into somatic cells.

Non-viral gene systems for *in vivo* delivery into target cells include a variety of DNA-mediated methods, like direct injection of naked DNA and particle bombardment. To overcome the limitations of these DNA-based delivery methods (low transfer efficiency, and cell toxicity), alternative systems have been developed utilizing the entrapment of the DNA in vesicles (like liposomes), or binding of the DNA to synthetic conjugates (like e.g. transferrin-polylysine). Such conjugate-DNA complexes can be delivered via receptor-mediated endocytosis.

Viral gene-transfer systems are based on the natural capability of viruses to deliver their genes in mammalian cells. The high gene-transfer efficiency of viruses has led to the development of viral vectors in which part of the viral genome has been replaced by a transgene to be introduced into eukaryotic cells. The most commonly used viral systems are based on retroviruses, adenoviruses (Ad) and adeno-associated viruses. Each of these viral delivery systems has its own characteristics in terms of efficiency of gene delivery, integration capability, maximum insert size of the recombinant (recombinant) gene, vector yields, stability of expression, etc. Such characteristics may determine the suitability of a certain delivery system for a specific gene therapy protocol (Bout, 1997). Retroviruses and adeno-associated viruses have been a focus for development because of their capacity to stably integrate DNA sequences into chromosomes of the target cells. The advantage of adenoviruses is their ability to mediate efficient expression of the transgene in a variety of cells, including post-mitotic and/or non-dividing cells, as well as the ease with which these viruses can be propagated and purified to very high titers.

Inherent to all gene transfer methods is the presentation of foreign antigenic material on the target cell, derived from the vehicle and/or from the transgene encoded product. The presentation of foreign antigens usually elicits a response of the immune system; immune responses directed against the gene therapy vehicle and/or the transgene product may lead to inflammation, elimination of the transduced cells, and difficulties with re-administration of the vector due to neutralizing activity against the vehicle. As a consequence, the clinical application of gene therapy vectors can be impeded by the potent host immune response to the vector, which limits the duration of its effects. Immune effectors have been identified as cytotoxic T lymphocytes (CTLs), which destroy vector-transduced cells, as well as B cells, which secrete neutralizing antibodies that can block repeated gene transfer.

CTLs continuously monitor the cells and tissues of the body in search of cells synthesizing foreign or abnormal proteins. The recognition of virus-infected cells by CTLs requires fragments (i.e. peptides) of foreign antigens that are presented at the cell surface in association with class I molecules of the major histocompatibility complex (MHC; for a recent review see Ploegh et al., 1998). The majority of these peptides are generated in the cytosol of virus-infected cells by degradation of poly-ubiquitinated viral proteins. The resulting viral peptides are transported from the cytosol to the endoplasmic reticulum (ER), through the action of the ATP dependent transporter for antigen presentation (TAP) complex. In the ER, MHC class I heavy chains assemble with β2 microglobulin and peptide into stable hetero-trimeric MHC class I complexes that are transported via the secretory route to the plasma membrane. Their expression at the cell surface enables CTLs to play their decisive role in the antiviral defense.

In the first generation of adenoviral vectors for gene therapy, the E1 region was replaced by foreign genetic information, e.g., the therapeutic gene. The absence of E1 renders the recombinant virus replication defective. Because E1 has been reported to trigger the transcription of other Ad genes, it was previously thought that E1-deleted vectors would not express any Ad genes. However, it has been shown by others and us that early (e.g. E2A, E2B and E4) and late (e.g. fiber, hexon and penton-base) genes are still residually expressed from such vectors. This residual Ad gene expression is due to background replication of the recombinant viral genome and the background activity of the promoters driving the transcription of the respective Ad genes (Yang et al., 1994; Lusky et al., 1998). This means that the delivery of a therapeutic gene into target cells using E1-deleted Ad vectors results in expression of the therapeutic gene as well as of the viral genes. Eventually, this will lead to the presentation of viral peptides by MHC class I complexes followed by a cytotoxic immune response against the transduced cells. It has been shown that CTLs directed against the transgene product as well as against the Ad gene products are activated following administration of the vector into immune-competent animals (Song et al., 1997; Yang et al., 1996). The activated CTLs subsequently eradicate the transduced cells from the recipient.

In order to reduce the residual expression of viral genes from recombinant Ad vectors, we have generated Ad vectors that are deleted for the E1 and E2A region. To complement the deletions of E1 and E2A, we have generated an E1 + E2A complementing cell line for the manufacturing of high titer E1/E2A deleted recombinant Ad vector batches, as described herein. Due to the toxicity of the E2A encoded DNA binding protein (DBP), it is difficult to generate a cell line that constitutively expresses E2A. Therefore, we have made use of a mutant E2A gene, derived from H5ts125, that encodes a temperature sensitive (ts) DBP (van der Vliet, 1975). The tsDBP is functionally active at the permissive temperature (32°C), whereas it is nonactive at the nonpermissive temperature (39°). In addition, tsDBP is not toxic at the nonpermissive temperature. Thus, we established a new cell line, designated PER/E2A, that constitutively expresses high levels of the tsE2A gene. This cell line can easily be cultured at the nonpermissive temperature. When functionally active DBP is needed, e.g., for replication of E2A-deleted Ad vectors, the cells can be incubated at the permissive temperature. The PER/E2A cell line and the E1/E2A deleted vectors were designed such that overlap between the Ad sequences in the cell line, i.e. E1 and E2A coding adenoviral sequences, and sequences in the recombinant Ad vector was excluded, at least to the extent that may lead to homologous recombination between vector DNA and adenoviral sequences present in the complementing cell line, which could lead to the formation of reverted viruses that have recaptured the E1 and/or E2A genes.

The deletion of the E2A gene eliminated the residual expression of E2A and the expression of Ad late genes, e.g., penton-base and fiber (Figure 1A). However, these vectors still expressed significant amounts of the E4, e.g. E4-orf6, and E2B genes, e.g., pTP (Figure 1B). Transcription of E4 may even be up regulated in the absence of DBP since the E4 promoter is a natural target for repression by DBP (Blanton and Carter, 1979; Nevins and Winkler, 1980; Chang and Shenk, 1990). Hence, cells infected by E1/E2A deleted vectors still produce and present non-self antigens, when delivered to humans and may be eradicated from the recipient by the immune system. In order to solve such problems the present invention provides modifications to E2B and/or E4 regions or regions controlling the E2B and/or E4 regions in adenoviral vectors and/or in the complementing cell lines therefor.;

E4 constitutes approximately 10% of the total length of the Ad genome. Several differentially spliced mRNAs are synthesized from the E4 region during infection and are predicted to code for seven different polypeptides, six of which have been identified in infected cells.

Genetic studies have shown that E4 encoded proteins have an important function in virus growth in cultured cells since mutant viruses that lack the entire E4 region have a severe defect in replication (Weinberg and Ketner, 1986; Huang and Hearing, 1989). Such E4 lacking viruses display defects in viral DNA replication, viral late mRNA accumulation, viral late protein synthesis, and the shut-off of host cell protein synthesis. Although the exact function of all individual E4-encoded polypeptides has not been defined to date, mutagenesis of individual open reading frames (orfs) has shown that multiple products encoded by E4 are functionally compensatory. Thus, it was found that either the E4-orf3 or the E4-orf6 product is prerequisite for virus replication in cultured cells whereas the other E4 products are dispensable (Huang and Hearing, 1989).

E4-orf3 as well as E4-orf6 encoded proteins are (independently) involved in post-transcriptional processes that increase viral late protein synthesis. They do so by facilitating the cytoplasmic accumulation of the mRNAs encoding these proteins (Sandler and Ketner, 1991). Moreover, they maintain the nuclear stability of unprocessed pre-mRNAs transcribed from the major late promoter, presumably by affecting the splicing of late RNAs. This leads to an expansion of the pool of late RNAs available for maturation and transport to the cytoplasm. In addition, the E4-orf6 encoded 34 kDa protein forms a complex with the E1B 55 kDa protein that selectively increases the rate of export of viral late mRNAs from the nucleus. The E4orf6-34 kDa/E1B-55 kDa complex is located in so called viral inclusion bodies, the region where viral DNA replication, viral late gene transcription and RNA processing occur (Pombo et al., 1994). Finally, both the E4-orf6 and E4-orf3 encoded proteins are required for Ad DNA synthesis.

Proteins encoded by E4 also interact with cellular proteins and antagonize cellular processes. For example, E4 protein products are involved in controlling the cellular transcription factor E2F as well as the phosphorylation of cellular (and viral) proteins. Moreover, both E4-orf1 and E4-orf6 products have oncogenic potential. The E4-orf6 protein, either alone or in a complex with the E1B-55-kDa protein, binds the cellular protein p53 thereby blocking its potential to activate the transcription of tumor-suppressing genes (Dobner et al., 1996; Moore et al., 1996). As a result, the E4-orf6 protein may prevent the induction of apoptosis by p53.

Taken together, disrupting or deleting the E4 function from recombinant Ad vectors may further reduce viral genome replication and expression of early and late viral genes. This may, in turn, diminish the antigenicity of the vectors. In addition, vectors from which the E4 function is impaired can be considered safer than E4-containing vectors because they do not express E4-encoded proteins that can induce oncogenesis or which are toxic to the host cell.

It is important to note, however, that the impairment of the E4 function within the Ad vector backbone can influence the activity of the promoter that drives the expression of the transgene. For example, it has been reported that the activity of either the cytomegalovirus (CMV) promoter or the Rous sarcoma virus (RSV) promoter is down-regulated when the E4 region is largely or completely deleted from the Ad vector (Armentano et al., 1997; Brough et al., 1997; Dedieu et al., 1997). Thus, although the transduced cells and the vector DNA may persist, the expression of the transgene from the CMV or the RSV promoter is only transient when an E4-specific factor(s) is absent. The underlying mechanism causing the silencing of these promoters is unclear, but a recent study point to the requirement of E4-orf3 for long-term expression from the CMV promoter (Armentano, 1999). The same study also showed that a truncated form of the CMV promoter could persistently drive expression in the absence of E4. It is yet unknown whether promoters from genes of mammals (mammalian promoters) are influenced by the E4 region when present in the context of a recombinant Ad vector. It is therefore highly valuable to determine whether mammalian promoters, such as the elongation factor-1 alpha (EF-1$\alpha$) or the ubiquitin-C (UbC) promoter, can mediate persistent expression of the transgene from an Ad vector that does not produce E4.
The early region 2 (E2) of Ad encodes three gene products that are required for Ad DNA replication. E2 can be divided into two transcription units, E2A and E2B. Both E2A and E2B are transcribed from the same promoter region, designated the E2 promoter. At early time points after infection, E2 is transcribed from the E2-early promoter located at 76 map units. At intermediate times after infection, the transcription switches to another promoter, the E2-late promoter located at 72 map units (reviewed by Swaminathan and Thimmapaya, 1995). Transcription initiation from the E2-early promoter is strongly induced by the polypeptides encoded by E1, which is mediated via E2F and jun/ATF transcription factors. Transcription initiation from the E2-late promoter is less well understood. This promoter consists of a TATA-like sequence, SP-1 binding sites and a CAAT box. The E2-late promoter is not regulated by proteins of the E1A region.

The E2A region encodes the 72kDa single stranded DNA binding protein (DBP). It plays a pivotal role in both the initiation and elongation of Ad DNA replication (reviewed by van der Vliet, 1995 and references therein). Briefly, DBP is thought to increase the affinity of the host cell nuclear factor 1 (NF1) to the auxiliary region of the inverted terminal repeat (ITR) of the Ad genome. This, in turn, facilitates binding of the pTP/pol complex (see below) to the core region of the ITR. Secondly, DBP stimulates the NF1 dependent formation of pTP-dCMP, which forms the DNA-replication initiation complex. Thirdly, DBP facilitates Ad DNA template unwinding by destabilization of the DNA helix in the replication fork. Following unwinding, DBP binds cooperatively to the single stranded Ad DNA in a non-sequence specific manner, thereby forming a protein chain at the displaced strand. This may be the mechanism by which DBP destabilizes the duplex DNA ahead of the replication fork. Fourthly, DBP prevents intramolecular renaturation of the ITRs of the displaced DNA strand, whereas it facilitates intermolecular renaturation of two displaced strands of opposite polarity, originating from initiation of DNA replication at different molecular ends. Finally, DBP has a positive regulatory effect on the activity of the major late promoter, the promoter that drives expression of the Ad late genes (Chang and Shenk, 1990).

Transcription of E2 gives rise to two E2B-specific mRNAs that are derived from differential splicing. They encode two polypeptides, the 80 kDa pTP and the 140 kDa DNA polymerase (pol) that form a stable heterodimer. The pTP/pol complex is recruited to the ITR by NF1, where it binds to the core region and forms the pre-initiation complex. Next, the ITR partially unwinds and the replication-initiation reaction is primed, i.e. a pTP-dCMP coupling takes place that is catalyzed by pol. This is followed by synthesis of a pTP-CAT intermediate at G4-T5-A6 of the ITR. The pTP-trinucleotide intermediate jumps back to the G1-T2-A3 position of the ITR. Elongation of the Ad DNA synthesis starts after dissociation of DNA-bound pTP from pol. The elongation reaction is enhanced by DBP, as discussed above, and progeny DNA accumulates. Finally, the DNA-bound pTP as well as free pTP are proteolytically cleaved into TP by the Ad protease late in the infection cycle. The proteolytic maturation of free pTP destroys its capacity to function as a primer for DNA replication and thus the initiation of new DNA replication cycles is stopped. Mature TP that is covalently bound to newly synthesized Ad DNA protects the DNA from exonuclease activity and is involved in the attachment of the viral DNA to the nuclear matrix. Finally, DNA-bound TP stabilizes the incoming pTP/pol heterodimer at the core region of the ITR during the initiation of DNA replication in the next lytic infection cycle.

The deletion approach to knock out the viral gene functions from the recombinant Ad vector is difficult to apply to E2B and E4 for the following reasons. First, since both E4 and E2B encoded proteins are pivotal for the lytic infection cycle of Ad, the production of recombinant Ad vectors that lack these regions would require a complementing cell line that is equipped with expression vectors that ectopically express, in addition to E1 and/or E2A, at least E4-orf6 and/or E4-orf3 as well as pTP and pol. Although the generation of such cell lines may be possible, these cell lines will normally be unstable. In addition, some of these viral gene products, e.g., E4orf6 34 kDa and E4orf3 11 kDa, are considered to be highly toxic when constitutively synthesized in a complementing cell, meaning that the expression of the corresponding genes needs to be regulated tightly. This generally leads to complementing cells that do not support manufacturing of high titer batches of the respective recombinant Ad vectors (Lusky et al., 1998 and references therein) .

Secondly, the approach to delete viral gene functions from the recombinant Ad vectors is difficult to apply to E2B. Although it is possible to generate cell lines that constitutively express pTP and pol (Amalfitano et al., 1997), it is not possible to entirely delete the pTP and pol coding sequences from the vector genome. This is due to the fact that other viral regulatory elements and genes are present in this area of the viral genome. This area includes the second and third tripartite leader sequences, the i-leader, portions of the major-late promoter intronic sequences and the IVa2 gene (Amalfitano et al., 1997). As a consequence, only partial deletions in the pTP and pol coding sequences can be made. This yields a substantial sequence overlap between the remaining sequences in the recombinant adenoviral vector and the pTP and pol sequences in the complementing cell line. This, in turn, can lead to homologous recombination and reversion of the pTP and pol deleted phenotype during virus replication. Therefore, the deletion approach is not suitable for the production of a homogeneous population of E2B-crippled Ad vectors.

Thirdly, deletion of the entire E4 transcription unit is feasible but it seems to impair the expression of the L5 region encoding the fiber protein (Brough et al., 1996). This causes a severe reduction in virus yields when an E4 deleted vector is grown in E4 complementing cells. This defect can be partially solved by introducing a spacer sequence, e.g., an expression cassette, in place of the deleted E4 sequences (GenVec, US patent 5,851,806).

Fourthly, it should be noted that recombinant Ad vectors that are deleted of some or even all viral coding sequences (so-called *gutless* or *minimal* vectors) can only be propagated using a helper Ad that supports replication and packaging of such vectors by providing all the necessary proteins *in trans* (Fisher et al., 1996; Hardy et al., 1997; Kochanek et al., 1996; Kumar-Singh and Chamberlain, 1996). Generally, the packaging sequence of such a helper virus is flanked by lox sites which are targets for the CRE-recombinase. Hence, the packaging signal is excised from the helper vector when the packaging cells (used for production and packaging of the gutted vectors) express the CRE-recombinase, thereby preventing the packaging of the helper vector. This system, however, has serious limitations with respect to the efficiency of excision of the packaging signal and to the low yields of the gutted vector. Therefore, the crude vector batches produced in this way are contaminated with helper virus that is formed due to inefficient excision of the packaging signal. Moreover, the removal of this contaminating helper virus is laborious and incomplete, which means that it is practically impossible to obtain a helper virus free vector batch.

A final problem is that extensive deletion of the coding sequences from the Ad genome renders the virus unstable and leads to rearrangement of the viral DNA during replication (Parks and Graham, 1997). This is presumably due to fact that genomes smaller than 75% of the wild-type genome are inefficiently packaged into virus particles. To circumvent this problem, the deletion of large parts of the viral genome has to be compensated by addition of heterologous sequences to increase the net size of the vector genome. Such an approach has the intrinsic risk of introduction of unintentional, and perhaps yet unknown cryptic transcriptional signals and open reading frames within the vector backbone and increases the risk of (homologous) recombination with the cellular DNA during replication.

The present invention now provides a method for producing a recombinant adenovirus-like gene delivery vehicle having reduced expression of adenoviral E2B and/or E4 gene products in a target cell for gene therapy, comprising generating a recombinant adenoviral vector lacking E1A and preferably E1B sequences, but having at least the E2B and/or E4 sequences encoding products essential for adenoviral replication, wherein said E2B and/or E4 sequences have been modified to lead to a reduced expression and/or induced expression of at least one of said essential products. Modification in this respect means any change at the nucleic acid level that diminishes the expression and/or the function of any of the gene products of the relevant genes, be it by mutation in a coding sequence or mutation in a regulatory sequence, whereby the expression is not completely or permanently deleted. It also means replacing a regulatory sequence of any of these genes by one or more inducible regulatory sequences, be it repressors, transactivation sites, inducible promoters or any other inducible sequence, whereby non-leaky ones are preferred. Combinations may be made to avoid leakage in the non-activated or repressed state. In a preferred embodiment at least open reading frame 1, 3 or 6 of E4 is so modified. An additional advantage of at least some E4 modifications according to the invention, is that concurrently E2B expression is at least in part reduced in said gene delivery vehicle. Thus expression of E2B can be reduced directly by modifying E2B sequences or indirect by modifying E4 sequences or by both type of modifications. Similarly, expression of E4 gene products can be reduced by modification of E4 sequences or by modification of E2B sequences or both. As disclosed herein, open reading frame 1, 3 or 6 of E4 are sequences that are essential for replication and/or sequences that lead to toxicity for target cells and/or complementing cells. Therefor down regulation or induction of such sequences is highly desired. Attenuation is preferably achieved through at least one mutation in at least an E2B and/or E4 promoter. Thus the invention also provides a method as disclosed herein before, wherein said vector further comprises an E2B and/or an E4 promoter, wherein said E2B and/or E4 promoter are attenuated through a mutation therein. In the alternative or in combination with the above the invention provides in one embodiment a method according as disclosed herein before, wherein E2B and/or E4 is placed under control of at least one, preferably synthetic inducible promotor and/or repressor. Suitable inducible promoters are well known in the art. A couple of suitable and preferred ones are disclosed herein in the detailed description. Highly preferred inducible promoters are the ones that are described as synthetic, comprising e.g. an artificial TATA-box and a sequence capable of being recognized by a prokaryotic or similar transactivation signal. Typically a complementation cell would then be able to provide said signal through e.g. an expression cassette introduced therein.
As described below it is preferred that the vector also lacks a functional E2A region, which can be elegantly provided by a complementing cell, especially in the form of a temperature sensitive variant of E2A.
A function of the E4 34 kDa protein can also be attenuated by inhibiting the binding to E1B 55kD. Thus the invention also provides a method according wherein said vector lacks a sequence encoding E1B 55kD protein capable of binding an E4 34 kDa gene product.
In order to produce a gene delivery vehicle according to the invention the vectors according to the invention are propagated in complemanting cells. Thus the invention also provides a method as disclosed above, further comprising transducing a complementing cell with said recombinant adenoviral vector wherein said complementing cell provides all functions and/or elements essential for replication of said recombinant adenoviral vector, which are lacking in the genome of said vector. This is the normal concept for making gene delivery vehicles, except that it now has the advantages as disclosed herein by modification of E2B and/or E4 expression. A gene delivery vehicle according to the invention is defined as any viral particle derived from an adenovirus, a chimaeric adenovirus or comprising adenoviral elements, capable of infecting cells and delivering a gene there to. A chimaeric adenovirus may be a chimaera of two or more different adenoviruses, manipulated to give good infection and yet low antigenicity, etc. It may also be a chimaera of adenovirus with another virus such as AAV or a retrovirus, in order to be able to integrate a nucleic acid of interest into a host cell genome. It may even be only a fiber or an adenoviral receptor recognising part of an adenovirus coupled to another virus or non viral vehicle comprising the vector.

The invention also provides a method wherein said complementing cell further comprises all necessary functions and/or elements essential for producing a recombinant adenovirus-like gene delivery vehicle comprising said recombinant adenoviral vector. Here the function of the complementing cell goes beyond replication of the vector and typically includes packaging of the vectors according to the invention, which should then typically possess a functional packaging signal.

As stated herein before, the complementing cell according to the invention preferably also comprises the capability to provide the induction of the E4 and/or E2B products. Thus the invention provides a method wherein said cell further comprises an expression cassette encoding a proteinaceous substance capable of transactivating the inducible (synthetic) promoter on the vector, preferably a method wherein said proteinaceous substance comprises a DNA binding domain from a prokaryote or a lower eukaryote and/or a transactivator domain. More details are given in the detailed description. To avoid problems associated with the production of replication competent adenoviruses and/or the production of transforming activity in the preparation of gene delivery vehicles it is preferred to apply a method wherein said recombinant vector and said complementing cell have no sequence-overlap that leads to homologous recombination resulting in replication competent adenovirus and/or recombinant adenovirus comprising E1 sequences.

The invention also provides the vectors obtainable by methods as disclosed herein. In one embodiment the invention thus provides a recombinant adenoviral vector lacking E1A and preferably E1B sequences, but having at least the E2B and/or E4 sequences encoding products essential for adenoviral replication, wherein said E2B and/or E4 sequences have been modified to lead to a reduced expression and/or induced expression of at least one of said essential products, said vector being obtainable as an intermediate in a method as disclosed above. Also provided are the adenovirus-like gene delivery vehicles having reduced expression of adenoviral E2B and/or E4 gene products in a target cell for gene therapy, obtainable by a method as described herein. Of course such a vector and/or vehicle preferably comprises a therapeutic sequence such as a gene encoding a therapeutic protein, an anti-sense sequence, etc. Such a vector can subsequently be used to introduce the therapeutic protein, anti-sense sequence etc. in cells of a patient, for example (but not limited to) to correct a certain inherited or acquired disorder or for vaccination purposes.

In gene therapy settings one advantage of a recombinant adenoviral vector of the invention is that expression of a nucleic acid interest delivered to a host through said vector is prolonged compared to a adenoviral vectors of the art. This is not only due to improved capabilities to avoid the host immune system (in or outside a cell) but also to other factors. Another reason for the observed prolonged expression is that promoters that are commonly used for the expression of gene of interest in a gene therapy setting are at least in part protected from shut down of expression in the host. A non-limiting example of such a commonly used promoter is the CMV promoter.

**Detailed description**

The present invention provides means and methods for the generation and manufacturing of recombinant Ad vectors that are modified in E2B and/or E4 functions, preferably, said vectors comprise E1 and/or E2A deletions. For this purpose, the vector genome is modified in the respective promoter regions, such that the promoter is only active in a suitable complementing cell line or only active following a certain signal in the case of an inducible promoter. The modified promoter is, on the other hand, inactive under normal conditions, and in normal mammalian and/or human cells. Hence, vectors that possess said modified promoter in the E2B and/or E4 region do not express the respective transcription regions in mammalians and/or humans. The promoter modifications can be made in recombinant Ad vector genomes that lack E1, or E1 and E2A, or E1 and E3, or E1 and E2A and E3, or E1 and E4, or E1 and E2A and E4, or E1 and E3 and E4, or E1 and E2A and E3 and E4. In one aspect the invention provides the latter four variants in the case of E2B promoter modification only. Since there is no overlap between vector sequences and adenoviral sequences in the complementing cell line, reversion of the modified phenotype due to homologous recombination cannot occur.

Successful replacement of the E4 promoter by a synthetic promoter has recently been reported (Fang et al., 1997). In this study, the E4 promoter was replaced by synthetic Gal4 binding sites and the vectors modified in the E4 region could be efficiently propagated in 293 cells expressing the appropriate Gal4/VP16 fusion protein. However, replication of the vectors modified in the E4 region was dramatically impaired in cells that did not express the Gal4/VP16 fusion protein.

It was assumed impossible by persons skilled in the art to manipulate the E2 promoters `because of overlap with the major late open reading frame L4' (quote-unquote by Rittner et al., 1997). However, in the present invention, we provide means and methods to produce vectors that are attenuated in botheE2 early and late promoter regions, in particular in vectors that are also deleted for the E2A gene.

For use in gene therapy, it would clearly be advantageous to have recombinant Ad vectors available that do not produce or produce reduced amounts of viral polypeptides and hence are less immunogenic, but yet retain all capabilities of the current vectors. The present invention provides novel recombinant Ad vectors that improve persistence and diminish pathology in mammals and/or humans. The invention also provides means, methods and materials for the generation, manufacturing and use of such recombinant Ad vectors.

In one embodiment of the invention, the genome of an E1 deleted recombinant Ad vector (as described in WO97/00326) is modified in the E4 promoter region. The entire promoter region of the E4 transcription unit is deleted and replaced by a synthetic promoter that consists of an artificial TATA-box preceded by a specific sequence that is recognized by the DNA binding domain (DBD) of a DBD-transactivating fusion protein. Said DBD can be derived from a DNA binding protein that originates from prokaryotes and/or lower eukaryotes but not from mammals and/or humans. Alternatively, the DBD can be rationally designed to recognize a synthetic promoter. Transcription of the E4 region of such recombinant Ad genome would not occur in mammalian and/or human cells. As a consequence, such an E1 deleted /E4 disabled recombinant Ad vector does not produce E1 encoded polypeptides and produces markedly reduced levels of the E4 encoded polypeptides. In addition, such a vector produces reduced levels of the viral late proteins, since it has been shown that E4 polypeptides are required for the expression of late genes from E1 deleted vectors (Lusky et al., 1998).

In a more preferred embodiment, the genome of an E1 deleted recombinant Ad vector is further deleted for E2A and modified in the E4 promoter region. The entire promoter region of E4 is deleted from the E1+E2A deleted recombinant Ad DNA and replaced by a synthetic promoter as described above. Transcription of E4 from such a recombinant Ad genome would not occur in mammalian and/or human cells. As a consequence, such an E1+E2A deleted and E4 disabled Ad vector does neither produce E1 encoded polypeptides, nor the E2A encoded polypeptide and produces markedly reduced levels of the E4 encoded polypeptides. In addition, such a vector expresses reduced levels of viral late genes, since removing E2A and/or disabling E4 functions inhibits the Ad late gene expression from recombinant Ad vectors (see Figure 1A and Lusky et al., 1998).

In an even more preferred embodiment, the genome of an E1+E2A deleted recombinant Ad vector is deleted for the E4 promoter region and modified in the E2 promoter region. The entire promoter region of the E4 transcription unit is removed from the E1+E2A deleted recombinant Ad DNA and replaced by a synthetic promoter as described above. In addition, the E2 promoter of said vector is inactivated. This inactivation is accomplished by deletion of E2F binding sites from the E2-early promoter, and/or mutation of the TATA box and/or CAP site of the E2-early promoter and/or mutation of the SP1 binding sites and/or TATA-box of the E2-late promoter. A synthetic promoter, as described above, is inserted directly adjacent to the pTP and pol coding sequences of the E2B region. Transcription of the E4 and the E2B region of such recombinant Ad genome would not occur in mammalian and/or human cells. As a consequence, cells infected with said E1+E2A deleted and E2B+E4 attenuated recombinant Ad vector do neither produce E1 encoded polypeptides, nor the E2A encoded polypeptide, and produce markedly reduced levels of the E2B encoded polypeptides and the E4 encoded polypeptides. In addition, cells infected with these vectors produce reduced levels of viral late gene products since the expression of late genes is abrogated in the absence of E2A and E4 encoded proteins.

In another embodiment, the genome of an E1/E2A deleted recombinant Ad vector is modified only in the E2 promoter region, as described above. Transcription of the E2B region of such recombinant Ad genome would not occur in mammalian and/or human cells. Therefore, cells infected with such E1/E2A deleted and E2B attenuated Ad vector do neither produce E1-encoded polypeptides, nor the E2A encoded polypeptide, and produce markedly reduced levels of the E2B encoded polypeptides. In addition, cells infected with such vector do not produce viral late encoded proteins since the expression of viral late gene is abrogated in the absence of the E2A encoded protein.

In a preferred embodiment, the genome of an E1/E2A deleted and E2B attenuated recombinant Ad vector is further deleted for the entire E4 region, or parts thereof. Cells infected with such E1/E2A/E4 deleted and E2B attenuated Ad vector do neither produce E1-encoded polypeptides nor the E2A-encoded polypeptide nor the E4-encoded polypeptides, and produce markedly reduced levels of the E2B-encoded polypeptides. In addition, said infected cells produce reduced levels of viral late gene encoded proteins since expression of viral late genes is abrogated in the absence of E2A- and/or E4-encoded proteins.

In another embodiment, all the above-described vectors are deleted for the E3 region of the Ad genome, or parts thereof.

A prerequisite for producing Ad vectors that are deleted for the coding sequences of essential genes or that are modified in the promoter region of essential genes is that these defects are complemented in the producing cell lines. Previously, we have established a cell line that efficiently complements the deletion of E1 (WO97/00326). According to the invention, this cell line (designated PER.C6) was further equipped with an expression vector expressing the temperature sensitive mutant of E2A derived from the adenovirus H5ts125, giving rise to PER.C6/E2A. The cell lines allow the manufacturing of high titer batches of recombinant Ad vectors deleted for E1 or double deleted for E1 and E2A, respectively. Reversion of the E1 deleted phenotype, i.e. the formation of replication competent Ad (RCA) in PER.C6 or reversion of the E1 + E2A deleted phenotype in PER.C6/E2A was prevented by elimination of sequence overlap between Ad sequences present in the cell line and sequences present in the recombinant vector.

In one embodiment of the invention, the E1 complementing cell line PER.C6 is equipped with an expression cassette coding for a DBD-transactivation fusion protein. Said DBD recognizes the synthetic sequence preceding the artificial TATA box in the synthetic promoter(s) that are present in the modified genome of the recombinant Ad vectors of the invention. Said DBD is derived from a DNA binding protein that originates from prokaryotes and/or lower eukaryotes but not from mammals and/or humans. Alternatively, the DBD is rationally designed or developed by randomizing techniques to specifically recognize the synthetic promoter. Because said DBD recognizes the synthetic promoter specifically, it does not interfere with other transcription processes in PER.C6 or PER.C6-derived cells and is therefore not toxic. The DBD of the ectopically expressed DNA binding protein is either fused to its native transactivation domain or fused to a transcription activation (TA) domain derived from viral transcription factors (as non-limiting example, the herpes simplex virus VP16 protein) or fused to a TA domain from eukaryotic transcription factors (as non-limiting example, the TA domain of p65-NfkappaB), or fused to a rationally designed TA domain (as non limiting example, an amphipatic $\alpha$ -helix) or fused to a TA domain that is generated by randomizing techniques (as non-limiting example, an acidic blob). Said cell line is suitable for the manufacturing of recombinant Ad vectors that are deleted for E1 and/ or E3 or parts thereof, and in which the E4 promoter region is replaced by a synthetic promoter according to the invention.

In a further embodiment, the E1/E2A complementing cell line PER.C6/E2A is equipped with an expression cassette coding for the above-described DBD-transactivating fusion protein. Said cell line is suitable for the manufacturing of recombinant Ad vectors that are deleted for E1+E2A, and that either do or do not have a deletion of the entire E3 region or parts thereof, and in which E4 and/or E2B is equipped with a synthetic promoter according to the invention.

In yet another embodiment, the E1+E2A complementing cell line PER.C6/E2A is equipped with an expression cassette coding for the E4-orf6 protein and an expression cassette for the above described DBD-TA fusion protein. Said cell line is suitable for the manufacturing of recombinant Ad vectors that are deleted for E1+E2A+E4 or parts thereof and/or E3 or parts thereof, and in which the E2 early and late promoters are knocked-out by mutations and in which E2B is equipped with a synthetic promoter according to the invention.

In another aspect of the invention, the E4 and/or E2 promoter sequences of the recombinant Ad vector genome are deleted and appropriately replaced by a so-called inducible promoter, such as, but not limited to, the metallothionein promoter or the mouse mammary tumor virus (MMTV) promoter. Hence, the transcription of the E4 and/or the E2B genes can be switched on or off at will following a specific signal. Said vectors can be produced in complementing cell lines expressing E1 and/or E2A and/or E4orf-6 in the presence of the signal that triggers the expression of the E4 and/ or E2 genes.

The vectors according to the invention can be equipped with any foreign nucleic acid, preferably a nucleic acid encoding a therapeutic molecule. The expression can be driven by a strong viral enhancer/promoter such as but not limited to the CMV promoter. More preferably, said expression is driven by strong mammalian promoters such as but not limited to the EF-1á promoter or the UbC promoter to achieve long term expression of the therapeutic gene.

The vectors according to the invention can also be applied for purposes other than gene therapy such as, but not limited to, functional characterization of gene products *in vitro* and *in vivo*. For instance, the vectors according to the invention can be used for overexpression of a variety of known and novel genes in cell lines, tissues or animals in order to find genes that encode for proteins with a desired function such as, but not limited to, those that interfere with cell proliferation and differentation. For this application, it is of critical importance that the vector itself does not interfere with cellular processes and that it does not overrule the effect of the transgene. The vectors according to the invention are replication defective and do express the remaining viral genes, if at all, only at background levels; interference of the vector with the function and the effect of the gene of interest is therefore at least in part prevented.

The vectors according to the invention can also be used as a vaccine. As a non-limiting example, the vectors according to the invention can be equipped with an expression cassette that codes for a protein against which an immune response has to be raised. For such application, it is of critical importance that the vector itself does not dominantly elicit a response of the immune system but that the immune response, instead, is directed primarily against the transgene product. Because the vectors according to the invention are replication defective and express the remaining viral genes, if at all, only at background levels, it is expected that the immune response will be directed primarily against the transgene product.

The vectors according to the invention can also be used for protein production in mammalian cells. Therefore, the vectors according to the invention can be equipped with an expression cassette that encodes a protein of interest to be synthesized and processed in said cells. For this application, it is of importance that the vector itself does not interfere with the cellular metabolism which is harmful to the cell and which impairs synthesis and processing of the protein of interest. Because the vectors according to the invention are replication defective and express the remaining viral genes, if at all, only at background levels, said vectors are less toxic to the cells which, in turn, results in a prolonged synthesis of the protein of interest.

Adenoviral vectors typically do not or very inefficiently integrate into the host cell genome. To increase at least in part the integration frequency of adenovirus vectors elements from integrating viruses can be included in the vector. The vectors and the gene delivery vehicles of the invention are suited for integrating vectors since the vectors are at least in part not toxic to the cells. The invention therefor provides a recombinant adenoviral vector and/or a recombinant adenovirus-like gene delivery vehicle according to the invention, wherein said adenoviral vector comprises at least one adeno-associated virus terminal repeat or a functional equivalent thereof. Preferably, said adenovirus vectors comprise at least two adeno-associated virus terminal repeat or a functional equivalent thereof. A functional equivalent of an adeno-associated virus terminal repeat comprises the same integrating function in kind not necessarily in amount. In a preferred aspect, the adeno-associated virus terminal repeat is present at the extreme ends of the adenoviral vector.

The invention further provides a recombinant adenoviral vector and/or a recombinant adenovirus-like gene delivery vehicle according to the invention comprising elements derived from at least two different adenovirus serotypes. Such vectors are preferred for a variety of reasons based on the observation that in this way at least part of the favourable properties of different adenovirus serotypes may be combined.

The invention further provides a method for *ex vivo* production of a gene product in a cell comprising providing said cell with a recombinant adenoviral vector and/or a recombinant adenovirus-like gene delivery vehicle according to the invention comprising nucleic acid encoding said gene product, culturing said cell to allow expression of said gene product and optionally harvesting said cell and/or medium said cell was exposed to.

In another aspect the invention provides the use of a recombinant adenoviral vector and/or a recombinant adenovirus-like gene delivery vehicle according to the invention, for the preparation of a medicament.

In yet another aspect the invention provides a vaccine comprising a recombinant adenoviral vector and/or a recombinant adenovirus-like gene delivery vehicle according to the invention, wherein said adenoviral vector comprises a nucleic acid encoding a proteinaceous molecule against which an immune response has to be raised.

## EXAMPLES

The invention will now be described with respect to the generation of recombinant Ad vectors that are deleted for the E1 and/or E2A and/or E4 region, and that, in addition, harbor E4 and/or E2B genes whose expression is controlled by synthetic promoters as well as with respect to complementing cell lines. However, the scope of the present invention is not intended to be limited thereby.

### Example 1

### Plasmid based system for the generation of recombinant Ad vectors that are deleted for E1 and/or E2A and in which the E4 and/or E2B promoter is replaced by a synthetic promoter

*A. Generation of pBr/Ad.Bam-rITR (ECACC deposit P97082122)*

In order to facilitate blunt end cloning of the ITR sequences, wild-type human adenovirus type 5 (Ad5) DNA was treated with Klenow enzyme in the presence of excess dNTPs. After inactivation of the Klenow enzyme and purification by phenol/chloroform extraction followed by ethanol precipitation, the DNA was digested with *Bam*HI. This DNA preparation was used without further purification in a ligation reaction with pBr322 derived vector DNA prepared as follows: pBr322 DNA was digested with *Eco*RV and *Bam*HI, dephosphorylated by treatment with TSAP enzyme (Life Technologies) and purified on LMP agarose gel (SeaPlaque GTG). After transformation into competent *E.coli* DH5a (Life Techn.) and analysis of ampiciline resistant colonies, one clone was selected that showed a digestion pattern as expected for an insert extending from the *Bam*HI site in Ad5 to the right ITR.

Sequence analysis of the cloning border at the right ITR revealed that the most 3' G residue of the ITR was missing, the remainder of the ITR was found to be correct. Said missing G residue is complemented by the other ITR during replication.

*B. Generation of pBr/Ad.Sal-rITR (ECACC deposit P97082119)*

pBr/Ad.Bam-rITR was digested with *Bam*HI and *Sal*I. The vector fragment including the adenovirus insert was isolated from LMP agarose (SeaPlaque GTG) and ligated to a 4.8 kb *Sal*I-*Bam*HI fragment obtained from wt Ad5 DNA and purified with the Geneclean II kit (Bio 101, Inc.). One clone was chosen and the integrity of the Ad5 sequences was determined by restriction enzyme analysis. Clone pBr/Ad.Sal-rITR contains adeno type 5 sequences from the *Sal*I site at bp 16746 up to and including the rITR (missing the most 3' G residue).

*C. pBr/Ad.Cla-Bam (ECACC deposit P97082117)*

Wild-type Ad type 5 DNA was digested with *Cla*I and *Bam*HI, and the 20.6-kb fragment was isolated from gel by electro-elution. pBr322 was digested with the same enzymes and purified from agarose gel by Geneclean. Both fragments were ligated and transformed into competent DH5α. The resulting clone pBr/Ad.Cla-Bam was analyzed by restriction enzyme digestion and shown to contain an insert with adenovirus sequences from bp 919 to 21566.

*D. Generation of pBr/Ad.AflII-Bam (ECACC deposit P97082114)*

Clone pBr/Ad.Cla-Bam was linearized with *Eco*RI (in pBr322) and partially digested with *Afl*II. After heat inactivation of *Afl*II for 20' at 65°C the fragment ends were filled in with Klenow enzyme. The DNA was then ligated to a blunt double stranded oligo linker containing a *Pac*I site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess of *Pac*I enzyme to remove concatamers of the oligo. The 22016 bp partial fragment containing Ad5 sequences from bp 3534 up to 21566 and the vector sequences, was isolated in LMP agarose (SeaPlaque GTG), re-ligated and transformed into competent DH5α. One clone that was found to contain the *Pac*I site and that had retained the large adeno fragment was selected and sequenced at the 5' end to verify correct insertion of the *Pac*I linker in the (lost) *Afl*II site.

*E. Generation of pBr/Ad.Bam-rITRpac#2 (ECACC deposit P97082120) and pBr/Ad.Bam-rITR#8 (ECACC deposit P97082121)*

To allow insertion of a *Pac*I site near the ITR of Ad5 in clone pBr/Ad.Bam-rITR about 190 nucleotides were removed between the *Cla*I site in the pBr322 backbone and the start of the ITR sequences. This was done as follows: pBr/Ad.Bam-rITR was digested with *Cla*I and treated with nuclease *Bal*31 for varying lengths of time (2, 5, 10 and 15 minutes). The extend of nucleotide removal was followed by separate reactions on pBr322 DNA (also digested at the *Cla*I site), using identical buffers and conditions. *Bal*31 enzyme was inactivated by incubation at 75°C for 10 minutes, the DNA was precipitated and re-suspended in a smaller volume TE buffer. To ensure blunt ends, DNA's were further treated with T4 DNA polymerase in the presence of excess dNTPs. After digestion of the (control) pBr322 DNA with *Sal*I, satisfactory degradation (~150 bp) was observed in the samples treated for 10 or 15 minutes. The pBr/Ad.Bam-rITR samples that were treated for 10 or 15 minutes were then ligated to the above described blunted *Pac*I linkers (See pBr/Ad.AflII-Bam). Ligations were purified by precipitation, digested with excess *Pac*I and separated from the linkers in an LMP agarose gel. After re-ligation, DNA's were transformed into competent DH5α and colonies analyzed. Ten clones were selected that showed a deletion of approximately the desired length and these were further analyzed by T-track sequencing (T7 sequencing kit, Pharmacia Biotech). Two clones were found with the *Pac*I linker inserted just downstream of the rITR. After digestion with *Pac*I, clone #2 has 28 bp and clone #8 has 27 bp attached to the ITR.

*F. Generation of pWE/Ad.AflII-rITR (ECACC deposit P97082116)*

Cosmid vector pWE15 (Clontech) was used to clone larger Ad5 inserts. First, a linker containing a unique *Pac*I site was inserted in the *Eco*RI sites of pWE15 creating pWE15.pac. To this end, the double stranded *Pac*I oligo as described for pBr/Ad.AflII-BamHI was used but now with its *Eco*RI protruding ends. The following fragments were then isolated by electro-elution from agarose gel: pWE15.pac digested with *Pac*I, pBr/AflII-Bam digested with *Pac*I and *Bam*HI and pBr/Ad.Bam-rITR#2 digested with *Bam*HI and *Pac*I. These fragments were ligated together and packaged using λ phage packaging extracts (Stratagene) according to the manufacturer's protocol. After infection of host bacteria, colonies were grown on plates and analyzed for presence of the complete insert. pWE/Ad.AflII-rITR contains all adenovirus type 5 sequences from bp 3534 (*Afl*II site) up to and including the right ITR (missing the most 3' G residue).

*G. Generation of pWE/Ad.AflII-EcoRI*

pWE15.pac was digested with *Cla*I and 5' protruding ends were filled using Klenow enzyme. The DNA was then digested with *Pac*I and isolated from agarose gel. pWE/AflII-rITR was digested with *Eco*RI and after treatment with Klenow enzyme digested with *Pac*I. The large 24-kb fragment containing the adenoviral sequences was isolated from agarose gel and ligated to the *Cla*I-digested and blunted pWE15.pac vector using the Ligation Express$^{tm}$ kit from Clontech. After transformation of Ultra-competent XL10-Gold cells (Stratagene), clones were identified that contained the expected insert. pWE/AflII-EcoRI contains Ad5 sequences from bp 3534-27336.

*H. Generation of pBR/Ad.AflII-Bam.tetO-E2B*

First, the shuttle vector pAAO-E-TATA was constructed using the following primers:

```
TATAplus: 5'-AGC TTT CTT ATA AAT TTT CAG TGT TAG ACT AGT AAA
          TTG CTT AAG-3'
TATAmin:  5'-AGC TCT TAA GCA ATT TAC TAG TCT AAC ACT GAA AAT
          TTA TAA GAA
```

(The underlined sequences form a modified TATA box.) The primers TATAplus and TATAmin were annealed to yield a double stranded DNA fragment flanked by 5' overhangs that are compatible for ligation with *Hind*III digested DNA. Thus, the product of the annealing reaction was used in a ligation reaction with *Hind*III digested pGL3-Enhancer Vector (Promega) to yield pAAO-E-TATA.

Next, the heptamerized tet-operator sequence was amplified from the plasmid pUHC-13-3 (Gossen and Bujard, 1992) in a PCR reaction using the Expand PCR system (Boehringer) according to the manufacturers protocol. The following primers were used:

tet3: 5'-CCG **GAG CTC** CAT GGC CTA ACT CGA GTT TAC CAC TCC C-3'

tet5: 5'-CCC **AAG CTT** AGC TCG ACT TTC ACT TTT CTC-3'

The amplified fragment was digested with *Sst*I and *Hind*III (*Sst*I and *Hind*III sites generated in the tet3 and tet5 primers are represented by the nucleotides in bold) and cloned into *Sst*I/*Hind*III digested pAAO-E-TATA giving rise to pAAO-E-TATA-7xtetO. Sequence analysis confirmed the integrity of the heptamerized tet-operator sequence in the latter plasmid.

Next, pAAO-E-TATA-7xtetO was digested with *Nco*I, and the resulting fragment containing the 7xtetO sequence was purified from agarose and used in a ligation reaction with *Nco*I digested pNEB.PmAs yielding pNEB. PmAs7xtetO. The plasmid pNEB.PmAs was obtained as follows: pBR/Ad.AflII-Bam (ECACC deposit P97082114) was digested with *Pme*I/*Asc*I and the resulting *Pme*I/*Asc*I fragment containing the 5' end of the E2B region was purified from agarose and subcloned into *Pme*I/*Asc*I digested pNEB 193 (New England BioLabs Inc.) yielding pNEB.PmAs. Then, pNEB.PmAs7xtetO was digested with *Asc*I, *Pme*I and *Sca*I and the resulting *Asc*I/*Pme*I fragment (2808 bp) containing the 5' E2B region preceded by 7xtetO was purified from agarose using the Geneclean II kit, and used in a ligation reaction with *Pme*I/*Asc*I digested pBR/Ad.AflII-Bam (the fragment that lacks the 5' end of the E2B region) yielding pBR/Ad.AflII-Bam.tetO-E2B.

*I. Generation of pBR/Ad.Bam-rITRΔE2A*

Deletion of the E2A coding sequences from pBR/Ad.Bam-rITR (ECACC deposit P97082122) has been accomplished as follows. The adenoviral sequences flanking the E2A coding region at the left and the right site were amplified from the plasmid pBr/Ad.Sal-rITR (ECACC deposit P97082119) in a PCR reaction with the Expand PCR system (Boehringer) according to the manufacturers protocol. The following primers were used:

Right flanking sequences (corresponding Ad5 nucleotides 24033 to 25180):

**ΔE2A.SnaBI:** 5'-GGC GTA CGT AGC CCT GTC GAA AG-3'

**ΔE2A.DBP-start:** 5'-CCA ATG CAT TCG AAG TAC TTC CTT CTC CTA TAG GC-3'

The amplified DNA fragment was digested with *Sna*BI and *Nsi*I (*Nsi*I site is generated in the primer ΔE2A.DBP-start, underlined).

Left flanking sequences (corresponding Ad5 nucleotides 21557 to 22442):

**ΔE2A.DBP-stop:** 5'-CCA ATG CAT ACG GCG CAG ACG G-3'

**ΔE2A.BamHI:** 5'-GAG GTG GAT CCC ATG GAC GAG-3'

The amplified DNA was digested with *Bam*HI and *Nsi*I (*Nsi*I site is generated in the primer ΔE2A.DBP-stop, underlined). Subsequently, the digested DNA fragments were ligated into *Sna*BI/*Bam*HI digested pBr/Ad.Bam-rITR, yielding pBr/Ad.Bam-rITRΔE2A. The unique *Nsi*I site can be used to introduce an expression cassette for a gene to be transduced by the recombinant vector.

*J. Generation of pBR/Ad.Bam-rITRΔE2AΔE2p*

First, pBR/Ad.Bam-rITRΔE2A was digested with *Asc*I and *Xba*I and the resulting *Asc*I/*Xba*I fragment containing the E2 promoter region was purified from agarose using the Geneclean II kit and subcloned into *Asc*I/*Xba*I digested pNEB 193 (New England BioLabs Inc.) yielding pNEB-AX. The latter plasmid contains the Ad E2 early and late promoter sequences. A part of the E2 early promoter sequence was amplified from pNEB-AX using the primers E2eSpeI and E2eSrf whereas a part of the E2 late promoter sequence was amplified from pNEB-AX using the primers E21Aat and E21SpeI.

E2eSpeI: 5' GG**A CTA GT**C TAA GTC TTC TCC AGC GGC CAC ACC CGG 3'

E2eSrf: 5' GAG TTA TAC CCT **GCC CGG GC**G ACC GCA CC 3'

E21Aat: 5' GGG CTG TG**G ACG TC**G GCT TAC CTT CGC AAG TTC GTA CCT GAG GAC TAC CAT GCA CAC GAG ATT AGG 3'

E21SpeI: 5' GCG **AAA CTA GT**C CTT CAG AGT CAG CGC GCA GTA CTT GCT AAA AAG AGC CTC CGC 3'

The nucleotides indicated in bold generate the unique *Spe*I, *Srf*I and *Aat*I restriction sites whereas the underlined nucleotides generate mutations in the promoter sequences that knock out the E2F binding sites and mutate the TATA box and CAP site of the E2 early promoter, and mutate the SP1 binding sites as well as the TATA box of the E2 late promoter.

The amplification products of the E2 early and late promoter regions were purified from gel using the Geneclean II kit and digested with *Spe*I. Thereafter, the *Spe*I digested PCR products were ligated. After chloroform/phenol extraction, the ligated DNA's were digested with *Aat*II and *Srf*I. The resulting DNA's were separated in an agarose gel and the ligation product that comprised the reconstituted and modified E2 (early and late) promoter was purified from agarose using the Geneclean II kit. The purified fragment was then used in a ligation reaction with *Aat*II/*Srf*I digested pNEB-AX yielding pNEB-AXΔE2p. The primers for amplification and mutation of the E2 promoter were chosen such that part of the native E2 early promoter spanning the E2F sites and a putative TATA-box was excluded from amplification. As a result, the modified E2 promoter region that is reconstituted by ligation of the two PCR products is deleted for the above mentioned promoter sequences. Next, pNEB-AXΔE2p was digested with *Asc*I and *Srf*I and the resulting *Asc*I/*Srf*I fragment containing the mutated E2 promoter was purified from agarose using the Geneclean Spin kit and cloned into *Asc*I/*Srf*I digested pBR/Ad.Bam-rITRΔE2A (the fragment that lacks the E2 promoter) giving rise to pBR/Ad.Bam-rITRΔE2AΔE2p.

*K. Generation of pBR/Ad.Bam-rITR.tetO-E4, pBR/Ad.Bam-rITR.ΔE2A.tetO-E4 and pBR/Ad.Bam-rITR.ΔE2A.ΔE2p.tetO-E4*

First, pBR/Ad.Bam-rITR was digested with *Pac*I and *Sse*83871 and the resulting *Pac*I/*Sse*83871 fragment containing the E4 region was purified from agarose using the Geneclean II kit and subcloned into *Pac*I/*Sse*83871 digested pNEB 193 (New England BioLabs Inc.) giving rise to pNEB-PaSe. The latter plasmid was used to amplify the Ad sequences that flank the E4 promoter. Sequences upstream of the E4 promoter were amplified using the primers 3ITR and 5ITR whereas sequences downstream of the E4 promoter were amplified using the primers H3DE4 and AvDE4.

| | |
|---|---|
| 3ITR: | 5' CCG GAT CC**T TAA TTA A**GT TAA CAT CAT C 3' |
| 5ITR: | 5' GAT CCG **GAG CTC** TAC GTC ACC CGC CCC G 3' |
| H3DE4: | 5' CCC **AAG CTT** AGT CCT ATA TAT ACT CGC TC 3' |
| AvDE4: | 5' CTC CTG **CCT AGG** CAA AAT AGC 3' |

The nucleotides indicated in bold form unique restriction sites for *Pac*I, *Sst*I, *Hind*III and *Avr*II, respectively. The PCR products were first purified using the QIAquick PCR purification kit. The PCR product of the sequence downstream of the E4 promoter was digested with *Hind*III and *Avr*II. The PCR product of the sequence upstream of the E4 promoter was digested with *Sst*I and ligated to a fragment that contained the 7xtetO sequence which was obtained by digestion of pAAO-E-TATA-7xtetO with *Sst*I and *Hind*III. The ligation products were thereafter digested with *Hind*III and *Pac*I and the ligation product comprising the region upstream of the E4 promoter linked to the 7xtetO sequence was purified from gel. The latter fragment was used in a ligation reaction with *Hind*III/*Avr*II digested PCR product of the sequence downstream of the E4 promoter and with *Pac*I/*Avr*II digested pNEB-PaSe giving rise to pNEB-PaSe.tetO.

Next, pNEB-PaSe.tetO was digested with *Pac*I and *Sse*83871 and the fragment containing the artificial 7xtetO promoter sequences in front of the E4 region was purified from agarose and cloned into *Pac*I/*Sse*83871 digested pBR/Ad.Bam-rITRΔE2A giving rise to pBR/Ad.Bam-rITRΔE2A.teto-E4. The latter plasmid was used for digestion with *Pac*I and *Not*I and the resulting *Not*I/*Pac*I fragment containing the 7xtetO promoter sequences was purified from agarose and used in a ligation reaction with *Not*I/*Pac*I digested pBR/Ad.Bam-rITR (the fragment that lacks the E4 promoter) giving rise to pBR/Ad.Bam-rITRtetO-E4. Finally, the above described *Not*I/*Pac*I fragment from pBR/Ad.Bam-rITR.ΔE2A.tetO-E4 was also cloned into *Not*I/*Pac*I digested pBR/Ad.Bam-rITR.ΔE2A.ΔE2p (the fragment that lacks the E4 promoter region) giving rise to pBR/Ad.Bam-rITR.ΔE2A.ΔE2p.teto-E4.

*L. Generation of pWE/Ad.AflII-rITR.ΔE2A. tetO-E2B, pWE/Ad.AflII-rITR.tetO-E4, pWE/Ad.AflII-rITR.ΔE2A, pWE/Ad.AflII-rITR.ΔE2A.tetO-E4, and pWE/Ad.AflII-rITR.Δ E2A.tetO-E2B.tetO-E4.*

The cosmid pWE15.pac was linearized by digestion with *Pac*I and used in a ligation reaction with *Bam*HI/*Pac*I digested pBR/Ad.AflII-Bam.tetO-E2B and *Bam*HI/*Pac*I digested pBR/Ad.Bam-rITR.ΔE2A.ΔE2p. The ligation mixture was used in a packaging reaction using λ phage-packaging extracts (Stratagene) according to the manufacturer's protocol, yielding the cosmid pWE/Ad.AflII-rITR.ΔE2A.teto-E2B.

Similarly, *Pac*I-linearized pWE15.pac was used in a ligation reaction with *Bam*HI/*Pac*I digested pBR/Ad.AflII-Bam and *BamH*I/*Pac*I digested pBR/Ad.Bam-rITR-tetO-E4. The ligation mixture was used in a packaging reaction using λ phage-packaging extracts (Stratagene) according to the manufacturer's protocol, yielding the cosmid pWE/Ad.AflII-rITR.tetO-E4.

Similarly, *Pac*I-linearized pWE15.pac was used in a ligation reaction with *Bam*HI/*Pac*I digested pBR/Ad.AflII-Bam and *Bam*HI/*Pac*I digested pBR/Ad.Bam-rITR.ΔE2A. The ligation mixture was used in a packaging reaction using λ phage-packaging extracts (Stratagene) according to the manufacturer's protocol, yielding the cosmid pWE/Ad.AflII-rITR.ΔE2A.

Similarly, *Pac*I-linearized pWE15.pac was used in a ligation reaction with *Bam*HI/*Pac*I digested pBR/Ad.AflII-Bam and *Bam*HI/*Pac*I digested pBR/Ad.Bam-rITR.ΔE2A.tetO-E4. The ligation mixture was used in a packaging reaction using λ phage-packaging extracts (Stratagene) according to the manufacturer's protocol, yielding the cosmid pWE/Ad.AflII-rITR.ΔE2A.tetO-E4.

Similarly, *Pac*I-linearized pWE15.pac was used in a ligation reaction with *Bam*HI/*Pac*I digested pBR/Ad.AflII-Bam.tetO-E2B and *Bam*HI/*Pac*I digested pBR/Ad.Bam-rITR.ΔE2A.Δ E2p.tetO-E4. The ligation mixture was used in a packaging reaction using λ phage-packaging extracts (Stratagene) according to the manufacturer's protocol, yielding the cosmid pWE/Ad.AflII-rITR.ΔE2A.tetO-E2B.tetO-E4.

*M. Generation of the adapter plasmids*

Adapter plasmid pMLP.TK (described in EP 95202213) was modified as follows: SV40 polyA sequences were amplified with primer SV40-1 (introduces a *Bam*HI site) and SV40-2 (introduces a *Bgl*II site). In addition, Ad5 sequences present in this construct (from nt. 2496 to nt. 2779; Ad5 sequences nt. 3511 to 3794) were amplified with primers Ad5-1 (introduces a *Bgl*II site) and Ad5-2.

SV40-1: 5'-GGGGGATCCGAACTTGTTTATTGCAGC-3'
SV40-2: 5'-GGGAGATCTAGACATGATAAGATAC-3'
Ad5-1: 5'-GGGAGATCTGTACTGAAATGTGTGGGC-3'
Ad5-2: 5'-GGAGGCTGCAGTCTCCAACGGCGT-3'

Both PCR fragments were digested with *Bgl*II and ligated. The ligation product was amplified with primers SV40-1 and Ad5-2 and digested with *Bam*HI and *Afl*II. The digested fragment was then ligated into pMLP.TK predigested with the same enzymes. The resulting construct, named pMLPI.TK, contains a deletion in adenovirus E1 sequences from nt. 459 to nt. 3510.

This plasmid was used as the starting material to make a new vector in which nucleic acid molecules comprising specific promoter and gene sequences can be easily exchanged. First, a PCR fragment was generated from pZipΔMo+PyF101($N^-$) template DNA (described in PCT/NL96/00195) with the following primers:

LTR-1: 5'-CTG TAC GTA CCA GTG CAC TGG CCT AGG CAT GGA AAA ATA CAT AAC TG-3' and
LTR-2: 5'-GCG GAT CCT TCG AAC CAT GGT AAG CTT GGT ACC GCT AGC GTT AAC CGG GCG ACT CAG TCA ATC G-3'.

Pwo DNA polymerase (Boehringer Mannheim) was used according to manufacturers protocol with the following temperature cycles: once 5 minutes at 95°C; 3 minutes at 55°C; and 1 minute at 72°C, and 30 cycles of 1 minute at 95°C, 1 minute at 60°C, 1 minute at 72°C, followed by once 10 minutes at 72° C. The PCR product was then digested with *Bam*HI and ligated into pMLP10 (Levrero et al., 1991) digested with *Pvu*II and *Bam*HI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Sequencing confirmed correct amplification of the LTR fragment however the most 5' bases in the pcr fragment were missing so that the *Pvu*II site was not restored. Next, the coding region of the murine HSA gene was inserted. pLTR10 was digested with *Bst*BI followed by Klenow treatment and digestion with *Nco*I. The HSA gene was obtained by PCR amplification on pUC18-HSA (Kay et al., 1990) using the following primers:

HSA1, 5'-GCG CCA CCA TGG GCA GAG CGA TGG TGG C-3' and
HSA2, 5'-GTT AGA TCT AAG CTT GTC GAC ATC GAT CTA CTA ACA GTA GAG ATG TAG AA-3'.

The 269 bp-amplified fragment was sub-cloned in a shuttle vector using the *Nco*I and *Bgl*II sites. Sequencing confirmed incorporation of the correct coding sequence of the HSA gene, but with an extra TAG insertion directly following the TAG stop codon. The coding region of the HSA gene, including the TAG duplication was then excised as a *Nco*I (sticky)-*Sal*I (blunt) fragment and cloned into the 3.5 kb *Nco*I (sticky)/*Bst*BI (blunt) fragment from pLTR10, resulting in pLTR-HSA10.

Finally, pLTR-HSA10 was digested with *EcoRI* and *Bam*HI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd5/L420-HSA that contains convenient recognition sites for various restriction enzymes around the promoter and gene sequences. *Sna*BI and *Avr*II can be combined with *Hpa*I, *Nhe*I, *Kpn*I, *Hind*III to exchange promoter sequences, while the latter site can be combined with the *Cla*I or *Bam*HI sites 3' from HSA coding region to replace genes in this construct.

The vector pAd5/L420-HSA was then modified to create a *Sal*I or *Pac*I site upstream of the left ITR. Hereto pAd5/L420-HSA was digested with *Eco*RI and ligated to a *Pac*I linker (5'-AATTGTCTTAATTAACCGCTTAA-3'). The ligation mixture was digested with *Pac*I and religated after isolation of the linear DNA from agarose gel to remove concatamerised linkers. This resulted in adapter plasmid pAd5/L420-HSA.pac.

Another adapter plasmid that was designed to allow easy exchange of nucleic acid molecules was made by replacing the promoter, gene and polyA sequences in pAd5/L420-HSA with the CMV promoter, a multiple cloning site, an intron and a polyA signal. For this purpose, pAd/L420-HSA was digested with *Avr*II and *Bgl*II followed by treatment with Klenow to obtain blunt ends. The 5.1 kb fragment with pBr322 vector and adenoviral sequences was isolated and ligated to a blunt 1570 bp fragment from pcDNA1/amp (Invitrogen) obtained by digestion with *Hha*I and *Avr*II followed by treatment with T4 DNA polymerase. This adapter plasmid was named pAd5/Clip. To enable removal of vector sequences from the left ITR in pAd5/Clip, this plasmid was partially digested with *Eco*RI and the linear fragment was isolated. An oligo of the sequence 5' TTAAGTCGAC-3' was annealed to itself resulting in a linker with a *Sal*I site and *EcoRI* overhang. The linker was ligated to the partially digested pAd5/Clip vector and clones were selected that had the linker inserted in the *Eco*RI site 23 bp upstream of the left adenovirus ITR in pAd5/Clip resulting in pAd5/Clip.sal.

To create an adapter plasmid that only contains a polylinker sequence and no promoter or polyA sequences, pAd5/L420-HSA.pac was digested with *Avr*II and *Bgl*II. The vector fragment was ligated to a linker oligonucleotide digested with the same restriction enzymes. The linker was made by annealing oligos of the following sequence:

PLL-1: 5'- GCC ATC CCT AGG AAG CTT GGT ACC GGT GAA TTC GCT AGC GTT AAC GGA TCC TCT AGA CGA GAT CTG G-3' and PLL-2: 5'- CCA GAT CTC GTC TAG AGG ATC CGT TAA CGC TAG CGA ATT CAC CGG TAC CAA GCT TCC TAG GGA TGG C-3'.

The annealed linkers were digested with *Avr*II and *Bgl*II and separated from small ends by column purification (Qiaquick nucleotide removal kit) according to manufacturers recommendations. The linker was then ligated to the *Avr*II/*Bgl*II digested pAd5/L420-HSApac fragment. A clone, named pAdMire, was selected that had the linker incorporated and was sequenced to check the integrity of the insert. Adapter plasmid pAdMire enables easy insertion of complete expression cassettes.

An adapter plasmid containing the human CMV promoter that mediates high expression levels in human cells was constructed as follows: pAd5/L420-HSA.pac was digested with *Avr*II and 5' protruding ends were filled in using Klenow enzyme. A second digestion with *Hind*III resulted in removal of the L420 promoter sequences. The vector fragment was isolated and ligated to a PCR fragment containing the CMV promoter sequence. This PCR fragment was obtained after amplification of CMV sequences from pCMVLacI (Stratagene) with the following primers:

CMVplus: 5'-GATCGGTACCACTGCAGTGGTCAATATTGGCCATTAGCC-3' and

CMVminA: 5'-GATCAAGCTTCCAATGCACCGTTCCCGGC-3'.

The PCR fragment was first digested with *Pst*I (underlined in CMVplus) after which the 3'-protruding ends were removed by treatment with T4 DNA polymerase. Then the DNA was digested with *Hind*III (underlined in CMVminA) and ligated into the above described pAd5/L420-HAS.pac vector fragment digested with *Avr*II and *Hind*III. The resulting plasmid was named pAd5/CMV-HSApac. This plasmid was then digested with *Hind*III and *Bam*HI and the vector fragment was isolated and ligated to the polylinker sequence obtained after digestion of pAdMire with *Hind*III and *Bgl*II. The resulting plasmid was named pAdApt. Adapter plasmid pAdApt contains nucleotides -735 to +95 of the human CMV promoter (Boshart et al., 1985).

The adapter plasmid pCMV.LacZ was generated as follows: The plasmid pCMV.TK (EP 95-202 213) was digested with *Hind*III, blunted with Klenow and dNTPs and subsequently digested with *Sal*I. The DNA fragment containing the CMV promoter was isolated. The plasmid pMLP.nlsLacZ (EP 95-202 213) was digested with *Kpn*I, blunted with T4 DNA polymerase and subsequently digested with *Sal*I. The DNA fragment containing the LacZ gene and adjacent adenoviral sequences was isolated. Next, the two DNA fragments were ligated with T4 DNA ligase in the presence of ATP, giving rise to pCMV.nlsLacZ.

The adapter plasmid pAd5/CLIP.LacZ was generated as follows: The E.coli LacZ gene was amplified from the plasmid pMLP.nlsLacZ (EP 95-202 213) by PCR with the primers

5'-GGGGTGGCCAGGGTACCTCTAGGCTTTTGCAA-3' and

5'-GGGGGGATCCATAAACAAGTTCAGAATCC-3'.

The PCR reaction was performed using Ex Taq (Takara) according to the suppliers protocol at the following amplification program: 5 minutes 94°C, 1 cycle; 45 seconds 94°C and 30 seconds 60°C and 2 minutes 72°C, 5 cycles; 45 seconds 94°C and 30 seconds 65°C and 2 minutes 72°C, 25 cycles; 10 minutes 72; 45 seconds 94°C and 30 seconds 60°C and 2 minutes 72°C, 5 cycles, I cycle. The PCR product was subsequently digested with *Kpn*1 and *Bam*HI and the digested DNA fragment was ligated into *Kpn*I/*Bam*HI digested pcDNA3 (Invitrogen), giving rise to pcDNA3.nlsLacZ. Next, the plasmid pAd/CLIP was digested with *Spe*I. The large fragment containing part of the 5' part CMV promoter and the adenoviral sequences was isolated. The plasmid pcDNA3.nlsLacZ was digested with *Spe*I and the fragment containing the 3'part of the CMV promoter and the lacZ gene was isolated. Subsequently, the fragments were ligated, giving rise to pAd/CLIP.LacZ. The reconstitution of the CMV promoter was confirmed by restriction digestion. Next the LacZ gene was digested from pAd/CLIP.LacZ with *Kpn*I and *Xba*I and isolated from agarose gel. The plasmid pAdApt was digested with *Kpn*I and *Xba*I and the large fragment was purified from agarose gel. Next, the LacZ fragment and pAdApt fragment were ligated, yielding pAdApt.LacZ.

The adapter plasmid pAd5/CLIP.Luc was generated as follows: The plasmid pCMV.Luc (EP 95-202 213) was digested with *Hind*III and *Bam*HI. The DNA fragment containing the luciferase gene was isolated. The adapter plasmid pAd/CLIP was digested with *Hind*III and *Bam*HI, and the large fragment was isolated. Next, the isolated DNA fragments were ligated, giving rise to pAd5/CLIP.Luc.

The adapter plasmid pAd5/ULIP.LacZ.sal was generated as follows: First, the Ubiquitin C promoter (Nenoi et al., 1996) was amplified from genomic DNA from human osteosarcoma cells (U2-OS) by a PCR reaction using the following primers:

Upstream: 5'-GAT CGA TAT CAC GGC GAG CGC TGC CAC G-3'

Downstream: 5'-GAT CGA TAT CTG TCT AAC AAA AAA GCC AAA AAC GGC C-3'

(The underlined sequences form *Eco*RV restriction sites).

Next, pAd/CLIP.Sal was digested with *Spe*I to remove the CMV promoter sequences and the remaining part of the vector was religated to yield pAd/LIP.Sal. The latter plasmid was digested with *Eco*RV and used in a ligation reaction with *Eco*RV digested Ubiquitin C promoter containing PCR product yielding pAd5/ULIP.Sal. Then, pAd5/ULIP.Sal was digested with *Not*I, and the resulting overhang was filled in by Klenow treatment and thereafter digested by *Xba*I, followed by dephosphorylation. The resulting DNA was used in a ligation reaction with *Kpn*I/*Xba*I digested pAd/CLIP.LacZ.Sal, from which the *Kpn*I sites was made blunt by treatment with T4 DNA polymerase in the absence of nucleotides, yielding pAd/ULIP.LacZ.Sal.

The adapter plasmid pAd/EF-1a.LacZ.Pac is generated as follows. The EF-1a promoter is amplified by PCR from the plasmid pEF-BOS (Mizushima and Nagata, 1990) using the primers

Upstream: 5'-GAT CGG TAC CCG TGA GGC TCC GGT GCC C-3'

Downstream: 5'-GAT CGG TAC CAA GCT TTT CAC GAC ACC TGA AA TGG-3'.

(The underlined sequences form *Acc*65I restriction sites).

Next, pAdApt.LacZ is digested with *Avr*II and *Acc*65I, treated with Klenow enzyme and religated to generate pAd5/Nop.

LacZ. The latter plasmid is digested with *Acc*65I and used in a ligation reaction with *Acc*65I digested PCR product containing the EF-1a promoter, yielding pAd5/EF-1a.LacZ.

## Example 2

**Generation of producer cell lines for the production of recombinant adenoviral vectors deleted in early region 1 and early region 2A**

Here is described the generation of cell lines for the production of recombinant adenoviral vectors that are deleted in early region 1 (E1) and early region 2A (E2A). The producer cell lines complement for the E1 and E2A deletion from recombinant adenoviral vectors *in trans* by constitutive expression of both E1 and E2A genes. The pre-established Ad5-E1 transformed human embryo retinoblast cell line PER.C6 (WO 97/00326) was further equipped with E2A expression cassettes.

The adenoviral E2A gene encodes a 72 kDa DNA Binding Protein with has a high affinity for single stranded DNA. Because of its function, constitutive expression of DBP is toxic for cells. The ts125E2A mutant encodes a DBP which has a Pro→Ser substitution of amino acid 413 (van der Vliet, 1975). Due to this mutation, the ts125E2A encoded DBP is fully active at the permissive temperature of 32°C, but does not bind to ssDNA at the non-permissive temperature of 39°C. This allows the generation of cell lines that constitutively express E2A, which is not functional and is not toxic at the non-permissive temperature of 39°C. Temperature sensitive E2A gradually becomes functional upon temperature decrease and becomes fully functional at a temperature of 32°C, the permissive temperature.

*A. Generation of plasmids expressing the wild type E2A-or temperature sensitive ts125E2A gene.*

pcDNA3wtE2A: The complete wild-type early region 2A (E2A) coding region was amplified from the plasmid pBR/Ad.Bam-rITR (ECACC deposit P97082122) with the primers DBPpcrl and DBPpcr2 using the Expand™ Long Template PCR system according to the standard protocol of the supplier (Boehringer Mannheim). The PCR was performed on a Biometra Trio Thermoblock, using the following amplification program: 94°C for 2 minutes, 1 cycle; 94°C for 10 seconds + 51°C for 30 seconds + 68°C for 2 minutes, 1 cycle; 94°C for 10 seconds + 58°C for 30 seconds + 68°C for 2 minutes, 10 cycles; 94°C for 10 seconds + 58°C for 30 seconds + 68°C for 2 minutes with 10 seconds extension per cycle, 20 cycles; 68°C for 5 minutes, 1 cycle. The primer DBPpcrl: CGG GAT CC*G CCA CC**A*** **TGG CCA GTC GGG AAG AGG AG** (5' to 3') contains a unique *Bam*HI restriction site (underlined) 5' of the Kozak sequence (italic) and start codon of the E2A coding sequence. The primer DBPpcr2: CGG AAT TC**T TAA AAA TCA AAG GGG TTC TGC CGC** (5' to 3') contains a unique *Eco*RI restriction site (underlined) 3' of the stop codon of the E2A coding sequence. The bold characters refer to sequences derived from the E2A coding region. The PCR fragment was digested with *Bam*HI/*Eco*RI and cloned into *Bam*HI/*Eco*RI digested pcDNA3 (Invitrogen), giving rise to pcDNA3wtE2A.

pcDNA3tsE2A: The complete ts125E2A-coding region was amplified from DNA isolated from the temperature sensitive adenovirus mutant H5ts125 (Ensinger et al., 1972; van der Vliet et al., 1975). The PCR amplification procedure was identical to that for the amplification of wtE2A. The PCR fragment was digested with *Bam*HI/*Eco*RI and cloned into *Bam*HI/*Eco*RI digested pcDNA3 (Invitrogen), giving rise to pcDNA3tsE2A. The integrity of the coding sequence of wtE2A and tsE2A was confirmed by sequencing.

*B. Growth characteristics of producer cells for the production of recombinant adenoviral vectors cultured at 32-, 37- and 39°C.*

PER.C6 cells were cultured in Dulbecco's Modified Eagle Medium (DMEM, Gibco BRL) supplemented with 10% Fetal Bovine Serum (FBS, Gibco BRL) and 10mM $MgCl_2$ in a 10% $CO_2$ atmosphere at either 32°C, 37°C or 39°C. At day 0, a total of 1 x $10^6$ PER.C6 cells were seeded per $25cm^2$ tissue culture flask (Nunc) and the cells were cultured at either 32°C, 37°C or 39°C. At day 1-8, cells were counted. Figure 2 shows that the growth rate and the final cell density of the PER.C6 culture at 39°C are comparable to that at 37°C. The growth rate and final density of the PER.C6 culture at 32°C were slightly reduced as compared to that at 37°C or 39°C. No significant cell death was observed at any of the incubation temperatures. Thus PER.C6 performs very well both at 32°C and 39°C, the permissive and non-permissive temperature for ts125E2A, respectively.

*C. Transfection of PER.C6 with E2A expression vectors; colony formation and generation of cell lines*

One day prior to transfection, 2 x $10^6$ PER.C6 cells were seeded per 6 cm tissue culture dish (Greiner) in DMEM, supplemented with 10% FBS and 10mM $MgCl_2$ and incubated at 37°C in a 10% $CO_2$ atmosphere. The next day, the cells were transfected with 3, 5 or 8µg of either pcDNA3, pcDNA3wtE2A or pcDNA3tsE2A plasmid DNA per dish, using the LipofectAMINE PLUS™ Reagent Kit according to the standard protocol of the supplier (Gibco BRL), except that the cells were transfected at 39°C in a 10% $CO_2$ atmosphere. After the transfection, the cells were constantly kept at 39°C, the non-permissive temperature for ts125E2A. Three days later, the cells were put in DMEM supplemented with 10% FBS, 10mM $MgCl_2$ and 0.25mg/ml G418 (Gibco BRL), and the first G418 resistant colonies appeared at 10 days post transfection. As shown in table 1, there was a dramatic difference between the total number of colonies obtained after transfection of pcDNA3 (~200 colonies) or pcDNA3tsE2A (~100 colonies) and pcDNA3wtE2A (only 4 colonies). These results indicate that the toxicity of constitutively expressed E2A can be overcome by using a temperature sensitive mutant of E2A (ts125E2A) and culturing of the cells at the non-permissive temperature of 39°C.

From each transfection, a number of colonies was picked by scraping the cells from the dish with a pipette. The detached cells were subsequently put into 24 wells tissue culture dishes (Greiner) and cultured further at 39°C in a 10% $CO_2$ atmosphere in DMEM, supplemented with 10% FBS, 10mM $MgCl_2$ and 0.25mg/ml G418. As shown in table 1, 100% of the pcDNA3 transfected colonies (4/4) and 82% of the pcDNA3tsE2A transfected colonies (37/45) were established to stable cell lines (the remaining 8 pcDNA3tsE2A transfected colonies grew slowly and were discarded). In contrast, only 1 pcDNA3wtE2A-transfected colony could be established. The other 3 died directly after picking.

Next, the E2A expression levels in the different cell lines were determined by Western blotting. The cell lines were seeded on 6 well tissue culture dishes and sub-confluent cultures were washed twice with PBS (NPBI) and lysed and scraped in RIPA (1% NP-40, 0.5% sodium deoxycholate and 0.1% SDS in PBS, supplemented with 1mM phenylmethylsulfonylfluoride and 0.1 mg/ml trypsin inhibitor). After 15 minutes incubation on ice, the lysates were cleared by centrifugation. Protein concentrations were determined by the Bio-Rad protein assay, according to standard procedures of the supplier (BioRad). Equal amounts of whole-cell extract were fractionated by SDS-PAGE on 10% gels. Proteins were transferred onto Immobilon-P membranes (Millipore) and incubated with the αDBP monoclonal antibody B6 (Reich et al., 1983). The secondary antibody was a horseradish-peroxidase-conjugated goat anti mouse antibody (BioRad). The Western blotting procedure and incubations were performed according to the protocol provided by Millipore. The complexes were visualized with the ECL detection system according to the manufacturer's protocol (Amersham). Figure 3 shows that all of the cell lines derived from the pcDNA3tsE2A transfection expressed the 72-kDa E2A protein (left panel, lanes 4-14; middle panel, lanes 1-13; right panel, lanes 1-12). In contrast, the only cell line derived from the pcDNAwtE2A transfection did not express the E2A protein (left panel, lane 2). No E2A protein was detected in extract from a cell line derived from the pcDNA3 transfection (left panel, lane 1), which served as a negative control. Extract from PER.C6 cells transiently transfected with pcDNA3ts125 (left panel, lane 3) served as a positive control for the Western blot procedure. These data confirmed that constitutive expression of wtE2A is toxic for cells and that using the ts125 mutant of E2A could circumvent this toxicity.

*D. Complementation of E2A deletion in adenoviral vectors on PER.C6 cells constitutively expressing full-length ts125E2A.*

The adenovirus Ad5.d1802 is an Ad 5 derived vector deleted for the major part of the E2A coding region and does not produce functional DBP (Rice et al., 1985). Ad5.dl802 was used to test the E2A trans-complementing activity of PER.C6 cells constitutively expressing ts125E2A. Parental PER.C6 cells or PER.C6tsE2A clone 3-9 were cultured in DMEM, supplemented with 10% FBS and 10mM $MgCl_2$ at 39°C and 10% $CO_2$ in 25 $cm^2$ flasks and either mock infected or infected with Ad5.dl802 at an m.o.i. of 5. Subsequently the infected cells were cultured at 32°C and cells were screened for the appearance of a cytopathic effect (CPE) as determined by changes in cell morphology and detachment of the cells from the flask. Full CPE appeared in the Ad5.dl802 infected PER.C6tsE2A clone 3-9 within 2 days. No CPE appeared in the Ad5.dl802 infected PER.C6 cells or the mock infected cells. These data showed that PER.C6 cells constitutively expressing ts125E2A complemented in trans for the E2A deletion in the Ad5.dl802 vector at the permissive temperature of 32°C.

*E. Serum-free suspension culture of PER.C6tsE2A cell lines.*

Large-scale production of recombinant adenoviral vectors for human gene therapy requires an easy and scaleable culturing method for the producer cell line, preferably a suspension culture in medium devoid of any human or animal constituents. To that end, the cell line PER.C6tsE2A c5-9 (designated c5-9) was cultured at 39°C and 10% $CO_2$ in a 175 $cm^2$ tissue culture flask (Nunc) in DMEM, supplemented with 10% FBS and 10mM $MgCl_2$. At sub-confluency (70-80% confluent), the cells were washed with PBS (NPBI) and the medium was replaced by 25 ml serum free suspension medium Ex-cell™ 525 (JRH) supplemented with 1 x L-Glutamine (Gibco BRL), hereafter designated SFM. Two days later, cells were detached from the flask by flicking and the cells were centrifuged at 1,000 rpm for 5 minutes. The cell pellet was resuspended in 5 ml SFM and 0.5 ml cell suspension was transferred to a 80 $cm^2$ tissue culture flask (Nunc), together with 12 ml fresh SFM. After 2 days, cells were harvested (all cells are in suspension) and counted in a Burker cell counter. Next, cells were seeded in a 125 ml tissue culture erlenmeyer (Corning) at a seeding density of 3 x $10^5$ cells per ml in a total volume of 20 ml SFM. Cells were further cultured at 125 RPM on an orbital shaker (GFL) at 39°C in a 10% $CO_2$ atmosphere. Cells were counted at day 1-6 in a Burker cell counter. In Figure 4, the mean growth curve from 8 cultures is shown. PER.C6tsE2A c5-9 performed well in serum free suspension culture. The maximum cell density of approximately 2 x $10^6$ cells per ml is reached within 5 days of culture.

*F. Growth characteristics of PER.C6 and PER.C6/E2A at 37°C and 39°C.*

PER.C6 cells or PER.C6ts125E2A (c8-4) cells were cultured in Dulbecco's Modified Eagle Medium (DMEM, Gibco BRL) supplemented with 10% Fetal Bovine Serum (FBS, Gibco BRL) and 10mM $MgCl_2$ in a 10% $CO_2$ atmosphere at either 37°C (PER.C6) or 39°C (PER.C6ts125E2A c8-4). At day 0, a total of 1 x $10^6$ cells were seeded per $25cm^2$ tissue culture flask (Nunc) and the cells were cultured at the respective temperatures. At the indicated time points, cells were counted. The growth of PER.C6 cells at 37°C was comparable to the growth of PER.C6ts125E2A c8-4 at 39°C (Figure 5). This shows that constitutive expression of ts125E2A encoded DBP had no adverse effect on the growth of cells at the non-permissive temperature of 39°C.

*G. Stability of PER.C6ts125E2A*

For several passages, the PER.C6ts125E2A cell line clone 8-4 was cultured at 39°C and 10% $CO_2$ in a 25 $cm^2$ tissue culture flask (Nunc) in DMEM, supplemented with 10% FBS and 10 mM $MgCl_2$ in the absence of selection pressure (G418). At sub-confluency (70-80% confluent), the cells were washed with PBS (NPBI) and lysed and scraped in RIPA (1% NP-40, 0.5% sodium deoxycholate and 0.1% SDS in PBS, supplemented with 1mM phenylmethylsulfonylfluoride and 0.1 mg/ml trypsin inhibitor). After 15 minutes incubation on ice, the lysates were cleared by centrifugation. Protein concentrations were determined by the BioRad protein assay, according to standard procedures of the supplier (BioRad). Equal amounts of whole-cell extract were fractionated by SDS-PAGE in 10% gels. Proteins were transferred onto Immobilon-P membranes (Millipore) and incubated with the αDBP monoclonal antibody B6 (Reich et al., 1983). The secondary antibody was a horseradish-peroxidase-conjugated goat anti mouse antibody (BioRad). The Western blotting procedure and incubations were performed according to the protocol provided by Millipore. The complexes were visualized with the ECL detection system according to the manufacturer's protocol (Amersham). The expression of ts125E2A encoded DBP was stable for at least 16 passages, which is equivalent to approximately 40 cell doublings (Figure 6). No decrease in DBP levels was observed during this culture period, indicating that the expression of ts125E2A was stable, even in the absence of G418 selection pressure.

## Example 3

**Generation of tTA expressing packaging cell lines**

*A. Generation of a plasmid from which the tTA gene is expressed.*

pcDNA3.1-tTA: The tTA gene, a fusion of the tetR and VP16 genes, was removed from the plasmid pUHD 15-1 (Gossen and Bujard, 1992) by digestion using the restriction enzymes *Bam*HI and *Eco*RI. First, pUHD15-1 was digested with *Eco*RI. The linearized plasmid was treated with Klenow enzyme in the presence of dNTPs to fill in the *EcoRI* sticky ends. Then, the plasmid was digested with *Bam*HI. The resulting fragment, 1025 bp in length, was purified from agarose. Subsequently, the fragment was used in a ligation reaction with *Bam*HI/*Eco*RV digested pcDNA 3.1 HYGRO (-) (Invitrogen) giving rise to pcDNA3.1-tTA. After transformation into competent *E. Coli* DH5 α (Life Techn.) and analysis of ampiciline resistant colonies, one clone was selected that showed a digestion pattern as expected for pcDNA3.1-tTA.

*B. Transfection of PER.C6 and PER.C6/E2A with the tTA expression vector; colony formation and generation of cell lines*

One day prior to transfection, 2x$10^6$ PER.C6 or PER.C6/E2A cells were seeded per 60 mm tissue culture dish (Greiner) in Dulbecco's modified essential medium (DMEM, Gibco BRL) supplemented with 10% FBS (JRH) and 10 mM $MgCl_2$ and incubated at 37°C in a 10% $CO_2$ atmosphere. The next day, cells were transfected with 4-8 µg of pcDNA3.1-tTA plasmid DNA using the LipofectAMINE PLUS™ Reagent Kit according to the standard protocol of the supplier (Gibco BRL). The cells were incubated with the LipofectAMINE PLUS™-DNA mixture for four hours at 37°C and 10% $CO_2$. Then, 2 ml of DMEM supplemented with 20% FBS and 10 mM $MgCl_2$ was added and cells were further incubated at 37°C and 10% $CO_2$. The next day, cells were washed with PBS and incubated in fresh DMEM supplemented with 10% FBS, 10 mM $MgCl_2$ at either 37°C (PER.C6) or 39°C (Per.C6/E2A) in a 10% $CO_2$ atmosphere for three days. Then, the media were exchanged for selection media; PER.C6 cells were incubated with DMEM supplemented with 10% FBS, 10 mM $MgCl_2$ and 50 μg/ml hygromycin B (GIBCO) while PER.C6/E2A cells were maintained in DMEM supplemented with 10% FBS, 10 mM $MgCl_2$ and 100 μg/ml hygromycin B. Colonies of cells that resisted the selection appeared within three weeks while nonresistant cells died during this period.

From each transfection, a number of independent, hygromycin resistant cell colonies were picked by scraping the cells from the dish with a pipette and put into 2.5 $cm^2$ dishes (Greiner) for further growth in DMEM containing 10% FBS, 10 mM $MgCl_2$ and supplemented with 50 μg/ml (PERC.6 cells) or 100 μg/ml (PERC.6/E2A cells) hygromycin in a 10% $CO_2$ atmosphere and at 37°C or 39°C, respectively.

Next, it was determined whether these hygromycin-resistant cell colonies expressed functional tTA protein. Therefore, cultures of PER.C6/tTA or PER/E2A/tTA cells were transfected with the plasmid pUHC 13-3 that contains the reporter gene luciferase under the control of the 7xtetO promoter (Gossens and Bujard, 1992). To demonstrate that the expression of luciferase was mediated by tTA, one half of the cultures was maintained in medium without doxycycline. The other half was maintained in medium with 8 μg/ml doxycycline (Sigma). The latter drug is an analogue of tetracycline and binds to tTA and inhibits its activity. All PER.C6/tTA and PER/E2A/tTA cell lines yielded high levels of luciferase, indicating that all cell lines expressed the tTA protein (Figure 7). In addition, the expression of luciferase was greatly suppressed when the cells were treated with doxycycline. Collectively, the data showed that the isolated and established hygromycin-resistant PER.C6 and PER/E2A cell clones all expressed functional tTA.

## Example 4

### Generation of recombinant adenoviral vectors.

*A. E1-deleted recombinant adenoviruses with wt E3 sequences*

To generate E1 deleted recombinant adenoviruses with the plasmid-based system, the following constructs are prepared:

a) An adapter construct containing the expression cassette with the gene of interest linearized with a restriction enzyme that cuts at the 3' side of the overlapping adenoviral genome fragment, preferably not containing any pBr322 vector sequences, and
b) A complementing adenoviral genome construct pWE/Ad.AflII-rITR (ECACC deposit P97082116) digested with *Pac*I.

These two DNA molecules are further purified by phenol/chloroform extraction and ethanol precipitation. Cotransfection of these plasmids into an adenovirus packaging cell line, preferably a cell line according to the invention, generates recombinant replication deficient adenoviruses by a one-step homologous recombination between the adapter and the complementing construct.

A general protocol as outlined below, and meant as a non-limiting example of the present invention, has been performed to produce several recombinant adenoviruses using various adapter plasmids and the Ad.AflII-rITR fragment. Adenovirus packaging cells (PER.C6) were seeded in ~25 $cm^2$ flasks and the next day, when they were at ~80% confluency, transfected with a mixture of DNA and lipofectamine agent (Life Techn.) as described by the manufacturer. Routinely, 40μl lipofectamine, 4μg adapter plasmid and 4 μg of the complementing adenovirus genome fragment AflII-rITR (or 2 μg of all three plasmids for the double homologous recombination) are used. Under these conditions transient transfection efficiencies of ~50% (48 hrs post transfection) are obtained as determined with control transfections using a pAd/CMV-LacZ adapter. Two days later, cells are passed to ~80 $cm^2$ flasks and further cultured. Approximately five (for the single homologous recombination) to eleven days (for the double homologous recombination) later a cytopathic effect (CPE) is seen, indicating that functional adenovirus has formed. Cells and medium are harvested upon full CPE and recombinant virus is released from the cells by freeze-thawing. An extra amplification step in a 80 $cm^2$ flask is routinely performed to increase the yield since at the initial stage the titers are found to be variable despite the occurrence of full CPE. After amplification, viruses are harvested and plaque purified using PER.C6 cells. Individual plaques are tested for viruses with active trans-genes.

Several different recombinant adenoviruses, comprising the luciferase gene (IG.Ad.CLIP.Luc), the bacterial LacZ gene (IG.Ad.CLIP.LacZ and IG.Ad.CMV.LacZ) or an empty CLIP cassette (IG.Ad.CLIP) have been produced using this protocol. In all cases, functional adenovirus was formed and all isolated plaques contained viruses with the expected expression cassettes.

*B. Generation of recombinant adenoviruses deleted for early region 1 and early region 2A*

Besides replacements in the E1 region, it is possible to delete or replace the E2A region in the adenovirus. This creates the opportunity to use a larger insert or to insert more than one gene without exceeding the maximum packagable size (approximately 105% of wt genome length).

Recombinant viruses that are both E1 and E2A deleted are generated by a homologous recombination procedure as described above for E1-replacement vectors using a plasmid-based system consisting of:

a) An adapter plasmid for E1 replacement according to the invention, with or without insertion of a first gene of interest.
b) The pWE/Ad.AflII-rITRΔE2A fragment, with or without insertion of a second gene of interest.

Generation and propagation of such viruses, e.g. IG.Ad.CMV.LacZΔE2A, IG.Ad.CLIP.LacZΔE2A, IG.Ad.CLIPΔE2A or IG.Ad.CLIP.LucΔE2A, requires a complementing cell line for complementation of both E1 and E2A proteins *in trans*, as described above.

Because E3 functions are not necessary for the replication, packaging and infection of the (recombinant) virus, it is also possible to delete or replace (part of) the E3 region in the E1- and/or E1/E2A-deleted adenoviral vector. This creates the opportunity to use larger inserts or to insert more than one gene without exceeding the maximum packagable size (approximately 105% of wt genome length). This can be done, e.g., by deleting part of the E3 region in the pBr/Ad.Bam-rITR clone by digestion with *Xba*I and re-ligation. This removes Ad5 wt sequences 28592-30470 including all known E3 coding regions. Another example is the precise replacement of the coding region of gpl9K in the E3 region with a polylinker allowing insertion of new sequences. This, leaves all other coding regions intact and obviates the need for a heterologous promoter since the transgene is driven by the E3 promoter and pA sequences, leaving more space for coding sequences.

To this end, the 2.7-kb *Eco*RI fragment from wt Ad5 containing the 5' part of the E3 region was cloned into the EcoRI site of pBluescript ($KS^-$) (Stratagene). Next, the *Hind*III site in the polylinker was removed by digestion with *Eco*RV and *Hinc*II and subsequent re-ligation. The resulting clone pBS.Eco-Eco/ad5ΔHIII was used to delete the gp19K-coding region. Primers 1 (5'-GGG TAT TAG GCC AA AGG CGC A-3') and 2 (5'-GAT CCC ATG GAA GCT TGG GTG GCG ACC CCA GCG-3') were used to amplify a sequence from pBS.Eco-Eco/ad5ΔHIII corresponding to sequences 28511 to 28734 in wt Ad5 DNA. Primers 3 (5'-GAT CCC ATG GGG ATC CTT TAC TAA GTT ACA AAG CTA-3') and 4 (5'-GTC GCT GTA GTT GGA CTG G-3') were used on the same DNA to amplify Ad5 sequences from 29217 to 29476. The two resulting PCR fragments were ligated together by virtue of the new introduced *Nco*I site and subsequently digested with *Xba*I and *Mun*I. This fragment was then ligated into the pBS.Eco-Eco/ad5ΔHIII vector that was digested with *Xba*I (partially) and *Mun*I generating pBS.Eco-Eco/ad5ΔHIII.Δ gpl9K. To allow insertion of foreign genes into the *Hind*III and *Bam*HI site, an *Xba*I deletion was made in pBS.Eco-Eco/ad5Δ HIII.Δgp19K to remove the *Bam*HI site in the Bluescript polylinker. The resulting plasmid pBS.Eco-Eco/ad5ΔHIIIΔgp19K ΔXbaI, contains unique *Hind*III and *Bam*HI sites corresponding to sequences 28733 (*Hind*III) and 29218 (*Bam*HI) in Ad5. After introduction of a foreign gene into these sites, either the deleted *Xba*I fragment is re-introduced, or the insert is recloned into pBS.Eco-Eco/ad5ΔHIII.Δgp19K using *Hind*III and for example *Mun*I. Using this procedure, we have generated plasmids expressing HSV-TK, hIL-1α, rat IL-3, luciferase or LacZ. The unique *Srf*I and *Not*I sites in the pBS.Eco-Eco/ad5Δ HIII. Δgp19K plasmid (with or without inserted gene of interest) are used to transfer the region comprising the gene of interest into the corresponding region of pBr/Ad.Bam-rITR, yielding construct pBr/Ad.Bam-rITRΔgp19K (with or without inserted gene of interest). This construct is used as described *supra* to produce recombinant adenoviruses. In the viral context, expression of inserted genes is driven by the adenovirus E3 promoter.

Recombinant viruses that are both E1 and E3 deleted are generated by a double homologous recombination procedure for E1-replacement vectors using a plasmid-based system consisting of:

a) an adapter plasmid for E1 replacement according to the invention, with or without insertion of a first gene of interest,
b) the pWE/Ad.AflII-EcoRI fragment, and
c) the pBr/Ad.Bam-rITRΔgp19K plasmid with or without insertion of a second gene of interest.

In addition to manipulations in the E3 region, changes of (parts of) the E4 region can be accomplished easily in pBr/Ad.Bam-rITR. Moreover, combinations of manipulations in the E3 and/or E2A and/or E4 region can be made. Generation and propagation of such vectors, however, demands packaging cell lines that complement for E2A and/or E4 *in trans.*

*C. Generation of E1 deleted recombinant Ad vectors that possess an attenuated E4 region in PER.C6/tTA cells*

Recombinant viruses that are E1 deleted and harbor a synthetic E4 promoter region are generated by a homologous recombination procedure as described above for E1-replacement vectors using a plasmid-based system consisting of:

a) an adapter plasmid for E1 replacement according to the invention, with or without insertion of a first gene of interest,
b) pWE/Ad.AflII-rITR.tetO-E4

Generation and propagation of such viruses, e.g., IG.Ad/LacZΔ E1tetO-E4 is done in the appropriate complementing cells, i.e. PERC.6/tTA cells. Several different recombinant adenoviruses, comprising the bacterial LacZ gene (IG.Ad. AdApt.LacZ and IG.Ad.ULIP.LacZ) have been produced using this protocol (see table I).

*D. Generation of recombinant Ad deleted for early region 1 and early region 2A and attenuated for E2B and/or E4*

Recombinant adenoviral vectors from which both E1 and E2A are deleted, and which possess E2B and/or E4 regions under transcriptional control of a synthetic promoter are generated by homologous recombination as described above using a combination of the following plasmid DNAs:

a) An adapter plasmid for replacement of E1, e.g., pULIP-LacZ, pAdApt-LacZ, pEF-1α-LacZ.
b) pWE/Ad.AflII-rITRΔE2AtetO-E4; pWE/Ad.AflII-rITRΔE2AtetO-E2B; pWE/Ad.AflII-rITRΔE2AtetO-E2BtetO-E4

Generation and propagation of such viruses is done in the appropriate complementing cells, i.e. PER/E2A/tTA cells. Several different recombinant adenoviruses, comprising attenuated E2B or E4 have been produced using this protocol (see table II).

*E. Growth of Ad vectors comprising attenuated E2B or E4 in cells* that *do not express tTA*

A selection of recombinant Ad vectors, i.e. IG.Ad.AdApt.LacZ.ΔE2AtetO-E4, IG.Ad.ULIP.LacZ.ΔE2AtetO-E4, IG.Ad.ULIP.LacZ.tetO-E2B, and IG.Ad.ULIP.LacZ.ΔE2A (control virus), that were generated by the procedure described above, were tested for their ability to replicate in PER/E2A cells that do not express tTA. The growth of these viruses in PER/E2A/tTA cells was analyzed in parallel. Table III shows that the growth of IG.Ad.AdApt.LacZ.ΔE2AtetO-E4, IG.Ad.ULIP.LacZ.ΔE2AtetO-E4, IG.Ad.ULIP.LacZ.tetO-E2B was drastically impaired in PER/E2A cells whereas these viruses can grow well in PER/E2A/tTA cells. This effect is not due to differences in susceptibility of these cell lines for the virus since the control virus, IG.Ad.ULIP.LacZ.ΔE2A, did grow very well in PER/E2A cells. Together, this indicated that the E2B and E4 attenuated viruses are strongly disabled in replication in the absence of tTA despite the fact that all other components necessary for replication were available. Together, this result indicates that the attenuation of the respective gene regions (E2B and E4) according to the invention has been successful.

## Example 5

**Biological activity of IG.Ad/ΔE1tetO-E4, IG.Ad/ΔE1ΔE2AtetO-E4, IG.Ad/ΔE1 ΔE2AtetO-E2B, and IG.Ad/ΔE1ΔE2AtetO-E2BtetO-E4 vectors *in vitro* and *in vivo.***

*A. Biological activity of IG.Ad/ΔE1tetO-E4, IG.Ad/ΔE1Δ E2AtetO-E4, IG.Ad/ΔE1ΔE2AtetO-E2B, and IG.Ad/ΔE1ΔE2AtetO-E2BtetO-E4 vectors in vitro*

In order to demonstrate that E1 or E1+E2A deleted recombinant Ad vectors with conditionally disabled E2B and/or E4 genes express reduced levels of E2B and/or E4 genes, in mammalian and/or human cells, the following experiment is performed: HeLa cells (ATCC CCL-2) or A549 cells are seeded at $1x10^6$ cells per tissue culture plate (Greiner) in DMEM (Gibco BRL) supplemented with 10% FBS (Gibco BRL) in a 10% $CO_2$ atmosphere at 37°C. The next day, cells are inoculated with 0, 10, 100, 1000 or 10,000 virus particles of IG.Ad/LacZ ΔE1tetO-E4, IG.Ad/LacZΔE1ΔE2AtetO-E4, IG.Ad/LacZΔE1ΔE2AtetO-E2B or IG.Ad/LacZΔE1ΔE2AtetO-E2BtetO-E4 per cell. As a control, parallel cell-cultures are inoculated with 10, 100, 1000, or 10,000 virus particles of IG.Ad/LacZΔE1 or IG.Ad/LacZΔE1ΔE2A. Forty-eight hours post inoculation, cells can be either assayed for viral gene (E2, E4 and late genes) expression or for LacZ expression.

The LacZ transducing efficiency is determined as follows: Infected cells are washed twice with PBS (NPBI) and fixed for 8 minutes in 0.25% glutaraldehyde (Sigma) in PBS. Subsequently, the cells are washed twice with PBS and stained for 8 hours with X-gal solution (1 mg/ml X-gal in DMSO (Gibco), 2mM $MgCl_2$ (Merck), 5mM$K_4$ $[Fe(CN)_6]$. $3H_2O$ (Merck) , 5mM $K_3[Fe(CN)_6]$ (Merck) in PBS. The reaction is stopped by removal of the X-gal solution and washing of the cells with PBS.

Expression of viral genes is assayed by Western blot analysis using E2B, E4 and viral late protein specific antibodies using the ECL (Amersham) detection system as described by the manufacturer.

*B. Longevity of transgene-expression from E1 or E1+E2A deleted recombinant Ad vectors possessing attenuated E2B and/or E4.*

In order to study whether the replacement of the native E2B and/or E4 promoter with the synthetic promoter increases the longevity of expression of transgene from the recombinant Ad vectors *in vivo,* the following experiments are performed. A total of $10^8$ or $10^9$ virus particles of either IG.Ad/LacZΔ E1, IG.Ad/LaCZΔE1ΔE2A, IG.Ad/LacZΔE1tetO-E4, IG.Ad/LacZΔE1Δ E2AtetO-E4, IG.Ad/LacZΔE1ΔE2AtetO-E2B, IG.Ad/LacZΔE1Δ E2AtetO-E2BtetO-E4 is injected into the tail vein of 8 weeks old C57/B16 or NOD-SCID mice. At day 7, 14, 28, and 56, two mice per group are sacrificed, the livers of these mice are isolated and fixed in formalin. Thin slices are cut and extensively washed in PBS. Subsequently, the slices are stained in X-gal solution (1 mg/ml X-gal in DMSO (Gibco), 2mM $MgCl_2$ (Merck), 5mM $K_4[Fe(CN)_6].3H_2O$ (Merck), 5mM $K_3[Fe(CN)_6]$ (Merck) in PBS. After an 8-hour incubation, the samples are washed in PBS. The longevity of LacZ expression from the different Ad vectors will thus be assayed. It is expected that the expression of LacZ in C57/B16 mice is prolonged when replication-conditioned Ad vectors were used instead of the conventional IG/Ad.LacZΔE1 and IG/Ad.LacZΔE1ΔE2A vectors. In contrast, the LacZ expression in the livers of immune-deficient NOD-SCID mice is expected to be stable in all cases.

Example 6

**Residual E4 gene expression from IG.Ad/ΔE1, IG.Ad/ΔE1ΔE2A, IG.Ad/ΔE1tetO-E4, and IG.Ad/ΔE1ΔE2AtetO-E4 vectors in vitro**

In order to demonstrate that E1- or E1+E2A-deleted recombinant Ad vectors with conditionally disabled E4 genes express reduced levels of E4 genes in mammalian and/or human cells, the following experiment was performed: A549 cells were seeded at $1x10^6$ cells per 10 $cm^2$ tissue culture dish (Greiner) in DMEM (Gibco BRL) supplemented with 10% FBS (Gibco BRL), and incubated in a 10% $CO_2$ atmosphere at 37°C. The next day, the cells were inoculated with 1000 virus particles of IG.Ad/AdAptLucΔE1, IG.Ad/AdAptLucΔE1ΔE2A, IG.Ad/AdAptLucΔE1teto-E4, or IG.Ad/AdAptLucΔE1ΔE2AtetO-E4. The cells were harvested at 30 h post-inoculation by lysis in 100 μl RIPA buffer (PBS + 1% NP40 + 0.5% deoxycholic acid + 0.1% SDS + protease inhibitor cocktail). After 15 min incubation on ice, the cell lysates were spun for 15 min at 14,000 rpm, 4°C in an eppendorf centrifuge. The total protein concentration in the supernatants was thereafter determined using the Bio-Rad DC Protein Assay. The (relative) amounts of the E4-orf6 (34kD) protein present in the cell extracts were determined by western blot analysis. For this purpose, equal amounts (30 μg) of total protein from the cleared cell extracts were run in an SDS-polyacrylamide gel and thereafter blotted onto an Immobilon-P membrane (Millipore). This membrane was processed by incubation with a rabbit E4-orf6 specific anti-peptide antiserum (first antibody, 1:2000 diluted; Boivin et al., 1999) and a blotting grade affinity purified Goat anti-Rabbit IgG (H+L)-HRP (secondary antibody, 1:7500 diluted; Biorad). The E4-orf6 protein was eventually visualized using the ECL Plus™ Western blotting detection reagents (Amersham Pharmacia Biotech) according to the manufacturer's recommendations. The data in Fig.8 and 9 clearly show that the E1- and E1+E2A-deleted Ad vectors with conditionally disabled E4 genes produced significantly less of the E4-orf6 protein than E1- or E1+E2A-deleted vectors possessing wt E4. This indicates that the expression of E4, in non-complementing cells, i.e., normal mammalian and/or human cells, is significantly reduced by the replacement of the native E4 promoter by the tet operon.

To demonstrate that the reduced expression of E4 was not simply due to a difference in transduction efficiency of the various vectors, a semi-quantitative southern blot analysis of the cell-associated viral genomes was performed. Therefore, total DNA was harvested from the infected cells at 30 h post-inoculation by using the Easy-DNA kit (Invitrogen) according to the manufacturer's recommendations. Ten μg of each DNA sample was digested with BamHI, and run in a 0.75% agarose gel. The DNA was thereafter blotted onto a Hybond™ N+ nylon transfer membrane (Amersham Pharmacia Biotech) and probed with a HindIII-NheI (484 bp) fragment of the Ad5 fiber gene that was labeled with $^{32}P$-CTP using the Rad Prime RTS System (GIBCO). Although some variation in the amount of vector DNA could be observed (Fig.10), it is clear that the reduced expression of E4 from IG.Ad/AdApt.LucΔE1tetO-E4, and IG.Ad/AdAptLucΔE1ΔE2AtetO-E4 cannot be explained by inefficient transduction. For example, despite the relatively low abundance of viral genomes of the E1+E2A deleted vector, this vector produced significantly more E4-orf6 protein than the vectors possessing conditionally disabled E4. Taken together, these results provide evidence that the attenuation of E4 according to the invention leads to a significant reduction of E4 expression in normal mammalian and/or human cells.

Example 7

**Residual E2 gene expression from IG.Ad/ΔE1, IG.Ad/ΔE1ΔE2A, IG.Ad/ΔE1ΔE2AtetO-E4, and IG.Ad/ΔE1ΔE2AtetO-E4 vectors in vitro**

In order to demonstrate that E1- or E1+E2A-deleted recombinant Ad vectors with conditionally disabled E4 genes express reduced levels of the E2A (DBP) gene in mammalian and/or human cells, the following experiment was performed: A549 cells were seeded at a density of $1x10^6$ cells per 10 $cm^2$ tissue culture dish (Greiner) in DMEM (Gibco BRL) supplemented with 10% FBS (Gibco BRL), and incubated in a 10% $CO_2$ atmosphere at 37°C. The next day, the cells were inoculated with 1000 virus particles of IG.Ad/AdAptLucΔE1, IG.Ad/AdAptLUCΔE1ΔE2A, IG.Ad/AdAptLucΔE1ΔE2AtetO-E4, or IG.Ad/AdAptLucΔE1ΔE2AtetO-E4. At 30 h post-inoculation, the cells were harvested as described supra and the cell extracts were analyzed for the presence of the E2A protein DBP by the western blot assay as described supra, except that the membrane was processed by incubation with the anti-DBP monoclonal antibody B6 (first antibody, 1:1000 diluted; Reich et al., 1983) and a blotting grade affinity purified Goat anti-Mouse IgG (H+L)-HRP (secondary antibody, 1:7500 diluted; Biorad).

The results (Fig.11) clearly show that the E1-deleted Ad vector with conditionally disabled E4 produced significantly reduced amounts of the DBP protein in comparison to the E1-deleted vector possessing wt E4. As expected, the E1+E2A-deleted vectors produced no DBP protein. Taken together, these results provide evidence that the attenuation of E4 according to the invention leads to a significant reduction of E2A expression in normal mammalian and/or human cells.

B) In order to demonstrate that E1- or E1+E2A-deleted recombinant Ad vectors with conditionally disabled E4 genes express reduced levels of E2B genes in mammalian and/or human cells, the following experiment was performed: A549 cells were seeded at a density of $1x10^6$ cells per 10 $cm^2$ tissue culture dish in DMEM supplemented with 10% FBS, and incubated in a 10% $CO_2$ atmosphere at 37°C. The next day, the cells were inoculated with 1000 and 10000 virus particles of IG.Ad/ΔE1, IG.Ad/ΔE1ΔE2A, or IG.Ad/ΔE1ΔE2AtetO-E4. The cells were harvested at 30h post-inoculation and the relative amount of the E2B (p)TP protein present in the cell extracts was determined by Western blot analysis as described above except that this time, a mixture (1:1:1) of three antibodies against (p)TP and Pol (kind gift of P. C. van der Vliet, Utrecht, the Netherlands) was used as primary antiserum (1:500 diluted). The data shown in Fig.12 clearly demonstrate that the E1- and E1+E2A-deleted Ad vectors with conditionally disabled E4 genes produced significantly reduced amounts of the pTP protein than E1 or E1+E2A deleted vectors possessing wt E4.

Example 8

**Residual late gene expression from IG.Ad/ΔE1, IG.Ad/ΔE1ΔE2A, IG.Ad/ΔE1ΔE2AtetO-E4, and IG.Ad/ΔE1ΔE2AtetO-E4 vectors in vitro.**

In order to demonstrate that E1-deleted Ad vectors with conditionally disabled E4 genes express reduced levels of late genes in mammalian and/or human cells, the following experiment was performed: A549 cells were seeded at $1x10^6$ cells per 10 $cm^2$ tissue culture dish in DMEM supplemented with 10% FBS, and incubated in a 10% $CO_2$ atmosphere at 37°C. The next day, the cells were inoculated with 1000 virus particles of IG.Ad/AdAptLucΔE1, IG.Ad/AdAptLucΔE1ΔE2A, IG.Ad/AdAptLucΔE1ΔE2AtetO-E4, or IG.Ad/AdAptLucΔE1ΔE2AtetO-E4.
The cells were harvested at 72 h post-inoculation and the relative amount of the fiber protein present in the cell extracts was determined by western blot analysis as described above except that the polyclonal E641/3 anti-knob domain of fiber (primary antibody, 1:5000 diluted; kind gift of R. Gerard, Leuven, Belgium) and the blotting grade affinity purified Goat anti-Rabbit IgG (H+L)-HRP (secondary antibody, 1:100000 diluted; Biorad) were used. The results in Fig.13 clearly show that the E1- and E1+E2A-deleted Ad vectors with conditionally disabled E4 genes, as well as the vector that was deleted of E1+E2A produced significantly reduced amounts of fiber protein than the vector that was deleted of E1 only. This shows that attenuation of the expression of the E4 genes by itself causes a reduction in late gene expression, a phenomenon that is also seen in Ad vectors that are deleted of E2A.

Example 9

Attenuation of E4 expression leads to a diminished liver toxicity in vivo.

To demonstrate that Ad vectors with conditionally disabled E4 are less toxic in vivo than Ad vectors possessing wt E4, mice are injected intravenously via the tail vein with 1E11 vp of IG.Ad/ΔE1, IG.Ad/ΔE1ΔE2A, IG.Ad/ΔE1tetO-E4, IG.Ad/ΔE1Δ E2AtetO-E4, or with PBS/0.5% sucrose only. For this purpose, as a non-limiting example, BALB/c, C57BL/6 and C3H mice are used. Ten mice per vector and per time-point are used. All vectors are suspended in PBS/0.5% sucrose, and except for modifications in the E2A and E4 regions, these vectors are genetically identical and lack a transgene and accompanying transcription elements to avoid any unintentional effect of a transgene and/or accompanying transcription elements on liver toxicity. The mice are sacrificed on day 3, 14, 28, 56 and 90 after injection, weighed and the livers of these mice are isolated and weighed as well. Removal of liver: liver and gall bladder are removed. Of each liver, a part of the anterior and posterior right lobe is cut-off with a clean scalpel and snap frozen in liquid nitrogen and processed for real-time PCR or Southern blot analysis (to check the transduction efficiency). The remaining liver lobes (median, left and caudate) are fixed in ample buffered formalin. The median lobe (with biliary cyst) and left lobe are trimmed at their largest cross section for HDS staining. The livers are examined histologically. Liver lesions, such as vacuolar change, apoptosis, dense nuclei, inclusions, mitotic increase, anisonucleosis, megalocytosis, and inflammation in peri-portal and sinusoidal areas are scored semi-quantitatively. In addition, blood is sampled for blood cell counts (erys, leucocytes, thrombocytes) and plasma is taken for biochemical measurement of ALAT, ASAT, AP, gamma-GT, ALB and TBIL. All procedures are executed according to procedures very well known to persons skilled in the art.

Example 10

The effect of the attenuation of E4 on the activity of the CMV promoter driving a transgene is cell-type specific

In order to determine the effect of the attenuation of E4 on the activity of the CMV promoter the following experiments have been performed. A549 cells were seeded at $1x10^6$ cells per 10 $cm^2$ tissue culture dish in DMEM supplemented with 10% FBS, and incubated in a 10% $CO_2$ atmosphere at 37°C. The next day, the cells were inoculated with 1000 virus particles of IG.Ad/AdAptLucΔE1, IG.Ad/AdAptLUCΔE1ΔE2A, IG.Ad/AdAptLucΔ E1tetO-E4, or IG.Ad/AdAptLucΔE1ΔE2AtetO-E4. Notably, all these vectors contain the luciferase gene under the control of the CMV promoter. The cells were harvested by detergent-mediated lysis at 48 h post-inoculation and the luciferase activity in the cell extracts was measured and expressed in RLU (relative light units) using the Luciferase Assay System (Promega) according to the manufacturer's recommendation. The RLU was normalized to the total amount of protein in the cell extracts, which was measured by using the BioRad DC Protein Assay. The results, as shown in Fig.14, indicate that the Ad vectors possessing conditionally disabled E4 produced significantly less luciferase than the vectors possessing wt E4. This implies that the CMV promoter that drives the expression of the luciferase gene was less active in vectors possessing conditionally disabled E4 than in vectors possessing wt E4.
To find out whether the inhibitory effect of the attenuation of E4 on the CMV promoter activity also occurs in other cell types, an experiment, similar to the one described above, was done using primary human endothelial cells. These cells were, for this purpose, inoculated with 1000 vp of IG.Ad/AdAptLucΔ E1 or 1000 vp of IG.Ad/AdAptLucΔE1ΔE2AtetO-E4. Forty-eight hours after inoculation, cell extracts were made and examined as described above. The results (Fig. 15) show that similar amounts of luciferase were found in cells infected with the Ad containing wt E4 and the one possessing conditionally disabled E4. This indicates that the activity of the CMV promoter is not impaired by the attenuation of E4 in primary human epithelial cells. From these experiments, it is concluded that the effect of the attenuation of E4 on the activity of the CMV promoter is cell type specific.

Example 11

The influence of E4 in transgene expression over longer periods of time in lung-, liver and breast tumor derived cell lines

To determine whether E4-expression plays a role in transgene expression from the CMV-promoter over longer periods of time in lung and liver derived cell-lines, the following infection- and subsequent luciferase activity experiments were performed. As a part of these experiments, total protein content was also determined.

***Transduction of A549 (lung derived) cells***

At day 1, A549 cells were seeded in 96-well plates with a density of 10,000 cells/well in a volume of 100 µl DMEM+10% heat-inactivated FBS and incubated in a humidified $CO_2$ incubator set at 37°C and 10% $CO_2$. At day 2, the cells were transduced with viruses derived from PER/E2A/tTA cells transfected with the adapter plasmid pAdApt-luc in combination with pWE/Ad.AflII-rITRΔE2AtetO-E4 or derived from PER.C6/E2A cells that were transfected with pAdApt-Luc in combination with pWE/Ad.AflII-rITRΔE2A. In this transduction experiment crude lysates as well as purified viruses were used. For both types of preparations the number of virus particles (vp) per ml were determined.

Infections were performed for 5 time points in quadruplate per time point. The Multiplicities of Infections (MOI's) were 5,000 and 50,000 vp/cell in a total volume of 150 µl. After transduction, the cells were again incubated in a humidified $CO_2$ incubator set at 37°C and 10% $CO_2$. At day 3, cells in plate for time point "24 hour" were washed with 100 µl PBS and lysed with 100 µl lysisbuffer (8 mM $MgCl_2$, 1 mM EDTA, 1 mM DTT, 1% v/v Triton X-100 and 15% v/v glycerol). Then the plate was frozen at -20°C until luciferase- and total protein assays could be performed.
At day 4, cells in plate for time point "48 hour" were washed, lysed and stored according to the protocol described supra. Medium of plates for time points "72 hour", "96 hour" and "168 hour" was removed and new medium was added, to avoid re-infection. At day 5, 6 and 9, cells in plates for respectively time points "72 hour", "96 hour" and "168 hour" were washed, lysed and stored according the protocol described supra.
Luciferase experiments were performed as follows. After thawing, the plates were centrifuged for 5 min at 1500 rpm and put on ice. Luciferase expression was determined with a luminometer [EG&G Berthold]. For this, 20 µl sample was put in an appropriate tube after which the machine added 100 µl luciferase assay substrate buffer (luciferase assay substrate dissolved in 10ml luciferase assay buffer [Promega Catno. E1501]). Some samples were diluted in lysisbuffer because expression was too high to measure.
To correct for total protein quantity in the samples, the CBQCA protein quantitation assay (Molecular Probes. Catno. C-6667) was performed according the manufacturers protocol. For all samples, 5 µl was used in the assay.
Fig.16 shows the results of the transduction of the A549 cells. These data show that the ΔE2A.tetO-E4 viruses (normal lines) give about a 100 fold lower expression over time as compared to the ΔE2A viruses (dashed lines). There is no clear decrease in luciferase expression for each virus separately over time. After 168 hours the level of luciferase activity using these different viruses (purified or crude) is comparable to levels detected after 24 and 48 hours.

**Transduction of HepG2 (liver derived) cells**

Transduction, luciferase and protein content determination experiments for HepG2 cells were performed according to the protocol described supra for A549 cells, with the following two exemptions. HepG2 cells were seeded in 96-well plates with a density of 22,500 cells/well (100 µl) and the MOI's that were used were 30 and 300 vp/cell in a total volume of 150 µl.
Fig.17 shows the luciferase activity results obtained after the transduction of the HepG2 cells. The results suggest that expression over time of the ΔE2A.tetO-E4 (normal lines) and the ΔE2A viruses (dashed lines) are comparable. The luciferase activity derived from both viruses apparently increase over time.

**Transduction of MCF-7 (breast cancer derived) cells**

Transduction, luciferase and protein content determination experiments for MCF-7 cells were performed according to the protocol described supra for A549 cells, with the exemption that the medium used was DMEM containing 10% non-heat inactivated FBS.
Fig.18 shows the luciferase activity results obtained after the transduction of the MCF-7 cells. These results suggest that expression over time of the ΔE2A.tetO-E4 (normal lines) and the ΔE2A viruses (dashed lines) are also comparable. The result obtained after the ΔE2A virus infection using a crude lysate with an MOI of 50,000 vp/cell, suggest that there is a drop in expression level after 72 hours. The results obtained after infection with ΔE2A.tetO-E4 using crude lysates and purified viruses show a slight decrease in luciferase activity after 96 hours.

Example 12

**Genomic analysis of Ad vectors with conditionally disabled E4**

The genomic identity of Ad vectors possessing conditionally disabled E4 was confirmed by Southern blot analysis of the vector genome. For this purpose, the viral genomic DNA was isolated from purified IG.Ad/ΔE1ΔE2A and IG.Ad/ΔE1ΔE2AtetO-E4 virus particles. Therefore, 100 µl virus suspension containing $1.4x10^{11}$ - $3x10^{11}$ virus particles was mixed with 18 µl buffer (50 mM $MgCl_2$, 1.2 mM $CaCl_2$, and 130 mM Tris pH 7.5), 9 µl DNaseI (10 mg/ml), and 3 µl $H_2O$. This mixture was incubated for 30 min at 37°C after which 3.6 µl EDTA (0.5 M), 4.5 µl SDS (10%), and 1.5 µl Proteinase K (20 mg/ml) was added. The mixture was then incubated for 1 h at 50°C. The viral DNA was then purified from the mixture using the GeneClean Kit and cut with PstI. Equal amounts of DNA purified from different vectors were run in a 1% agarose gel and blotted onto a Hybond™-N+ nylon transfer membrane and probed with a 313 bp HindIII/NcoI fragment of pNEB-PaSe.tetO. This fragment corresponds to the tet operon sequence and was labeled with $^{32}$P-CTP using the Rad Prime RTS System (GIBCO). The data in Fig.19 show that only a fragment of the genomic DNA of IG.Ad/ΔE1ΔE2AtetO-E4 was labeled whereas the genomic DNA of IG.Ad/ΔE1ΔE2A was unlabeled. This indicates that only the genomic DNA of IG.Ad/ΔE1ΔE2AtetO-E4 contained the tet operon sequence. The size of the labeled fragment fits the theoretic length of the genomic *Pst*I fragment (2203 bp) containing the rITR and sequences of E4 with the tet operon. From this result, it is concluded that IG.Ad/ΔE1ΔE2AtetO-E4 truly possesses a tet operon in place of the native E4 promoter.

Example 13

Serum-free suspension cultures of PER/E2A/tTA and PER.C6/tTA cells.

To obtain a serum-free suspension culture of PER/E2A/tTA cells (clone 1A1, pn 11), $5x10^6$ cells were thawed and cultured in ExCell 525 medium (JRH Biosciences) supplemented with 4mM L-glutamin with or without the addition of Hygromycin (100 ug/ml). Cells were cultured in T175 flasks. The concentration of Hygromycin is identical to what is used for adherent cultures. However, since the the ExCell 525 medium does not support the culturing of PER.C6 cells and derivatives thereof in the presence of Hygromycin, culturing was continued only with cultures in the absence of the Hygromycin selection pressure. Cultures that were kept in the presence of Hygromycin died after 8 days.
Culture conditions in the absence of Hygromycin were as follows: Passage of the cells was done after 2 or 3 days of incubation. A sample of the cells was subsequently taken and counted and stained for determination of the cell density and viability of the cultures. Then, cultures were passed to a new flask or to a roller bottle or diluted in the flask in which the culture was kept. Following this, the culture was sub-cultured to $2x10^5$ or to $3x10^5$ viable cells per ml and left for another incubation period of 3 or 2 days, depending on the concentration of cells.
The cell cultures were further incubated at 37°C or at 39°C. The standard temperature for incubating adherent cell cultures that contain a temperature sensitive E2A gene (like PER/E2A/tTA) is 39°C. The static cultures (in flasks) were incubated at 39°C, while the dynamic cultures in roller bottles were incubated at 37°C.
After 13 population doublings a serum free suspension cell bank of $5x10^6$ cells/vial was established. After 11 and 15 population doublings, infections using recombinant LacZ expressing and E4-attenuated adenovirus in roller bottles were performed. Roller bottles contained 100 ml suspension cultures with a cell density of approximately $1x10^6$ viable cells per ml. These infections yielded functional LacZ expressing virus as was determined after re-infection of the supernatants on A549 cell cultures and subsequent staining of the cells.
The dynamic serum free suspension culture was maintained in roller bottles for 61 days, with a total of 44 population doublings, which results in an average population doubling time of 33 hours. The static serum free suspension culture in the flasks was maintained for 34 days, with a total of 19 population doublings, which results in an average population doubling time of 42 hours.
Two 2-liter bio-reactor runs were also performed. In the first bio-reactor a run was performed with cells that were kept in the reactor for 8 days with a final density of approximately $4x10^6$ cells per ml using standard perfusion conditions known to persons skilled in the art.
In the second bio-reactor, cells were cultured for 4 days after which a virus infection was performed while the cell density was approximately $1x10^6$ cells per ml. For this an MOI of 70 virus particles per cell was applied with purified attenuated-E4 recombinant adenovirus. After 4 days of infection the bio-reactor run was terminated and the cells and medium were harvested.
The experiments to obtain a serum free suspension culture of PER.C6/tTA were similarly executed. For this $5x10^6$ PER.C6/tTA cells (clone 2C5 pn 8) were cultured in ExCell 525 medium supplemented with 4 mM L-glutamin. These cells adapted to serum free suspension medium after 2 passages after thawing.
Culture conditions were as follows: Passaging of the cells was done after 2 or 3 days of incubation. A sample of the cell culture was used for counting and staining for determination of the cell density and viability of these cultures. The culture was then passed to a roller bottle or diluted in the same flask as it was cultured in. The culture was diluted to $2x10^5$ or $3x10^5$ viable cells per ml for an incubation period of 3 or 2 days respectively. All roller bottle (dynamic) cultures were incubated at 37°C and flushed with $CO_2$.
After 18 population doublings an infection in roller bottles containing $5x10^5$ viable cells per ml was performed. The infection was performed with an attenuated-E4 recombinant adenovirus. The obtained virus titer from this infection (taken three days after infection) was $1.2x10^{10}$ virus particles per ml. This number equals 24,000 produced virus particles per cell (seeded cells).
After 22 population doublings a serum free suspension cell bank of $5x10^6$ cells per vial was established. This was performed after 27 days of culturing resulting in an average population doubling time of 29 hours.
Two 2-liter bio-reactor runs were also performed with the PER.C6/tTA cells. In the first bio-reactor cells were cultured for 8 days and reached a density of approximately $4x10^6$ cells per ml using standard perfusion known to persons skilled in the art. In the second bio-reactor cells reached a density of approximately $3x10^6$ cells per ml and were subsequently infected with a concentrated batch of recombinant attenuated-E4 adenovirus that was derived from the infected roller bottle cultures described supra. An MOI of 70 virus particles per cell was applied, using standard perfusion methods. The culture was kept at 37°C. Three days after infection the virus yield in crude samples taken from the bio-reactor was measured. The concentration of this virus stock was $7x10^{10}$ virus particles per ml. This equals a production rate of approximately 23,000 virus particles per cell.

Example 14

Plaque purification, propagation, and titration of Ad vectors comprising conditionally disabled E4

A.

Plaque purification: IG.Ad/ΔE1tetO-E4 and IG.Ad/ΔE1ΔE2AtetO-E4 vectors were plaque-purified by using PER.C6/tTA and PER/E2A/tTA cells, respectively. For this purpose, cells were seeded in 6-wells plates at a density of $1.5x10^6$ cells per 10 $cm^2$ well in DMEM + 10% FBS + 10 mM $MgCl_2$. After a 4 h incubation at 37°C, 10% $CO_2$ (PER.C6/tTA) or 39°C, 10% $CO_2$ (PER/E2A/tTA) the medium was replaced by 1 ml of inoculation medium. The inoculation medium consisted of IG.Ad/ΔE1tetO-E4 or IG.Ad/ΔE1ΔE2AtetO-E4 vectors that had been diluted in DMEM + 10% FBS + 10 mM $MgCl_2$. Usually, 10-fold dilutions were made ranging from $10^{-4}$ to $10^{-9}$. The cells were incubated in inoculation medium o/n at 37°C, 10% $CO_2$ (PER.C6/tTA) or 34°C, $CO_2$ (PER/E2A/tTA). Thereafter, the inoculation medium was removed and the cells were washed with PBS and overlaid with 3 ml MEM + 10 mM $MgCl_2$ + 2.5% agarose + 2-5% FBS per well. The cells were further incubated at 37°C, 10% $CO_2$ (PER.C6/tTA) or 34°C, 10% $CO_2$ (PER/E2A/tTA). Eleven to 14 days later, independent, free plaques were picked using a 20-µl pipette. Twenty µl of the plaque material was resuspended in 200 µl DMEM + 10% FBS + 10 mM $MgCl_2$, and frozen at -20°C.

B.

Propagation of plaque-purified vectors: the above-described resuspended plaque material containing IG.Ad/ΔE1tetO-E4 or IG.Ad/ΔE1ΔE2AtetO-E4 was thawed and 100 µl of each plaque material was mixed with 900 µl of DMEM + 10% FCS + 10 mM $MgCl_2$, and used to inoculate sub-confluent monolayers of PER.C6/tTA or PER/E2A/tTA cells in 2.5 $cm^2$ wells, respectively. Cells were incubated at 37°C, 10% $CO_2$ (PER.C6/tTA) or at 34°C, 10% $CO_2$ (PER/E2A/tTA). Full CPE usually occurred after 4 to 7 days. Thereafter, the cells were scraped from the dish and harvested together with the medium. The cell/medium suspension was then frozen at -20°C. After thawing, 0.5 ml of this suspension was used to inoculate sub-confluent monolayers of fresh cells in 80 $cm^2$-culture flasks (Nunc) for further amplification. This procedure could be repeated and scaled up to large-scale vector propagations. High yields (more than 25,000 vp per cell) of progeny vectors were obtained when fresh cells were inoculated at an MOI of 50-200 vp per cell.

C.

Determination of vector titers: PER.C6/tTA and PER/E2A/tTA cells were used to determine the infectious titer of batches of IG.Ad/ΔE1tetO-E4 and IG.Ad/ΔE1ΔE2AtetO-E4, respectively. For this purpose, cells were seeded in 96-wells at a density of $4x10^4$ cells per well in DMEM + 10% FBS + 10 mM $MgCl_2$. The medium was replaced after a 4-hr incubation at 37°C, 10% $CO_2$ (PER.C6/tTA) or 39°, 10% $CO_2$ (PER/E2A/tTA) by 200 µl of serial dilutions (made in DMEM + 10% FBS + 10 mM $MgCl_2$) of IG.Ad/ΔE1tetO-E4 or IG.Ad/ΔE1ΔE2AtetO-E4, respectively. Cells were further incubated at 37°C, 10% $CO_2$ and 34°C, 10% $CO_2$, respectively, and CPE was monitored every 3-4 days. After 14-16 days, the titer of the vectors was calculated from the highest dilution of vector that gave CPE.

Example 15

Stability of PER/E2A/tTA cells in producing E1+E2A-deleted Ad vectors that are conditionally disabled in E4

PER/E2A/tTA (clone 1A1) cells were kept in culture for at least 100 passages in medium containing the selection drug hygromycin (100 µg/ml). Routinely, the cells were split (1:3 - 1:5) twice per week. To verify that PER/E2A/tTA cells maintain their capacity to efficiently support the propagation of E1+E2A-deleted, E4-attenuated Ad vectors the propagation of such vectors was compared in PER/E2A/tTA cells that had been kept in culture for a long and short period. For this purpose, 2.5 x $10^6$ cells PER/E2A/tTA cells at passage 17 and at passage 64 were seeded in a 25 $cm^2$ culture flask (Nunc) in DMEM + 10% FBS + 10 mM $MgCl_2$, and incubated at 39°C, 10% $CO_2$. The next day, the cells were inoculated with IG.Ad/AdAptLacZΔE1ΔE2AtetO-E4 at an MOI of 200 vp/cell. After a 5-days incubation at 34°C and 10% $CO_2$, the progeny vectors were harvested by freeze-thawing the cells and medium. Thereafter, 10-fold dilutions, ranging from $10^{-1}$ to $10^{-6}$, of the progeny vectors were made in DMEM + 10% FBS, and used to inoculate sub-confluent monolayers of A549 cells in 2.5 $cm^2$ wells. After a 48 h incubation at 37°C, the cells were washed twice with PBS and fixed for 8 min with 1% formaldehyde, 0.2% glutar(di)aldehyde in PBS. The cells were thereafter stained with X-gal solution (1 mg/ml X-gal in DMSO (Gibco), 2 mM $MgCl_2$ (Merck), 5 mM $K_4[Fe(CN)_6].3H_2O$ (Merck), 5 mM $K_3[Fe(CN)_6]$ (Merck) in PBS). The reaction was stopped by removal of the X-gal solution and washing the cells with PBS. The percentage of blue cells was thereafter determined. The results (see Table V) revealed that the progeny vectors derived from PER/E2A/tTA at passage 17 and 64 yielded very similar percentages of blue cells at the various dilutions. This indicates that the yields of progeny vector from PER/E2A/tTA cells at passage 18 and 65 were very similar. This implies that PER/E2A/tTA cells maintain their capacity to support the propagation of E1+E2A-deleted, E4-attenuated vectors over an extensive period of time during which they are kept in culture.

Example 16

Construction of pAd/pIPspAdapt-eGFP, pAd/pIPspAdapt-lacZ, pAd/pIPspAdapt-ceNOS, pAd/pIPspAdapt-hIL3, pAd/pIPspAdapt-hEPO and pAd/pIPspAdapt-LacZ

pAd5/L420-HSA (described in published PCT patent application WO 99/55132) was digested with AvrII and BglII. The vector fragment was ligated to a linker oligonucleotide digested with the same restriction enzymes. The linker was made by annealing oligos of the following sequence: PLL-1 (5'- GCC ATC CCT AGG AAG CTT GGT ACC GGT GAA TTC GCT AGC GTT AAC GGA TCC TCT AGA CGA GAT CTG G-3') and PLL-2 (5'- CCA GAT CTC GTC TAG AGG ATC CGT TAA CGC TAG CGA ATT CAC CGG TAC CAA GCT TCC TAG GGA TGG C-3'). This ligation resulted in pAdMire. Another batch of pAd5/L420-HSA was also digested with AvrII and 5' protruding ends were filled in using Klenow enzyme. A second digestion with HindIII resulted in removal of the L420 promoter sequences. The vector fragment was isolated and ligated separately to a PCR fragment containing the CMV promoter sequence. This PCR fragment was obtained after amplification of CMV sequences from pCMVLacI (Stratagene) with the following primers: CMVplus (5'-GAT CGG TAC CAC TGC AGT GGT CAA TAT TGG CCA TTA GCC-3') and CMVminA (5'-GAT CAA GCT TCC AAT GCA CCG TTC CCG GC-3'). The PCR fragment was first digested with PstI after which the 3'-protruding ends were removed by treatment with T4 DNA polymerase. Then the DNA was digested with HindIII and ligated into the AvrII/HindIII digested pAd5/L420-HSA vector. The resulting plasmid was named pAd5/CMV-HSA. This plasmid was then digested with HindIII and BamHI and the vector fragment was isolated and ligated to the HindIII/BglII polylinker sequence obtained after digestion of pAdMire. The resulting plasmid was named pAdApt.
The full length human EPO cDNA (Genbank accession number: M11319) was cloned, employing oligonucleotide primers EPO-START:5' AAA AAG GAT CCG CCA CCA TGG GGG TGC ACG AAT GTC CTG CCT G-3' and EPO-STOP: 5'AAA AAG GAT CCT CAT CTG TCC CCT GTC CTG CAG GCC TC-3' (Cambridge Bioscience Ltd) in a PCR on a human adult liver cDNA library. The amplified fragment was cloned into pUC18 linearized with BamHI. Sequence was checked by double stranded sequencing. The full length human EPO cDNA containing a perfect Kozak sequence for proper translation was removed from the pUC18 backbone after a BamHI digestion. The cDNA insert was purified over agarose gel and ligated into pAdApt which was also digested with BamHI, subsequently dephosphorylated at the 5' and 3' insertion sites using SAP and also purified over agarose gel to remove the short BamHI-BamHI linker sequence. The obtained circular plasmid was checked with KpnI, DdeI and NcoI restriction digestions that all gave the right size bands. Furthermore, the orientation and sequence were confirmed by double stranded sequencing. The obtained plasmid with the human EPO cDNA in the correct orientation was named pAdApt.EPO and was further digested with HindIII and XbaI restriction enzymes. This EPO insert was isolated and ligated to a HindIII/XbaI digested pIPspAdapt6 plasmid (described in WO 99/64582). The resulting plasmid was named pAd/pIPspAdapt-hEPO. pIPspAdapt6 plasmids carrying human ceNOS (insert HindIII/XbaI), human IL-3 (insert HindIII/BamHI), LacZ (insert KpnI/BamHI) and eGFP (insert HindIII/EcoRI) were generated via pAd5/CLIP (described in WO 99/55132) and according to methods described in detail in WO 99/64582.

Example 17

Miniaturized, multiwell production of E1/E2A deleted and E4 attenuated recombinant adenoviral vectors in PER/E2A/tTA cells

Several different PER/E2A/tTA cell clones were tested for the production of recombinant adenoviral viruses with E1 and E2A deletions in combination with an E4 attenuation. This was performed in 96 well tissue culture plates, to test for usefulness in high-through-put screens in functional genomics.

First set of transfections

At day 1, eighteen different PER/E2A/tTA cell clones were harvested and diluted in culture medium (DMEM+10% Fetal Bovine Serum and 10 mM $MgCl_2$) to a density of 22,500 cells per 100 µl. These suspensions were seeded in two 96-well-tissue-culture plates with 100 µl per well in duplo (one clone in four wells divided over two plates). At day 2, one DNA mix was made for each PER/E2A/tTA clone, by diluting 3.9 µg of SalI linearized pAd/Adapt-LacZ and 3.9 µg of PacI linearized pWE/Ad.AflII-rITRΔE2A.tetO-E4, in 150 µl DMEM. To each DNA mix, 150 µl Lipofectamine mix (38.4 µl Lipofectamine (Life Techn.) + 111.6 µl DMEM) was added. This DNA/Lipofectamine mixture was left at room temperature for 30 min, followed by the addition of 1.95 ml DMEM. The latter mixtures were then added (30 µl per well) to the PER/E2A/tTA cells, after removal of the medium in which the cells were seeded. After 2 hours incubation in a humidified $CO_2$ incubator (39°C, 10% $CO_2$), 170 µl culture medium was added to each well and the plates were returned to the humidified $CO_2$ incubator (39°C, 10% $CO_2$). At day 3, the supernatant in each well was replaced with 200 µl culture medium. The plates were then placed again in another humidified $CO_2$ incubator (34°C, 10% $CO_2$). At day 5, one of the duplo 96-wells plates was used to determine the transfection efficiency using LacZ staining (Table III). The LacZ staining procedures are described in PCT/WO99/64582. The other plate was monitored for CPE formation during a period of three weeks after transfection. In Fig.20 the percentage of CPE positive wells, scored three weeks after transfection, is depicted. These results suggest that it was best to continue with PER/E2A/tTA clones 1A1, 1C1, 1C3, 2B3, 2C3 and 2D5 for 96 well adenoviral production settings, as outlined supra. These clones were used in a second round transfection procedure.

*Second set of transfections*

At day 1, six attached PER/E2A/tTA cell cultures (1A1, 1C1, 1C3, 2B3, 2C3 and 2D5) were harvested and diluted in culture medium (DMEM+10% FBS and 10 mM $MgCl_2$) to a density of 22,500 cells per 100 µl. Subsequently, two 96-well-tissue culture plates for each clone were used to seed 100 µl of the cell suspensions per well. At day 2, three different DNA mixes were prepared for each PER/E2A/tTA clone. 1 µg of a linearized adapter molecule (being either pAd/pIPspAdapt-eGFP, or pAd/pIPspAdapt-lacZ or pAd/Adapt-ceNOS) was mixed with 4 µg of PacI linearized pWE/Ad.AflII-rITRΔE2A.tetO-E4 in 100 µl DMEM. To each mix 100 µl Lipofectamine mix (25.6 µl Lipofectamine (Life Techn.) + 74.4 µl DMEM) was added. This DNA/Lipofectamine mixture was left at room temperature for 30 min, after which 1.3 ml DMEM was added. The latter mixtures were then added on top to the PER/E2A/tTA cells in a total volume of 30 µl per well, after removal of the medium in which the cells were seeded. After 2 hours incubation in a humidified $CO_2$ incubator (39°C, 10% $CO_2$), 170 µl culture medium was added to each well and the plates were placed back in the humidified $CO_2$ incubator (39°C, 10% $CO_2$). At day 3, the medium of each well was replaced with 200 µl culture medium. The plates were then returned to another humidified $CO_2$ incubator (34°C, 10% $CO_2$). At day 5, one of the two 96-wells plates of each clone that was transfected with the LacZ adapter plasmid was used to determine the transfection efficiency using LacZ staining as described supra. Table IV shows the transfection efficiency of each clone. The other plate for the LacZ transfectants and all other plates were monitored for CPE formation during a period of three weeks after transfection. In Fig.21 the percentage of CPE positive wells, scored three weeks after transfection, is depicted.

Example 18

Generation of pBr/Ad.Bam-rITRΔE2AtetO-E4.ΔE3(XbaI) and pWE/Ad.AflII-rITRΔE2AtetO-E4.ΔE3(XbaI)

To construct E3 deleted versions of the vectors carrying a tetO-E4 attenuation, the following cloning steps were performed. pBr/Ad.Bam-rITRΔE2AtetO-E4 was propagated in *E. coli* strain DM1 (*dam*⁻, *dcm*⁻) (Life Techn.). The purified plasmid was digested with *Xba*I, hereby removing the 1.88 kb *Xba*I-*Xba*I insert, and subsequently religated. By removing the *Xba*I-*Xba*I insert the following sequences were deleted: 191 bp of the E3-6.7K protein, the E3-19K glycoprotein, the E3-ADP (10.5K protein), RIDalpha (E3-10.4K protein), RIDbeta (E3-14.6K protein) and the first 21 bp of the E3-14.7K protein (for sequences, see Genbank accession number X03002). The resulting plasmid was named pBr/Ad.Bam-rITRΔE2AtetO-E4.Δ E3(XbaI) and was used to construct helper cosmid pWE/Ad.AflII-rITRΔE2AtetO-E4.ΔE3(XbaI). pBr/Ad.Bam-rITRΔ E2AtetO-E4.ΔE3(XbaI) was digested with BamHI and PacI restriction enzymes and an 11 kb fragment, with the *XbaI-XbaI* deletion, was isolated. The plasmid pWE/Ad.AflII-rITRΔE2A was first digested with BamHI and then partially digested with PacI, yielding amongst others a 26.2 kb fragment. The 11 kb BamHI-PacI fragment from and the 26.2 kb fragment were ligated, yielding cosmid pWE/Ad.AflII-rITRΔE2AtetO-E4.Δ E3(XbaI). This cosmid contains sequences identical to that of pWE/Ad.AflII-rITRΔE2AtetO-E4 but with the deletion of the XbaI-XbaI fragment. pWE/Ad.AflII-rITRΔE2AtetO-E4.ΔE3 (XbaI) was used in the production of adenoviruses with E1, E2A and E3 deletions and E4 attenuations in 96 well plates.

Example 19

Comparison between pWE/Ad.AflII-rITRΔE2AtetO-E4 and pWE/Ad.AflII-rITRΔE2AtetO-E4.ΔE3(XbaI) in the generation of E4 attenuated viruses in a 96-wells setting for the purpose of functional genomics.

At day 1, PER/E2A/tTA clone 1C3 was harvested and diluted with culture medium (DMEM+10% FBS and 10 mM $MgCl_2$) to a density of 22,500 cells per 100 µl, followed by seeding 100 µl per well in 96-well-tissue culture plates. At day 2, linearized adapter molecules pAd/pIPspAdapt-ceNOS, pAd/pIPspAdapt-eGFP, pAd/pIPspAdapt-hEPO, pAd/pIPspAdapt-hIL3, pAd/pIPspAdapt-lacZ and pAd/pIPspAdapt-luciferase were used for transfection in combination with 2 different *Pac*I linearized helper cosmids: pWE/Ad.AflII-rITRΔE2AtetO-E4 and pWE/Ad.AflII-rITRΔE2AtetO-E4.ΔE3 (XbaI).
The DNA transfection procedure was identical to that described in Example (above) describing transfections in a subset of PER/E2A/tTA clones. The transfection efficiency of wells, transfected with lacZ as adapter molecule, scored after lacZ staining, was 70-80% for both cosmid combinations. CPE formation was monitored during a period of three weeks after transfection. Fig.22 shows the percentage of CPE positive wells in a comparison between pWE/Ad.AflII-rITRΔE2AtetO-E4 and pWE/Ad.AflII-rITRΔE2AtetO-E4.ΔE3 (XbaI) transfections. Subsequently, the wells were subjected to freezing in the culture medium at -20°C, followed by thawing and resuspension by repeated pipetting. An aliquot of 100 µl of the freeze/thawed transfected cells was transferred to each well of a plate with freshly seeded and attached PER/E2A/tTA cells of clone 1C3. These cells were seeded as described above. The second 96-well plate with PER/E2A/tTA cells, incubated with the freeze/thawed cell lysates of the first transfected plate, was checked for CPE formation and stored at -20°C. Fig.23 shows the percentage of virus propagation (CPE positive wells) in 96-wells plates seeded with fresh PER/E2A/tTA cells. This was scored after infection with the supernatants from the freeze/thawed transfected cells as shown in Fig.22. The generation of functional virus using this set-up was shown by several assays (described in WO 99/64582) in which the presence of the proteins that are encoded by the adapter plasmids was determined. Fig. 24 shows the percentage of functional viruses that produce either human IL-3, LacZ or Luciferase in the 96-wells setting.

Example 20

Determination of toxicity using tetO-E4 attenuated viruses in comparison to non-tetO-E4 attenuated viruses using microarray technology

To determine the different effects on cellular gene expression and vector associated toxicity between adenoviral vectors with different deletions and attenuations, microarray experiments are performed. This allows the measurement of mRNA expression of thousands of genes simultaneously and the effect a particular adenoviral vectors has on these gene expressions.
On day 1, different cell lines, primary as well as established, are seeded in T25 tissue culture flasks. The next day cells are infected with E1, E1/E2A, E1/E2A/E3 deleted or E4 attenuated adenoviral vectors with a serie of different MOI's. After 24 hours, medium of the infected cells is changed. Then, 24, 48 and 72 hours after infection cells are harvested and trizol lysates are made from which RNA and subsequently cDNA is generated. This cDNA is used to generate Cy3 and Cy5 labeled probes using common techniques, known to those skilled in the art and described in Zhu et al (1998). Using the wild type E1 deleted Ad5 vector as a reference, expression profiles are determined for adenoviral vectors with different deletions and attenuations by hybridizing the labeled probes to microarrays (reviewed in Marshall and Hodgson 1998; Ramsay 1998). The fluorescent signals of both Cy3 and Cy5 are determined using a microarray scanner and converted using controls for hybridization and cDNA labeling and generation to normalized fluorescent values. The Cy3 and Cy5 values are then compared which results in relative data showing the difference in response of cells to infection with different types of adenoviral vectors including tetO-E4 attenuated and non tetO-E4 attenuated vectors.

Example 21

In vivo production of nitric oxid upon treatment with E4 attenuated viruses expressing ceNOS.

The in vivo duration of NOS3 transgene expression after aerosol gene transfer of rat lungs with the recombinant adenovirus IG.Ad/ΔE1ΔE2AtetO-E4-ceNOS was assessed. Twelve male Wistar rats (body weight 300~350 g) were aerosolized via a silastic catheter into the trachea with IG.Ad/ΔE1ΔE2AtetO-E4-ceNOS recombinant adenovirus (300 µl physiologic salt solution containing $3x10^9$ plaque forming units over 60 minutes). After viral delivery, the catheter was removed from the trachea and animals were extubated. No side effects were observed during aerosol delivery or following extubation.

At several time points (day 3, 7, 14 and 21) the transduced animals were re-anaesthetized and re-intubated for exhaled Nitric Oxide measurements during room air breathing. Nitric Oxide (NO) levels in exhaled air were measured using standard chemiluminescence (Sievers 280 - NO analyzer NOA™). Each day of exhaled NO measurements, calibration was performed using a calibration-gas-mixture of exactly 400 parts per million (ppm). The minimal detectable NO concentration was 1 ppb. Two animals died 24 hours after the aerosolisation (17%), probably due to traumatic intubation and persistent tracheal damage after extubation. Mean body weight of the transfected animals at baseline was 363±27 g and decreased to 319±15 g (P<0.05 vs baseline) three days after aerosolisation. Seven days after singe aerosol gene transfer, body weight was normalized (325±15 gr, P=NS vs baseline). Three days after aerosolisation, the exhaled NO levels of the aerosolized animals increased from baseline (5±1 ppb) to 14±5 ppb (n=3). Exhaled NO levels remained elevated at day 7 and 14: 12±9 (n=2) and 16±5 ppb (n=4) respectively. After 21 days exhaled NO levels returned back to baseline levels, which is approximately 5±1 ppb.

These results suggest that there is a longer duration of expression of ceNOS using IG.Ad/ΔE1ΔE2AtetO-E4-ceNOS virus as compared to the duration of ceNOS expression using a first generation (non-attenuated E4) adenoviral ceNOS virus, which was used in the acute and prolonged hypoxia model (Budts et al. 2000). In this model, first generation adenoviral vectors express ceNOS only for one week and the measured concentration of exhaled NO, as determined at day 7 and day 14 returns to baseline levels at day 14. No toxicity of clinical relevance and differences in loss of body weight as compared to non-E4 attenuated vectors, that were used in the past, were detected after gene transfer with the attenuated-E4 adenovirus.

## FIGURE AND TABLE LEGENDS

### Figure 1: (A) Expression of DBP, Penton and Fiber.

A549 cells were infected with a multiplicity of infection (m.o.i.) of either 0, 100, 1,000 or 10,000 vp/cell of IG. Ad/CLIP or IG.Ad.CLIPΔE2A. Seventy-two hours post infection, cell extracts were prepared and equal amounts of whole cell extract were fractionated by SDS-PAGE in 10% gels. The proteins were visualized with the aDBP monoclonal B6, the polyclonal α-Penton base Ad2-Pb571 or the polyclonal α-knob domain of fiber E641/3, using an ECL detection system. Cells infected with IG.Ad.CLIP express both E2A encoded DBP, Penton base and Fiber proteins. The proteins co-migrate with the respective proteins in the positive control (lane P, extract from PER.C6 cells infected with IG.Ad. CLIP harvested at starting CPE). In contrast, no DBP, penton-base or fiber was detected in the non-infected A549 cells or cells infected with IG.Ad.CLIPΔE2A. These data show that deletion of the E2A gene did not only eliminate residual DBP expression, but also the residual expression of the late adenoviral proteins penton-base and fiber.

### (B) Residual expression of E4-orf6 and pTP/TP.

The blot, as shown in A, was stripped and used to visualize the E4-orf6 and pTP/TP proteins using the method described above but now by using a monoclonal antibody against E4-orf6 or a polyclonal anti-pTP/TP antiserum, respectively. These proteins co-migrate with the respective proteins in the positive control (P). The results show that pTP/TP as well as E4-orf6 are still produced from the E1/E2A deleted Ad vector. This indicates that the deletion of the E2A gene did not eliminate the residual expression of the E2B and E4 genes.

### Figure 2: Temperature dependent growth of PER.C6.

PER.C6 cells were cultured in Dulbecco's Modified Eagle Medium supplemented with 10% Fetal Bovine Serum (FBS, Gibco BRL) and 10mM $MgCl_2$ in a 10% $CO_2$ atmosphere at either 32°C, 37°C or 39°C. At day 0, a total of 1 x $10^6$ PER.C6 cells were seeded per 25cm$^2$ tissue culture flask (Nunc) and the cells were cultured at either 32°C, 37°C or 39°C. At day 1-8, cells were counted. The growth rate and the final cell density of the PER.C6 culture at 39°C are comparable to that at 37°C. The growth rate and final density of the PER.C6 culture at 32°C were slightly reduced as compared to that at 37°C or 39°C.

PER.C6 cells were seeded at a density of 1 x $10^6$ cells per 25 cm$^2$ tissue culture flask and cultured at either 32-, 37- or 39°C. At the indicated time points, cells were counted in a Burker cell counter. PER.C6 grows well at both 32-, 37- and 39°C.

### Figure 3: DBP levels in PER.C6 cells transfected with pcDNA3, pcDNA3wtE2A or pcDNA3ts125E2A.

Equal amounts of whole-cell extract were fractionated by SDS-PAGE on 10% gels. Proteins were transferred onto Immobilon-P membranes and DBP protein was visualized using the aDBP monoclonal B6 in an ECL detection system. All of the cell lines derived from the pcDNA3ts125E2A transfection express the 72-kDa E2A-encoded DBP protein (left panel, lanes 4-14; middle panel, lanes 1-13; right panel, lanes 1-12). In contrast, the only cell line derived from the pcDNAwtE2A transfection did not express the DBP protein (left panel, lane 2). No DBP protein was detected in extract from a cell line derived from the pcDNA3 transfection (left panel, lane 1), which serves as a negative control. Extract from PER.C6 cells transiently transfected with pcDNA3ts125 (left panel, lane 3) served as a positive control for the Western blot procedure. These data confirm that constitutive expression of wtE2A is toxic for cells and that using the ts125 mutant of E2A can circumvent this toxicity.

**Figure 4: Suspension growth of PER.C6ts125E2A C5-9.**

The tsE2A expressing cell line PER.C6tsE2A.c5-9 was cultured in suspension in serum free Ex-cell™. At the indicated time points, cells were counted in a Burker cell counter. The results of 8 independent cultures are indicated. PER.C6tsE2A grows well in suspension in serum free Ex-cell™ medium.

**Figure 5: Growth curve PER.C6 and PER.C6tsE2A.**

PER.C6 cells or PER.C6ts125E2A (c8-4) cells were cultured at 37°C or 39°C, respectively. At day 0, a total of 1 x $10^6$ cells was seeded per $25cm^2$ tissue culture flask. At the indicated time points, cells were counted. The growth of PER.C6 cells at 37°C is comparable to the growth of PER.C6ts125E2A c8-4 at 39°C. This shows that constitutive overexpression of ts125E2A has no adverse effect on the growth of cells at the non-permissive temperature of 39°C.

**Figure 6: Stability of PER.C6ts125E2A.**

For several passages, the PER.C6ts125E2A cell line clone 8-4 was cultured at 39°C in medium without G418. Equal amounts of whole-cell extract from different passage numbers were fractionated by SDS-PAGE on 10% gels. Proteins were transferred onto Immobilon-P membranes and DBP protein was visualized using the aDBP monoclonal B6 in an ECL detection system. The expression of ts125E2A encoded DBP is stable for at least 16 passages, which is equivalent to approximately 40 cell doublings. No decrease in DBP levels were observed during this culture period, indicating that the expression of ts125E2A is stable, even in the absence of G418 selection pressure.

**Figure 7: tTA activity in hygromycin resistent PER.C6/tTA (A) and PER/E2A/tTA (B) cells.**

Sixteen independent hygromycin resistent PER.C6/tTA cell colonies and 23 independent hygromycin resistent PER/E2A/tTA cell colonies were grown in 10 $cm^2$ wells to sub-confluency and transfected with 2 µg of pUHC 13-3 (a plasmid that contains the reporter gene luciferase under the control of the 7xtetO promoter). One half of the cultures was maintained in medium containing doxycycline to inhibit the activity of tTA. Cells were harvested at 48 hours after transfection and luciferase activity was measured. The luciferase activity is indicated in relative light units (RLU) per µg protein.

Fig.8

Western blot to visualize the residual expression of E4-orf6 (E4-34 kDa) protein and a Southern blot to visualize the cell-associated viral DNA. A549 cells were inoculated with 1000 vp/cell of IG.Ad/AdAptLucΔE1 (dE1), IG.Ad/AdAptLucΔE1Δ E2A (dE2A), IG.Ad/AdAptLucΔE1tetO-E4 (dE1.tetO-E4), or IG.Ad/AdAptLucΔE1ΔE2AtetO-E4 (dE1.dE2A.tetO-E4). At 30 h post-inoculation, the cells were harvested and the relative amount of E4-orf6 protein in each sample was determined by Western blotting using an E4-orf6 specific anti-peptide serum (kind gift of Dr P. Branton). Parallel cultures, infected by the same vectors at the same time, were used to check the transduction efficiency of the vectors. This was done by Southern analysis.

Fig.9

The infections (described for Fig.8) for each analysis were done in triplicate and analyzed by western blotting.

Fig.10

The infections (described for Fig.8) for each analysis were done in triplicate and analyzed by Southern blotting.

Fig.11

A549 cells were inoculated with 1000 vp/cell of IG.Ad/AdAptLucΔE1 (dE1), IG.Ad/AdAptLucΔE1ΔE2A (dE2A), IG.Ad/AdAptLucΔE1tetO-E4 (dE1.tetO-E4), or IG.Ad/AdAptLucΔE1 ΔE2AtetO-E4 (dE1.dE2A.tetO-E4). All infections were performed in triplicate. At 30 h post-inoculation, the cells were harvested and the relative amount of DBP protein in each sample was determined by Western blotting using the anti-DBP monoclonal antibody B6 (first antibody, 1:1000 diluted; Reich et al., 1983). In parallel, one culture was analyzed that not been infected with any vector (mock). As a positive control, a lysate of cells in which an Ad vector had undergone replication, had been taken.

Fig.12

A549 cells were inoculated with 1000 vp per cell using E1-deleted Ad vectors (dE1), E1+E2A-deleted Ad vectors (dE2A), E1-deleted and E4-attenuated (dE1.tetO-E4), or E1+E2A-deleted and E4-attenuated (dE1.dE2A.tetO-E4). At 30 h post-inoculation, the cells were harvested and the relative amount of the E2B encoded protein pTP in each sample was determined by Western blotting using a mixture (1:1:1) of three antibodies against (p)TP and Pol.

Fig.13

A549 cells were inoculated with 1000 vp/cell of IG.Ad/AdAptLucΔE1 (dE1), IG.Ad/AdAptLucΔE1ΔE2A (dE2A), IG.Ad/AdAptLucΔE1tetO-E4 (dE1.tetO-E4), or IG.Ad/AdAptLucΔE1 ΔE2AtetO-E4 (dE1.dE2A.tetO-E4). All infections were performed in triplicate. At 72 h post-inoculation, the cells were harvested and the relative amount of fiber protein in each sample was determined by Western blotting using the polyclonal E641/3 anti-knob domain of fiber (primary antibody, 1:5000 diluted).

Fig.14

A549 cells were inoculated with IG.Ad/AdAptLucΔE1 (dE1), IG.Ad/AdAptLucΔE1ΔE2A (dE1.dE2A), IG.Ad/AdAptLucΔE1tetO-E4 (dE1.tetO-E4), or IG.Ad/AdAptLucΔE1ΔE2AtetO-E4 (dE1.dE2A.tetO-E4). All infections were performed in triplicate and using the indicated multiplicity of infection (MOI). The cells were harvested by detergent-mediated lysis at 48h post-inoculation and the luciferase activity in the cell extracts was measured and expressed in relative light units (RLU) per µg protein present in the cell extracts.

Fig.15

Primary human endothelial cells were inoculated with IG.Ad/AdAptLucΔE1ΔE2A (dE2A) or with IG.Ad/AdAptLucΔE1Δ E2AtetO-E4 (dE2A.tetO-E4). All infections were performed in triplicate and using the indicated multiplicity of infection (MOI). The cells were harvested by detergent-mediated lysis at 48h post-inoculation and the luciferase activity in the cell extracts was measured and expressed in relative light units (RLU) per µg protein present in the cell extracts.

Fig.16

Longevity of expression in A549 cells of Luciferase upon infection with crude lysates and purified virus in a comparison study with attenuated-E4 and non-attenuated-E4 recombinant adenoviruses.

Fig.17

Longevity of expression in HepG2 cells of Luciferase upon infection with crude lysates and purified virus in a comparison study with attenuated-E4 and non-attenuated-E4 recombinant adenoviruses.

Fig.18

Longevity of expression in MCF-7 cells of Luciferase upon infection with crude lysates and purified virus in a comparison study with attenuated-E4 and non-attenuated-E4 recombinant adenoviruses.

Fig.19

Viral genomic DNA was isolated from equal numbers of purified E1+E2deleted (Ad.dE2A) and E1+E2A-deleted, E4-attenuated (Ad.dE2A.tetO-E4) Ad vector particles and cut with PstI. As positive controls, the plasmid pWE/Ad.AflII-rITR.tetO-E4 was digested with *Pac*I and PstI, and the plasmid pNEB-PaSe.tetO was digested with HindIII. The DNA samples were run in a 1% agarose gel, blotted onto a membrane and probed with a $^{32}P$-labeled 313 bp HindIII/NcoI fragment of pNEB-PaSe.tetO.

Fig.20

Percentage of CPE positive wells, based on the results of the first transfection round. CPE was scored three weeks after transfection.

Fig.21

Percentage of CPE positive wells in the second transfection round, scored three weeks after transfection in six different PER/E2A/tTA clones transfected with either pAd/pIPspAdapt-eGFP, or pAd/pIPspAdapt-lacZ or pAd/Adapt-ceNOS. All three different transfections were in addition to pWE/Ad.AflII-rITR ΔE2A.tetO-E4.

Fig.22

Percentage of cpe positive wells in 96-wells plates using PER/E2A/tTA cells transfected with lacZ adapter plasmid in combination with either pWE/Ad.AflII-rITRΔE2AtetO-E4 or pWE/Ad.AflII-rITRΔE2AtetO-E4.ΔE3 (XbaI).

Fig.23

Percentage of cpe positive wells in 96-wells plates using fresh PER/E2A/tTA cells infected with supernatants from PER/E2A/tTA cells that were transfected with lacZ adapter plasmid in combination with either pWE/Ad.AflII-rITRΔE2AtetO-E4 or pWE/Ad.AflII-rITRΔE2AtetO-E4.ΔE3 (XbaI).

Fig.24

Percentage of cpe positive wells harboring viruses that produce functional hIL-3, LacZ and Luciferase.

**Table I: Generation of E2B and E4 attenuated recombinant adenoviral vectors.**

This table shows a selection of E2B and E4 attenuated Ad vectors that have been generated by cotransfection of the indicated plasmid DNAs into the indicated cells. A cytopathic effect (CPE) typical for adenovirus replication (the appearance of rounded cells and so-called comets in the cell monolayer) is usually detected at 8-14 days after transfection. Transfection of only one of the plasmids did not result in CPE (not shown).

**Table II A and B: Growth of E2B and E4 attenuated recombinant adenoviral vectors in PER.C6/E2A and PER/E2A/tTA cells.**

The indicated viruses were diluted as indicated (from $10^{-3}$ - $10^{-7}$) and used to inoculate subconfluent monolayers of the indicated cells grown in 10 $cm^2$ wells. Parallel cultures of the cells were not incubated with virus (mock). The cells were incubated at 34°C. CPE was scored at 4 (A) and 5 (B) days after inoculation. 0 means no CPE; 0-1 means sporadic comet formation; 1 means several comets present in the culture, 25% of the cells show CPE; 2 means 50% of the cells shows CPE ; 3 means 75% of the cells show CPE; 4 means 100% of the cells show CPE.

**CITED LITERATURE**

Amalfitano A., and J. S. Chamberlain (1997) Isolation and characterization of packaging cell lines that coexpress the adenovirus E1, DNA polymerase, and preterminal proteins: implications for gene therapy. Gene Ther. 4; 258-263.
Armentano, D., J. Zabner, C. Sacks, C. C. Sookdeo, M. P. Smith, J. A. St. George, S. C. Wadsworth, A. E. Smith, and R. J. Gregory (1997) Effect of the E4 region on the persistence of transgene expression from adenovirus vectors. J. Virol. 71;2408-2416.
Armentano, D., M. P. Smith, C. C. Sookdeo, J. Zabner, M. A. Perricone, J. A. St. George, S. C. Wadsworth, and R. J. Gregory (1999) E40RF3 requirement for achieving long-term transgene expression from the cytomegalovirus promotor in adenovirus vectors. J. Virol. 73;7031-7034.

Blanton, R. A., and T. H. Carter (1979) Autoregulation of adenovirus type 5 early gene expression. III. Transcription studies in isolated nuclei. J. Virol. 29;458-465.

Boivin, D., M. R. Morrison, R. C. Marcellus, E. Querido, and P. E. Branton (1999) Analysis of synthesis, stability, phosphorylation, and interacting polypeptides of the 34-kilodalton product of open reading frame 6 of the early region 4 protein of human adenovirus type 5. J. Virol. 73;1245-1253.

Boshart et al., 1985; A very strong enhancer is located upstream of an immediate early gene of human cytomegalovirus, Cell 41,521-530, 1985

Bout, A. (1997) Gene therapy. *In* Pharmaceutical Biotechnology, chapter 7, pp. 167-182.

Bridge, E., and G. Ketner (1989) Redundant control of adenovirus late gene expression by early region 4. J. Virol. 63;631-638.

Brough, D. E., A. Lizonova, C. Hsu, V. A. Kulesa, and I. Kovesdi (1996) A gene transfer vector-cell line system for complete functional complementation of adenovirus early regions E1 and E4. J. Virol. 70;6497-6501.

Brough, D. E., C. Hsu, V. A. Kulesa, G. M. Lee, L. J. Cantolupo, A. Lizonova, and I. Kovesdi (1997) Activation of transgene expression by early region 4 is responsible for a high level of persistent transgene expression from adenovirus vectors in vivo. J. Virol. 71;9206-9213.

Budts W., Pokreisz P., Nong Z., Van Pelt N., Gillijns H., Gerard R., Lyons R., Collen D., Bloch K.D. and Janssens S. (2000) Aerosol gene transfer with inducible nitric oxide synthase reduces hypoxic pulmonary hypertension and pulmonary vascular remodeling in rats. Circulation: in press.

Chang, L.-S., and T. Shenk (1990) The adenovirus DNA-binding protein stimulates the rate of transcription directed by adenovirus and adeno-associated virus promoters. J. Virol. 64;2103-2109.

Dedieu, J.-F., E. Vigne, C. Torrent, C. Jullien, I. Mahfouz, J.-M. Caillaud, N. Aubailly, C. Orsini, J.-M. Guillaume, P. Opolon, P. Delaere, M. Perricaudet, and P. Yeh (1997) Long-term gene delivery into the livers of immunocompetent mice with E1/E4-defective adenoviruses. J. Virol. 71;4626-4637.

Dobner, T., N. Horikoshi, S. Rubenwolf, S., and T. Schenk (1996) Blockage by adenovirus E4orf6 of transcriptional activation by the p53 tumor suppressor. Science 272;1470-1473.

Ensinger, M. J., and H. S. Ginsberg (1972) Selection and preliminary characterization of temperature-sensitive mutants of type 5 adenovirus. J. Virol. 10;328-339.

Fang, B., and P. Koch, and J. A. Roth (1997) Diminishing adenovirus gene expression and viral replication by promotor replacement. J. Virol. 71;4798-4803.

Filmus, J., J. Remani, and M. H. Klein (1992) Synergistic induction of promoters containing metal- and glucocorticoid-responsive elements. Nucleic Acids Res. 20;2755-2760.

Fisher, K. J., H. Choi, J. Burda, S.-J. Chen, and J. M. Wilson (1996) Recombinant adenovirus deleted of all viral genes for gene therapy of cystic fibrosis. Virology 217;11-22.

Gossen, M., and H. Bujard (1992) Tight control of gene expression in mammalian cells by tetracycline-responsive promoters. Proc. Natl. Acad. Sci. USA 89;5547-5551.

Hardy, S., M. Kitamura, T. Harris-Stansil, Y. Dai, and M. L. Phipps (1997) Construction of adenovirus vectors through crelox recombination. J. Virol. 71;1842-1849.

Huang, M.-M., and P. Hearing (1989) Adenovirus early region 4 encodes two gene products with redundant effects in lytic infection. J. Virol. 63;2605-2615.

Kaufman, R. J. (1990) Vectors used for expression in mammalian cells. Meth. Enzym. 185;487-511.

Kay, R., F. Takei, and R. K. Humphries (1990) Expression cloning of a cDNA encoding Ml/69-Jlld heat-stable antigens. J. Immunol. 145;1952-1959.

Kochanek, S., P. R. Clemens, K. Mitani, H.-H. Chen, S. Chan, and C. T. Caskey (1996) A new adenoviral vector: replacement of all viral coding sequences with 28 kb of DNA independently expressing both full-length dystrophin and β-galactosidase. Proc. Natl. Acad. Sci. USA 93;5731-5736.

Kriegler, M. (1990) Assembly of enhancers, promoters, and splice signals to control expression of transferred genes. Meth. Enzym. 185;512-527.

Kumar-Singh R., and J. S. Chamberlain (1996) Encapsidated adenovirus minichromosomes allow delivery and expression of a 14 kb dystrophin cDNA to muscle cells. Hum. Mol. Genet. 5;913-921.

Lanier, L. M., K. Storm, A. Shafaie, and L. E. Volkman (1997) Copper treatment increases recombinant baculovirus production and polyhedrin and p10 expression. BioTechniques 23;728-735.

Leppard, K. N. (1997) E4 gene function in adenovirus, adenovirus vector and adeno-associated virus infections. J. Gen. Virol. 78;2131-2138.

Levrero, M., V. Barban, S. Manteca, A. Ballay, C. Balsamo, M. L. Avantaggiati, G. Natoli, H. Schellekens, P. Tiollais, and M. Perricaudet (1991) Defective and nondefective adenovirus vectors for expressing foreign genes in vitro and in vivo. Gene 101;195-202.

Lusky, M., M. Christ, K. Rittner, A. Dieterle, D. Dreyer, B. Mourot, H. Schulz, F. Stoeckel, A. Pavirani, and M. Mehtali (1998) In vitro and in vivo biology of recombinant adenovirus vectors with E1, E1/E2A, or E1/E4 deleted. J.

Virol. 72;2022-2032.
Marshall A. and Hodgson J. (1998) DNA chips: an array of possibilities. Nature Biotechnology 16:27
Mizushima, S., and S. Nagata (1990) pEF-BOS, a powerful mammalian expression vector. Nucleic Acid Res. 18;5322.
Moore, M., N. Horikoshi, and T. Schenk (1996) Oncogenic potential of the adenovirus E4orf6 protein. Proc. Natl. Acad. Sci. USA 93;11295-11301.
Nenoi, M., K. Mita, S. Ichimura, I. L. Cartwright, E. Takahashi, M. Yamauchi, and H. Tsuji (1996) Heterogeneous structure of the polyubiquitin gene UbC of HeLa S3 cells. Gene 175;179-185.
Nevins, J. R., and J. J. Winkler (1980) Regulation of early adenovirus transcription: a protein product of early region 2 specifically represses region 4 transcription. Proc. Natl. Acad. Sci. USA 77;1893-1897.
Parks, R. J., and F. L. Graham (1997) A helper-dependent system for adenovirus vector production helps define a lower limit for efficient DNA packaging. J. Virol. 71;3293-3298.
Ploegh, H. L. (1998) Viral strategies of immune evasion. Science 280;248-253.
Pombo, A., J. Ferreira, E. Bridge, and M. Carmo-Fonseca (1994) Adenovirus replication and transcription sites are spatially separated in the nucleus of infected cells. EMBO J. 13;5075-5085.
Ramsay G. (1998) DNA chips: State of the art. Nature Biotechnology 16:40
Reich N. C., P. Sarnow, E. Duprey, and A. J. Levine (1983) Monoclonal antibodies which recognize native and denatured forms of the adenovirus DNA-binding protein. Virology 128;480-484.
Rice, S. A., and D. F. Klessig (1985) Isolation and analysis of adenovirus type 5 mutants containing deletions in the gene encoding the DNA-binding protein. J. Virol. 56;767-778.
Rittner K., H. Schultz, A. Pavirani, and M. Mehtali (1997) Conditional repression of the E2 transcription unit in E1-E3-deleted adenovirus vectors is correlated with a strong reduction in viral DNA replication and late gene expression in vitro. J. Virol. 71;3307-3311.
Roth, J., C. König, S. Wienzek, S. Weigel, S. Ristea, and M. Dobbelstein (1998) Inactivation of p53 but not p73 by adenovirus type 5 E1B 55-kilodalton and E4 34-kilodalton oncoproteins. J. Virol. 72;8510-8516.
Sandler, A. B., and G. Ketner (1991) The metabolism of host RNAs in cells infected by an adenovirus E4 mutant. Virology 181;319-326.
Song, W., H. L. Kong, P. Traktman, and R. G. Crystal (1997) Cytotoxic T lymphocyte responses to proteins encoded by heterologous transgenes transferred in vivo by adenoviral vectors. Hum. Gene Ther. 8;1207-1217.
Swaminathan, S., and B. Thimmapaya (1995) Regulation of adenovirus E2 transcription unit, p. 177-194. *In* W. Doerfler and P. Böhm (Eds.), The Molecular Repertoire of Adenoviruses III, Springer-Verlag, Berlin, Germany.
Stuart, G. W., P. F. Searle, H. Y. Chen, R. L. Brinster, and R. D. Palmiter (1984) A 12-base-pair DNA motif that is repeated several times in metallotheonein gene promoters confers metal regulation to a heterologous gene. Proc. Natl. Acad. Sci. USA 81;7318-7322.
Takekoshi, M., F. Maeda-Takekoshi, S. Ihara, S. Sakuma, and Y. Watanabe (1993) Inducible expression of a foreign gene inserted into the human cytomegalovirus genome. J. Gen. Virol. 74;1649-1652.
van der Vliet, P. C. (1995) Adenovirus DNA replication. *In* W. Doerfler, and P. Böhm (Eds.) The Molecular Reportoire of Adenoviruses II, Springer Verlag, Berlin, Germany, pp. 1-30.
van der Vliet, P. C., A. J. Levine, M. J. Ensinger, and H. S. Ginsberg (1975) Thermolabile DNA binding proteins from cells infected with a temperature-sensative mutant of adenovirus defective in viral DNA synthesis. J. Virol. 15; 348-354.
Weinberg, D. H., and G. Ketner (1986) A cell line that supports the growth of a defective early region 4 deletion mutant of human adenovirus type 2. Proc. Natl. Acad. Sci. USA 80;5383-5386.
Yang, Y., Nunes, F. A., K. Berencsi, E. Gonczol, J. F. Engelhardt, and J. M. Wilson (1996) Inactivation of E2A in recombinant adenoviruses improves the prospect for gene therapy in cystic fibrosis. Nat. Genet. 7;362-369.
Yang, Y., Q. Su, and J. M. Wilson (1996) Role of viral antigens in destructive cellular immune responses to adenovirus vector-transduced cells in mouse lungs. J. Virol. 70;7209-7212.
Yang, H., and J. Filmus (1996) Insertion of metal-responsive elements increase the inducibility of the mouse mammary tumor virus promoter. Gene 182;177-182.
Yarranton, G. T. (1992) Inducible vectors for expression in mammalian cells. Curr. Op. Biotech. 3;506-511.
Yeh, P., J.-F. Dedieu, C. Orsini, E. Vigne, P. Denefle, and M. Perricaudet (1996) Efficient dual transcomplementation of adenovirus E1 and E4 regions from a 293-derived cell line expressing a minimal E4 functional unit. J. Virol. 70;559-565.
Zhu H., Cong J-P., Mamtora C., Gingeras T. and Shenk T. (1998) Cellular gene expression altered by human cytomegalovirus: Global monitoring with oligonucleotide arrays. Proc.Natl.Acad.Sci.USA 95:14470

## TABLE I:GENERATION OF ATTENUATED ADENOVIRAL VECTORS

| TRANSFECTION | CELLS | CPE | GENERATED VECTOR |
|---|---|---|---|
| pAd/AdApt.LacZ + pWE/Ad.AflII-rITR.tetO-E4 | PER.C6/tTA | yes | IG/Ad.AdApt.LacZ.tetO-E4 |
| pAd/ULIP.LacZ + pWE/Ad.AflII-rITR-tetO-E4 | PER.C6/tTA | yes | IG/Ad.ULIP.LacZ.tetO-E4 |
| pAd/AdApt.LacZ + pWE/Ad.AflII-rITR.dE2A.tetO-E4 | PER/E2A/tTA | yes | IG/Ad.AdApt.LacZ.dE2A.tetO-E4 |
| pAd/ULIP.LacZ + pWE/Ad.AflII-rITR.dE2A.tetO-E4 | PER/E2A/tTA | yes | IG/Ad.ULIP.LacZ.dE2A.tetO-E4 |
| pAd/AdApt.LacZ + pWE/Ad.AflII-rITR.tetO-E2B | PER/E2A/tTA | yes | IG/Ad.AdApt.LacZ.tetO-E2B |
| pAd/ULIP.LacZ + pWE/Ad.AflII-rITR.tetO-E2B | PER/E2A/tTA | yes | IG/Ad.ULIP.LacZ.tetO-E2B |

**TABLE II (A): GROWTH OF ATTENUATED ADENOVIRAL VECTORS**

| VECTOR | CELLS | $10^{-3}$ | $10^{-4}$ | $10^{-5}$ | $10^{-6}$ | $10^{-7}$ | mock |
|---|---|---|---|---|---|---|---|
| IG/Ad.AdApt.LacZ.dE2A.tetO-E4 | PER/E2A/tTA | 0-1 | 0 | 0 | 0 | 0 | 0 |
| | PER/E2A | 0 | 0 | 0 | 0 | 0 | 0 |
| IG/Ad.ULIP.LacZ.dE2A.tetO-E4 | PER/E2A/tTA | 1 | 0-1 | 0 | 0 | 0 | 0 |
| | PER/E2A | 0 | 0 | 0 | 0 | 0 | 0 |
| IG/Ad.ULIP.LacZ.tetO-E2B | PER/E2A/tTA | 1 | 0-1 | 0-1 | 0 | 0 | 0 |
| | PER/E2A | 0 | 0 | 0 | 0 | 0 | 0 |
| IG/Ad.ULIP.LacZ.dE2A | PER/E2A/tTA | 1 | 0-1 | 0-1 | 0 | 0 | 0 |
| | PER/E2A | 2 | 1 | 0-1 | 0-1 | 0 | 0 |

**Incubated at 34°C, scored at 4 days after inoculation.**

**TABLE II (B): GROWTH OF ATTENUATED ADENOVIRAL VECTORS**

| VECTOR | CELLS | $10^{-3}$ | $10^{-4}$ | $10^{-5}$ | $10^{-6}$ | $10^{-7}$ | mock |
|---|---|---|---|---|---|---|---|
| IG/Ad.AdApt.LacZ.dE2A.tetO-E4 | PER/E2A/tTA | 1 | 1 | 0-1 | 0 | 0 | 0 |
| | PER/E2A | 0-1 | 0 | 0 | 0 | 0 | 0 |
| IG/Ad.ULIP.LacZ.dE2A.tetO-E4 | PER/E2A/tTA | 2 | 1 | 0-1 | 0>1 | 0 | 0 |
| | PER/E2A | 0-1 | 0>1 | 0 | 0 | 0 | 0 |
| IG/Ad.ULIP.LacZ.tetO-E2B | PER/E2A/tTA | 2 | 1 | 0-1 | 0 | 0 | 0 |
| | PER/E2A | 0 | 0 | 0 | 0 | 0 | 0 |
| IG/Ad.ULIP.LacZ.dE2A | PER/E2A/tTA | 2 | 1 | 0-1 | 0-1 | 0 | 0 |
| | PER/E2A | 4 | 3 | 2 | 1 | 0-1 | 0 |

**Incubated at 34°C, scored at 5 days after inoculation.**

Table III

Transfection efficiency in different PER/E2A/tTA clones, using pAd/Adapt-LacZ in combination with pWE/Ad.AflII-rITRΔE2A.tetO-E4 determined upon LacZ staining. pn= passage number.

| PER/E2A/tTA Clone | % Blue colored cells |
|---|---|
| 1A1 pn 16 | 50-60 |
| 1A1 pn 63 | 60 |
| 1A3 | 20-50 |
| 1A4 | 40-50 |
| 1A6 | 20-40 |
| 1B1 | 5-20 |
| 1B4 | 5-40 |
| 1B5 | 10-20 |
| 1C1 | 50-60 |
| 1C2 | 40 |
| 1C3 | 30-40 |
| 1C4 | 10-50 |
| 1D4 | 10 |
| 1D6 | 10-20 |
| 2A4 | 10-20 |
| 2A5 | 20-30 |
| 2B3 | 20-60 |
| 2C3 | 60 |
| 2D5 | 10-20 |

Table IV

Transfection efficiency of six different PER/E2A/tTA clones transfected with pAd/pIPspAdapt-lacZ and pWE/Ad.AflII-rITRΔE2A.tetO-E4 using LacZ staining. pn= passage number

| PER/E2A/tTA Clone | % Blue colored cells |
|---|---|
| 1A1 pn 16 | 5 |
| 1C1 | 5 |
| 1C3 | 50 |
| 2B3 | 60 |
| 2C3 | 20 |
| 2D5 | 30 |

TABLE V: Stability of PER.C6/tsE2A/tTA clone 1A1 in producing IG.Ad/AdAptLacZΔE1ΔE2AtetO-E4.

| | $10^{-1}$ | $10^{-2}$ | $10^{-3}$ | $10^{-4}$ | $10^{-5}$ | $10^{-6}$ |
|---|---|---|---|---|---|---|
| Passage 17 | 100% | 95% | 30% | 15-20% | 5-10% | < 5% |
| Passage 64 | 100% | 95% | 30% | 10% | 5-10% | < 5% |

## Claims

1. A method for producing a recombinant adenovirus-like gene delivery vehicle having reduced expression of adenoviral E2B and/or E4 gene products in a target cell for gene therapy, comprising generating a recombinant adenoviral vector lacking E1A and preferably E1B sequences, but having at least the E2B and/or E4 sequences encoding products essential for adenoviral replication, wherein said E2B and/or E4 sequences have been modified to lead to a reduced expression and/or induced expression of at least one of said essential products.

2. A method according to claim 1, wherein one such an essential product is open reading frame 1, 3 or 6 of E4.

3. A method according to claim 1 or 2, wherein said vector further comprises an E2B and/or an E4 promoter, wherein said E2B and/or E4 promoter are attenuated through a mutation therein.

4. A method according to claim 1 or 2, wherein E2B and/or E4 is placed under control of at least one, preferably synthetic inducible promotor and/or repressor.

5. A method according to any one of claims 1-4, wherein said vector further lacks a functional adenoviral DNA binding protein encoding sequence.

6. A method according to claim 5, wherein said vector lacks a functional E2A region.

7. A method according to any one of claims 1-6, wherein said vector lacks a sequence encoding E1B 55kD protein capable of binding an E4 34kDa gene product.

8. A method according to claim 4 wherein said repressor is activated and/or inactivated by an adenoviral DNA binding protein analogue.

9. A method according to any one of claims 1-8, further comprising transducing a complementing cell with said recombinant adenoviral vector wherein said complementing cell provides all functions and/or elements essential for replication of said recombinant adenoviral vector, which are lacking in the genome of said vector.

10. A method according to claim 9, wherein said complementing cell further comprises all necessary functions and/or elements essential for producing a recombinant adenovirus-like gene delivery vehicle comprising said recombinant adenoviral vector.

11. A method according to claim 9 or 10, wherein said cell further comprises an expression cassette encoding a proteinaceous substance capable of transactivating the inducible (synthetic) promoter according to claim 4.

12. A method according to claim 11, wherein said proteinaceous substance comprises a DNA binding domain from a prokaryote or a lower eukaryote.

13. A method according to claim 11 or 12 wherein said proteinaceous substance comprises a transactivator domain.

14. A method according to any one of claims 9-13, wherein said recombinant vector and said complementing cell have no sequence-overlap that leads to homologous recombination resulting in replication competent adenovirus and/or recombinant adenovirus comprising E1 sequences.

15. A recombinant adenoviral vector lacking E1A and preferably E1B sequences, but having at least the E2B and/or E4 sequences encoding products essential for adenoviral replication, wherein said E2B and/or E4 sequences have been modified to lead to a reduced expression and/or induced expression of at least one of said essential products, said vector being obtainable as an intermediate in a method according to any one of claims 1-14.

16. A recombinant adenovirus-like gene delivery vehicle having reduced expression of adenoviral E2B and/or E4 gene products in a target cell for gene therapy, obtainable by a method according to any one of claims 1-14.

17. A recombinant adenoviral vector according to claim 15 or a recombinant adenovirus-like gene delivery vehicle according to claim 16, comprising a therapeutic nucleic acid sequence.

**18.** A recombinant adenoviral vector and/or a recombinant adenovirus-like gene delivery vehicle according to anyone of claims 15-17, wherein said adenoviral vector comprises at least one adeno-associated virus terminal repeat or a functional equivalent thereof.

**19.** A recombinant adenoviral vector and/or a recombinant adenovirus-like gene delivery vehicle according to anyone of claims 15-18 comprising elements derived from at least two different adenovirus serotypes.

**20.** A method for *ex vivo* production of a gene product in a cell comprising providing said cell with a recombinant adenoviral vector and/or a recombinant adenovirus-like gene delivery vehicle according to anyone of claims 15-19 comprising nucleic acid encoding said gene product, culturing said cell to allow expression of said gene product and optionally harvesting said cell and/or medium said cell was exposed to.

**21.** Use of a recombinant adenoviral vector and/or a recombinant adenovirus-like gene delivery vehicle according to anyone of claims 15-19, for the preparation of a medicament.

**22.** A vaccine comprising a recombinant adenoviral vector and/or a recombinant adenovirus-like gene delivery vehicle according to anyone of claims 15-19, wherein said adenoviral vector comprises a nucleic acid encoding a proteinaceous molecule against which an immune response has to be raised.

# Figure 1A: Expression of DBP, Penton and Fiber

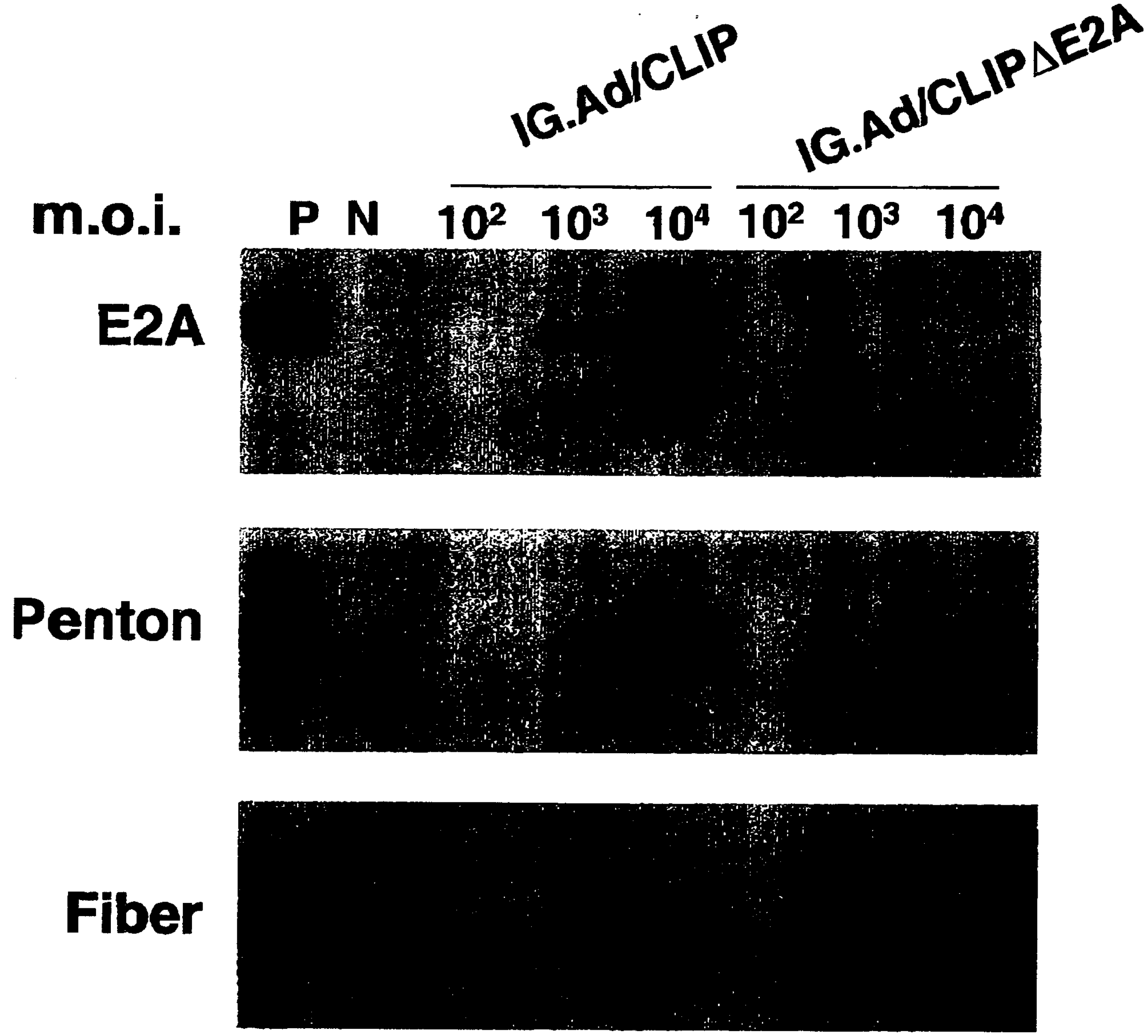

**Figure 1B:Expression of E4-Orf6 and pTP**

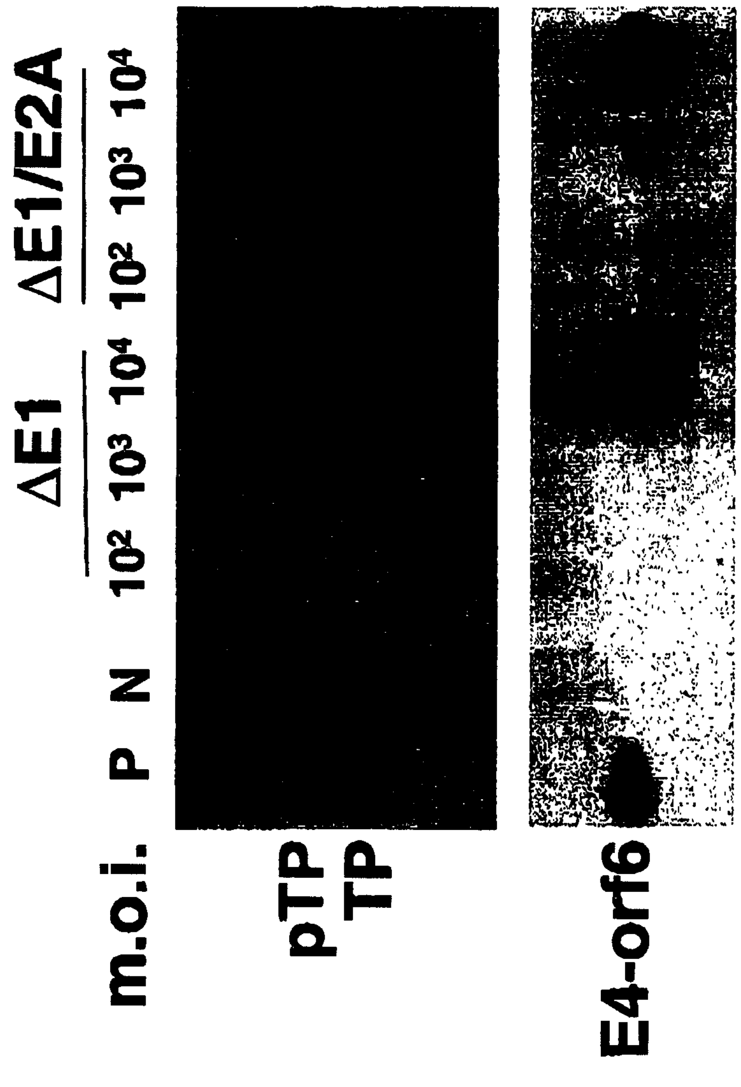

# Figure 2: Temperature dependent growth of PER.C6

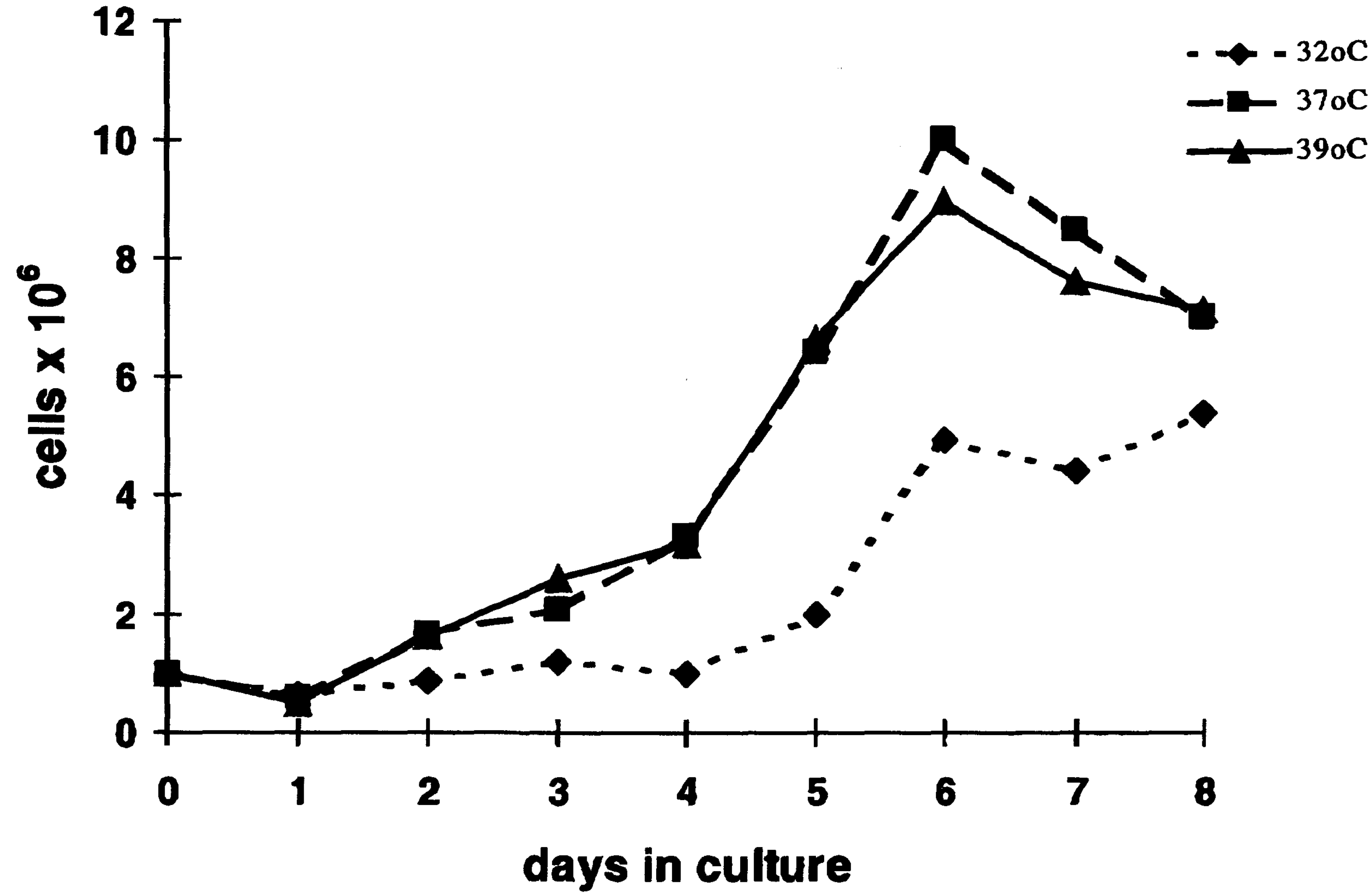

**Figure 3: DBP levels in PER.C6 cells transfected with pcDNA3, pcDNA3wtE2A or pcDNA3ts125E2A**

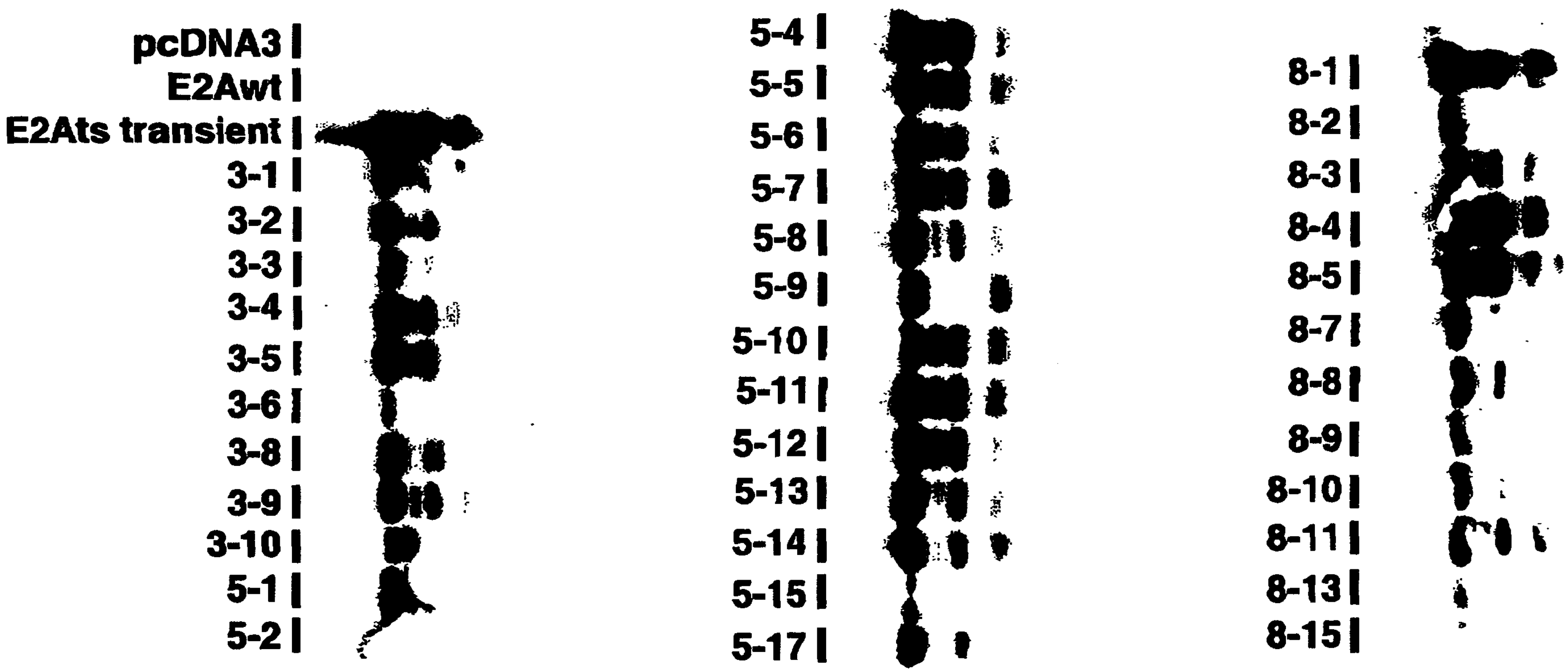

Figure 4: Suspension growth of PER.C6ts125E2A C5-9

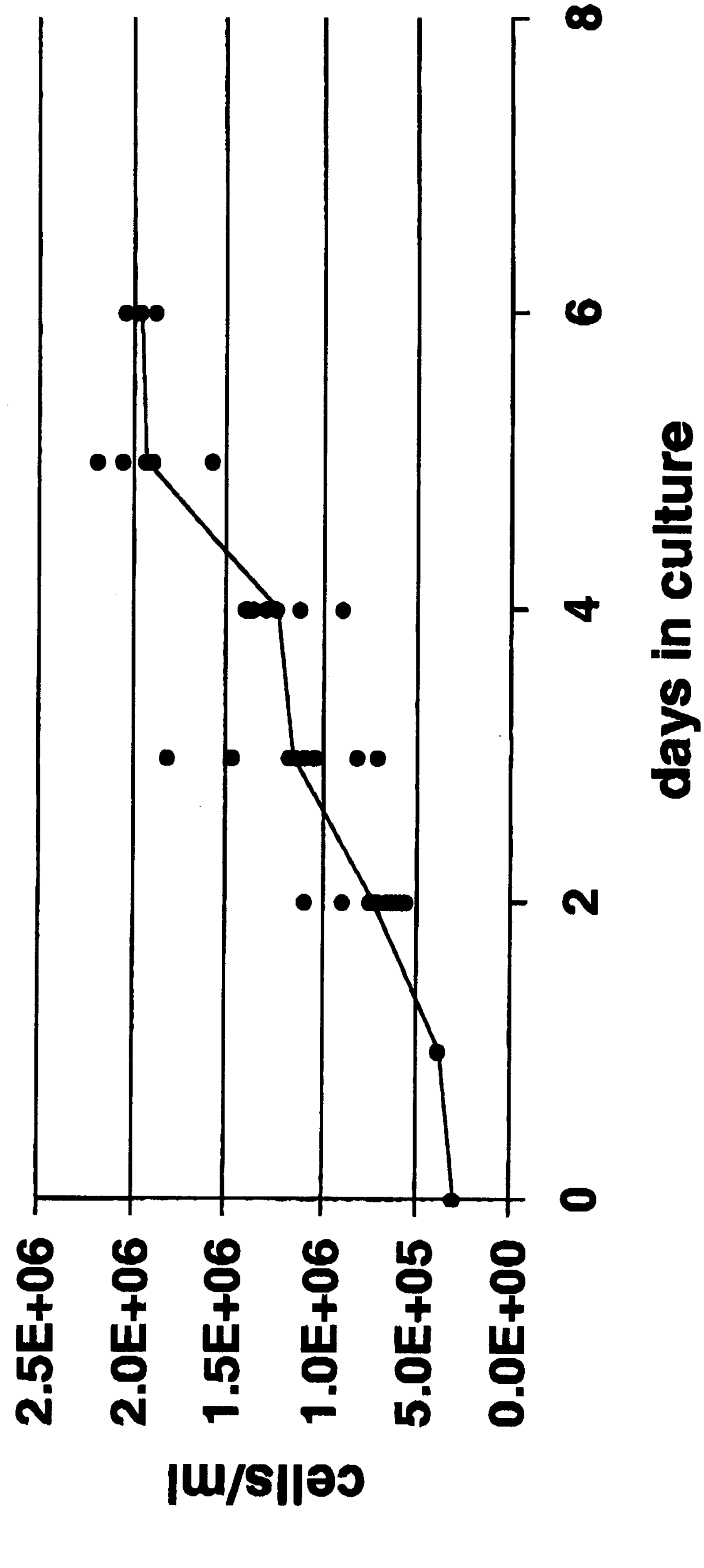

Figure 5: Growth curve PER.C6 and PER.C6tsE2A

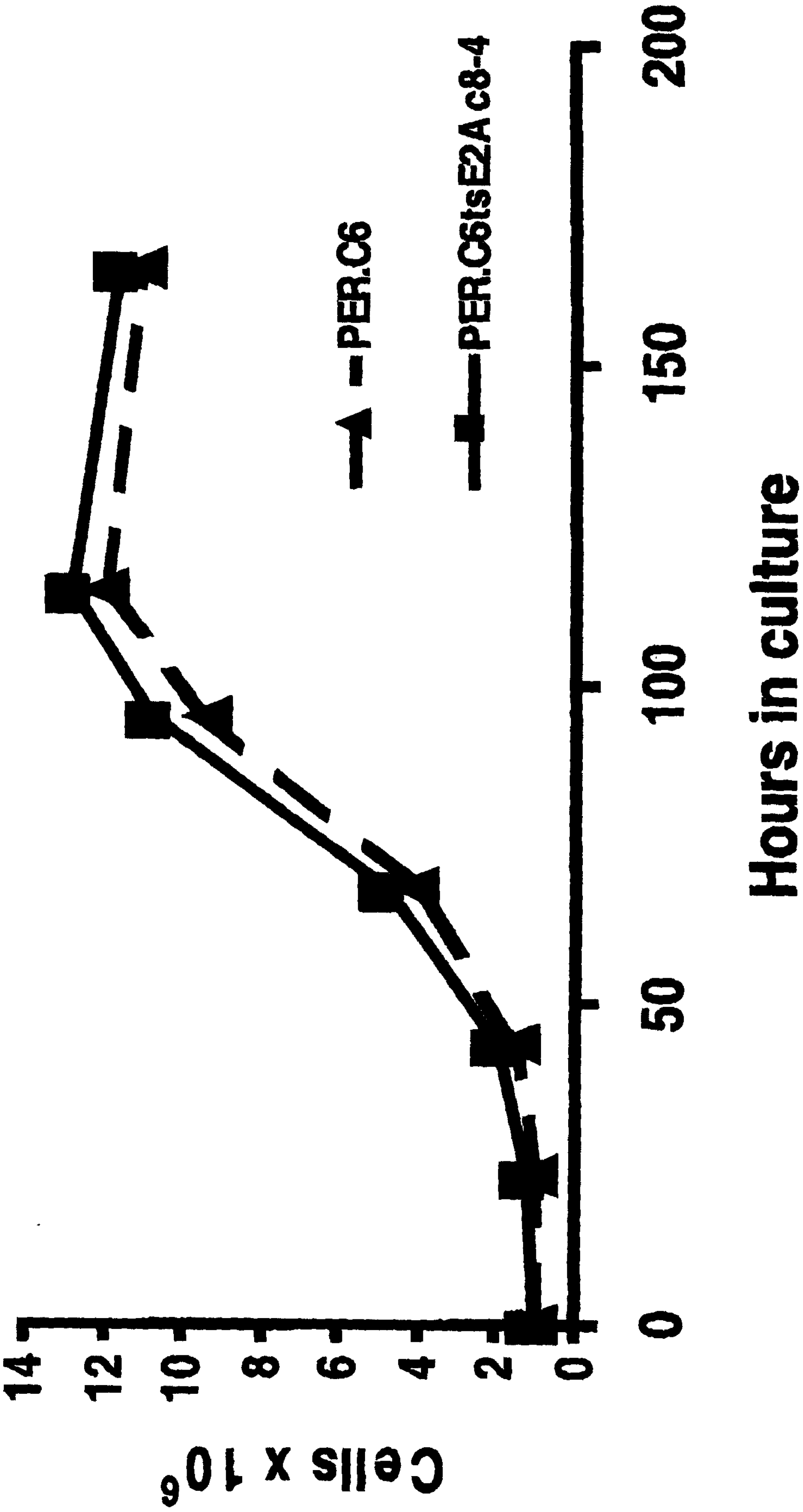

Figure 6: Stability of PER.C6ts125E2A

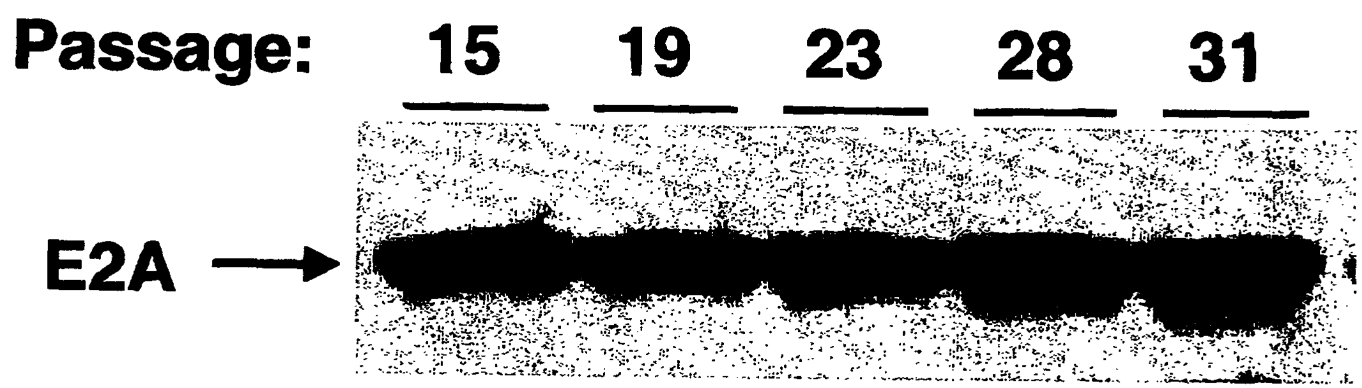

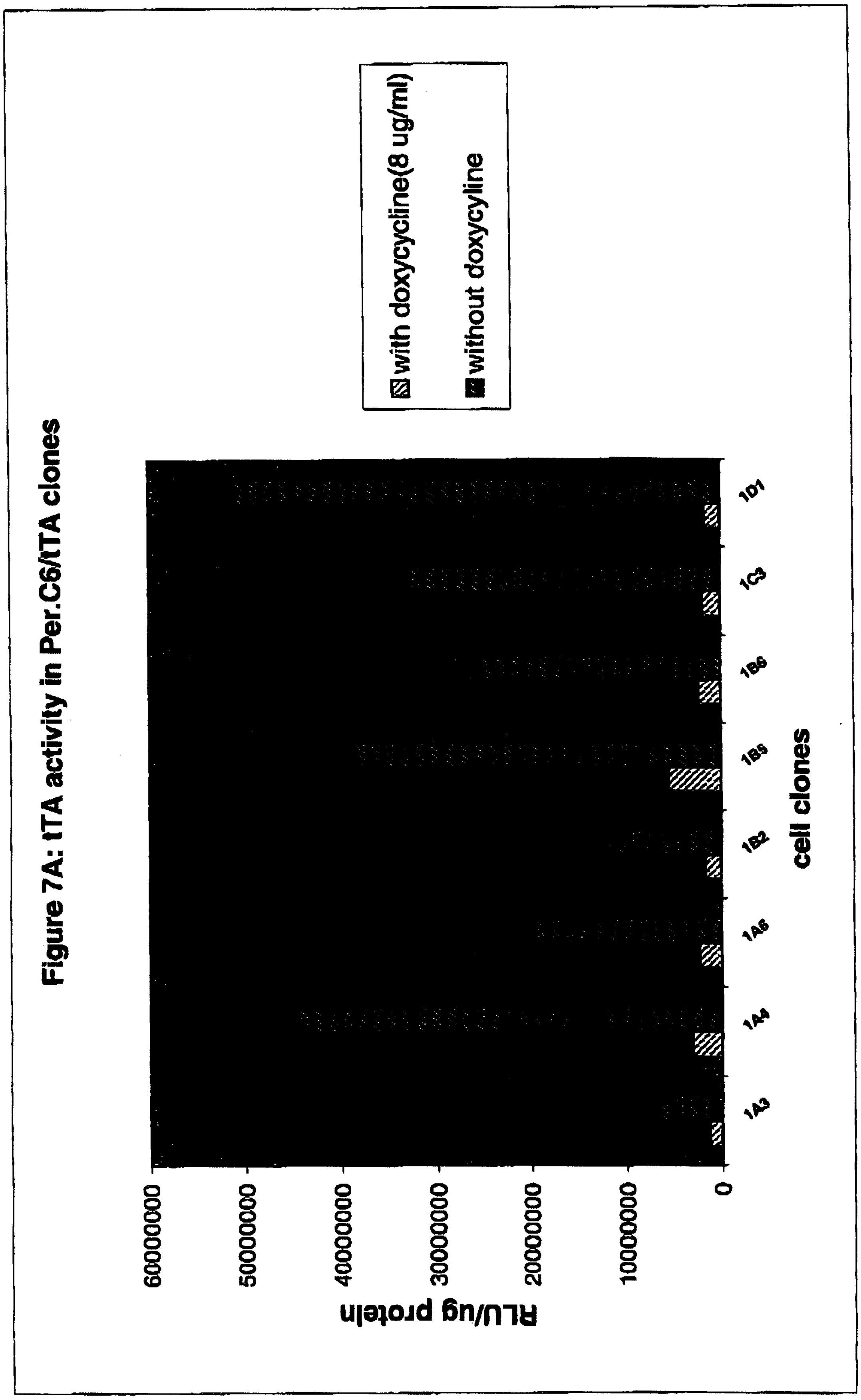


Figure 7A: tTA activity in Per.C6/tTA clones

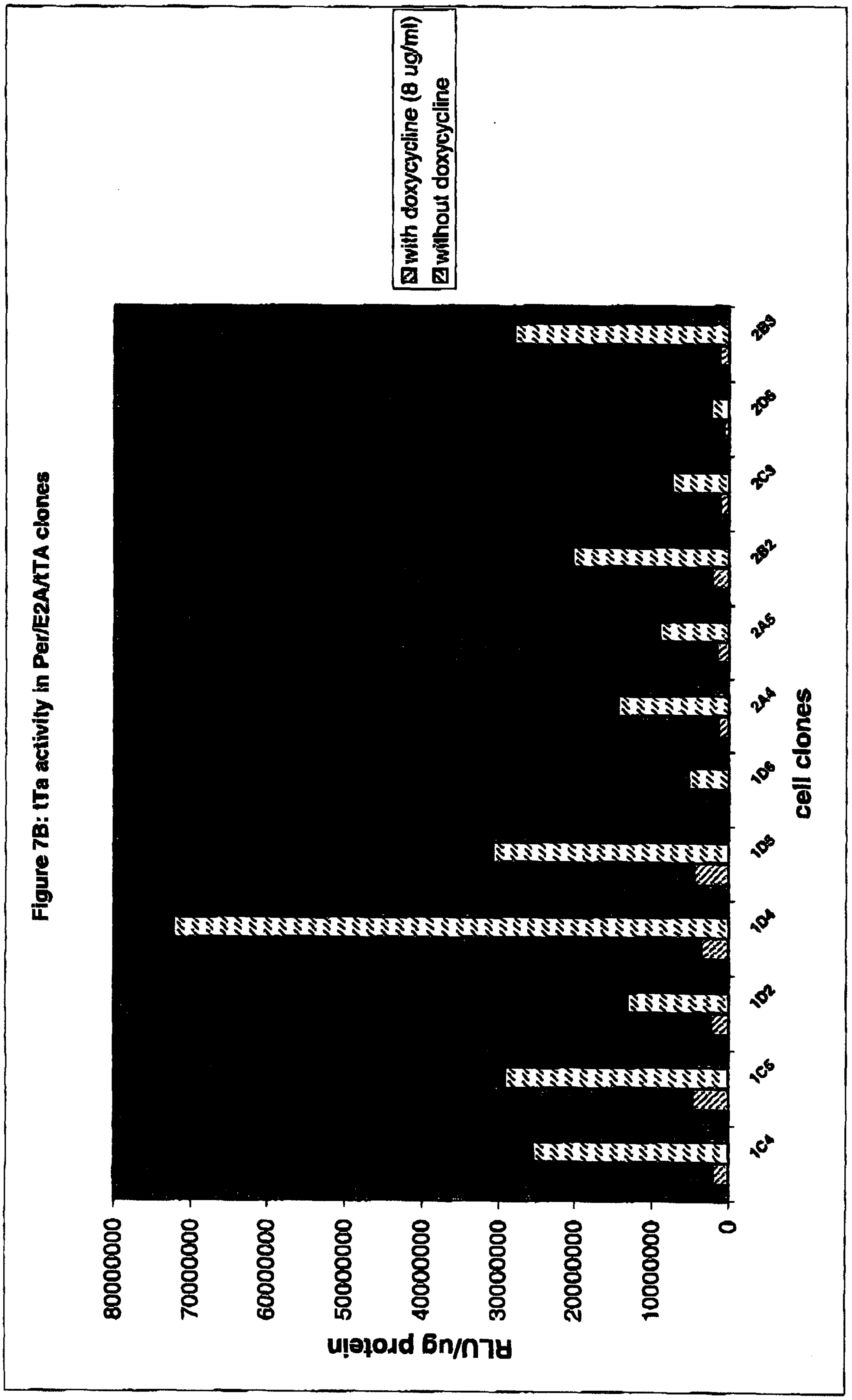

Figure 7B: tTa activity in Per/E2A/tTA clones

Figure 8. Residual Expression of E4
(western and Southern blot analysis)

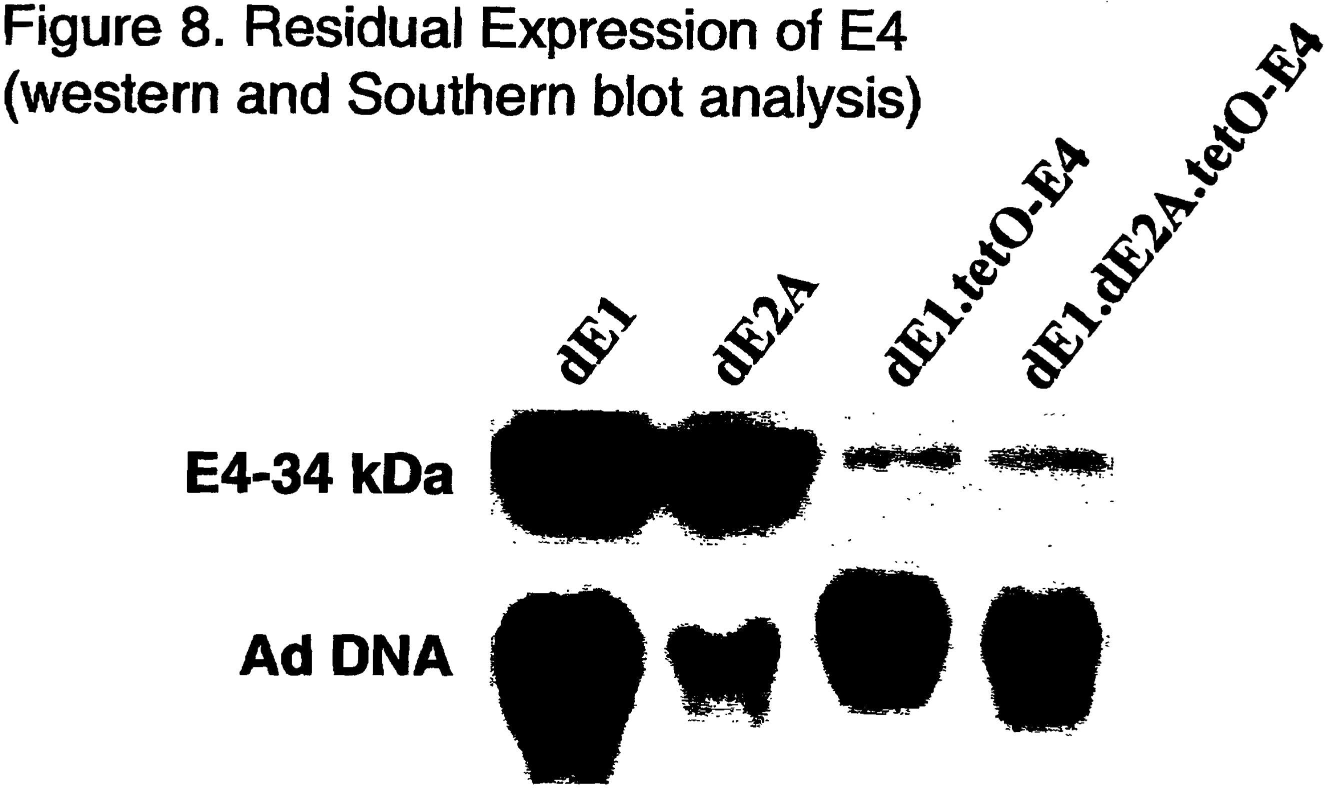


- A549 cells, MOI = 1000 vp/cell
- Harvested at 30 h p.i.

Figure 9. Residual Expression of E4: triple analysis (western analysis)

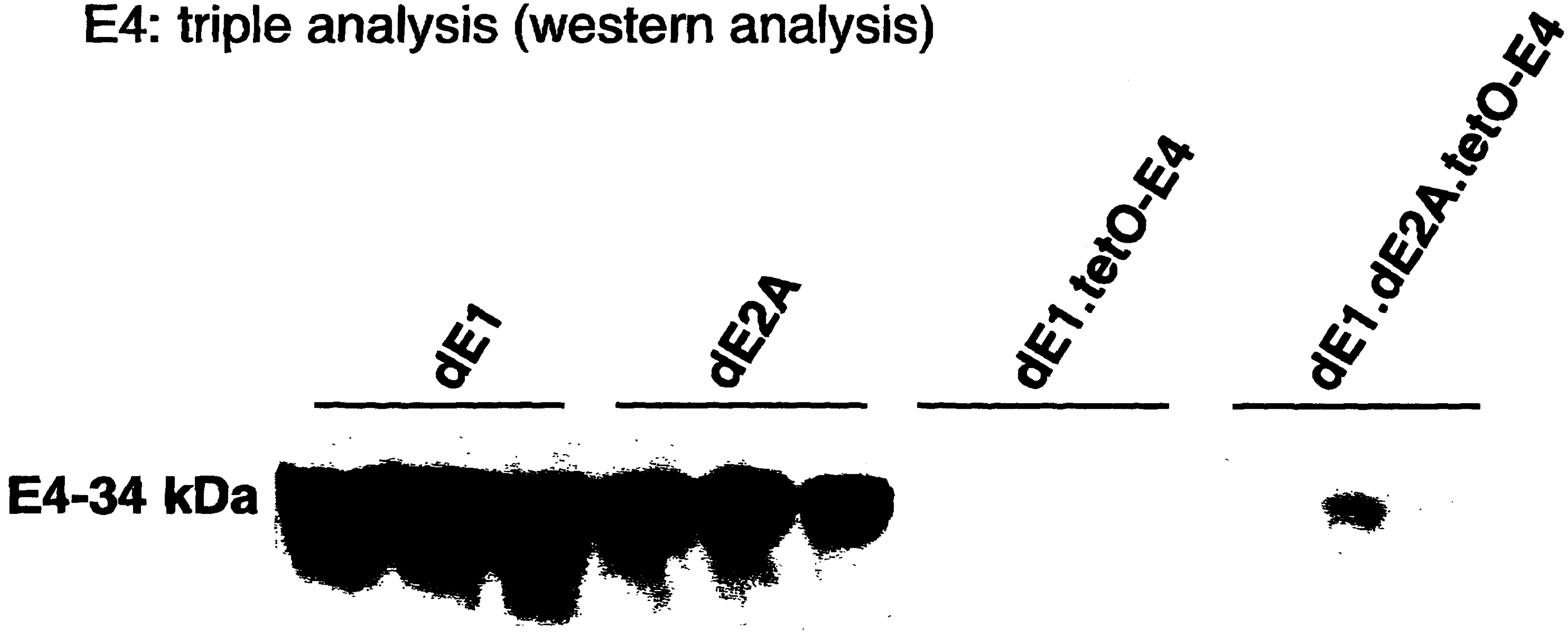


**A549 cells, MOI = 1000 vp/cell**

**harvested at 30 h p.i.**

Figure 10. Detection of vector DNA: triple (Southern) analysis

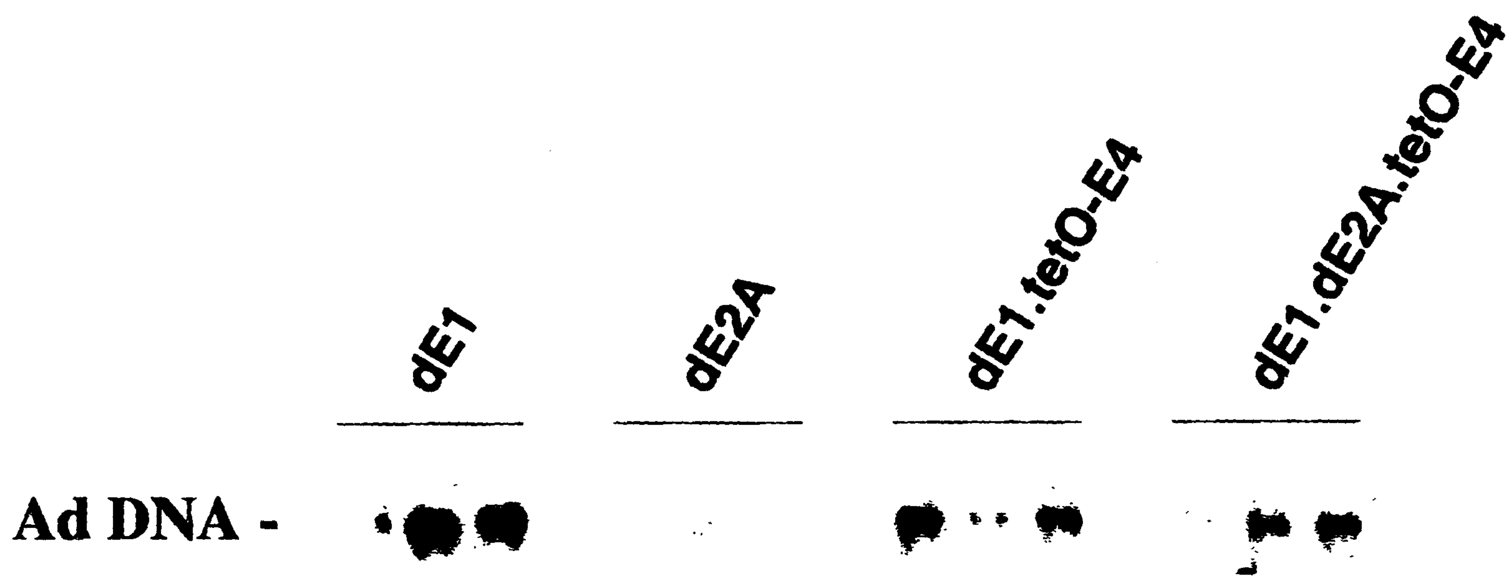


A549 cells, MOI = 1000 vp/cell

harvested at 30 h p.i.

Figure 11. Residual expression of E2A

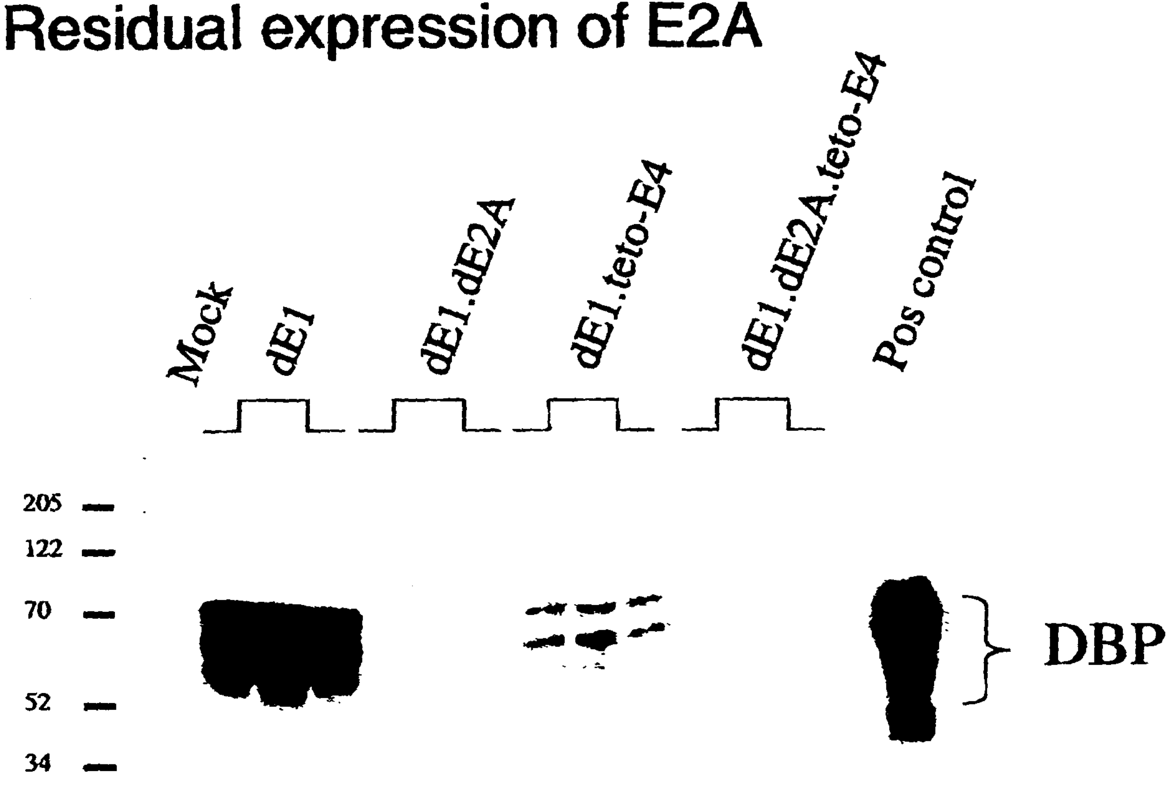


• A549 cells, MOI = 1000 vp/cell

• Harvested at 30 h p.i.

Figure 12. Residual Expression of pTP

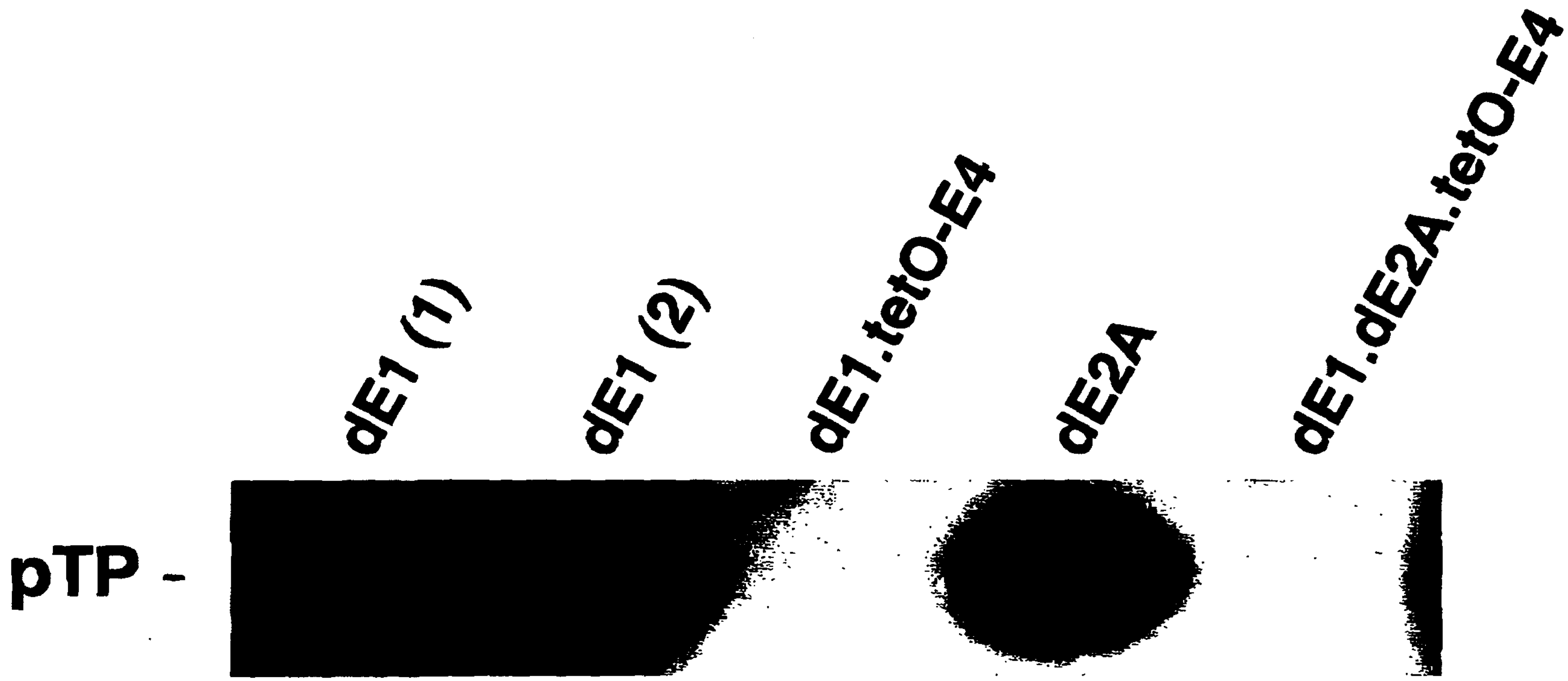


- A549 cells, MOI = 1000 vp/cell
- Harvested at 30 h p.i.

Figure 13. Residual Expression of Late Genes: triple analysis of fiber expression

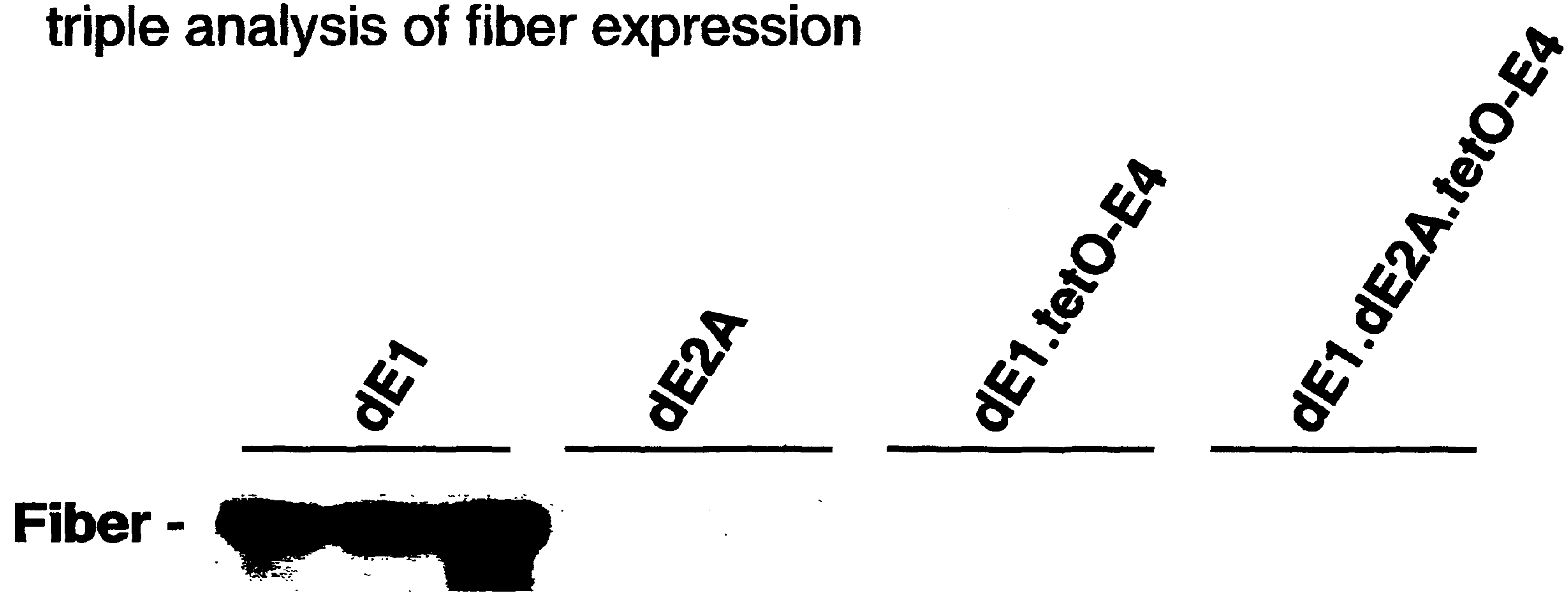


A549 cells, MOI = 1000 vp/cell

harvested at 72 h p.i.

Figure 14. CMV Promoter Activity: A549 cells

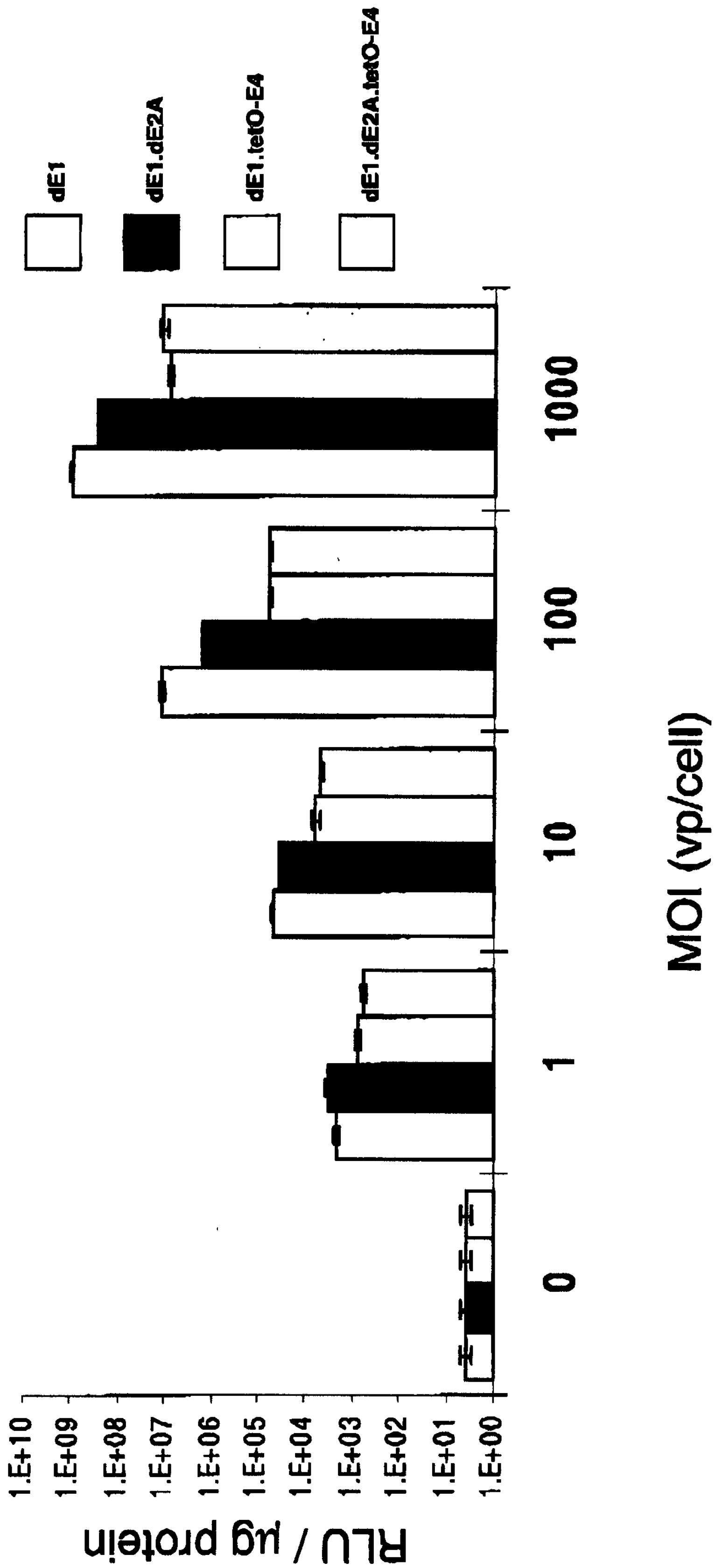

Figure 15. CMV Promoter Activity: primary human endothelial cells

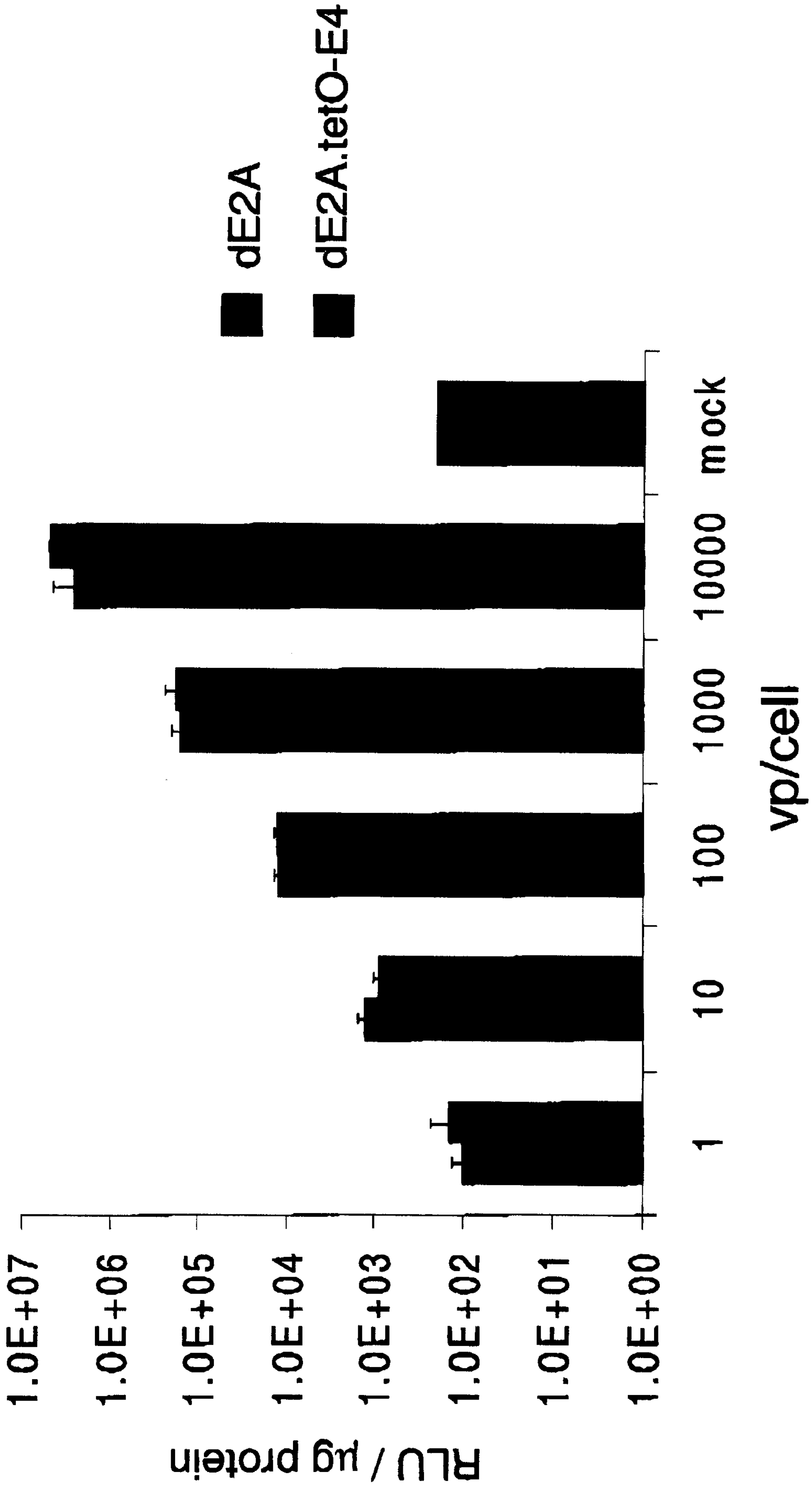

Figure 16. Longevity of expression in A549 cells

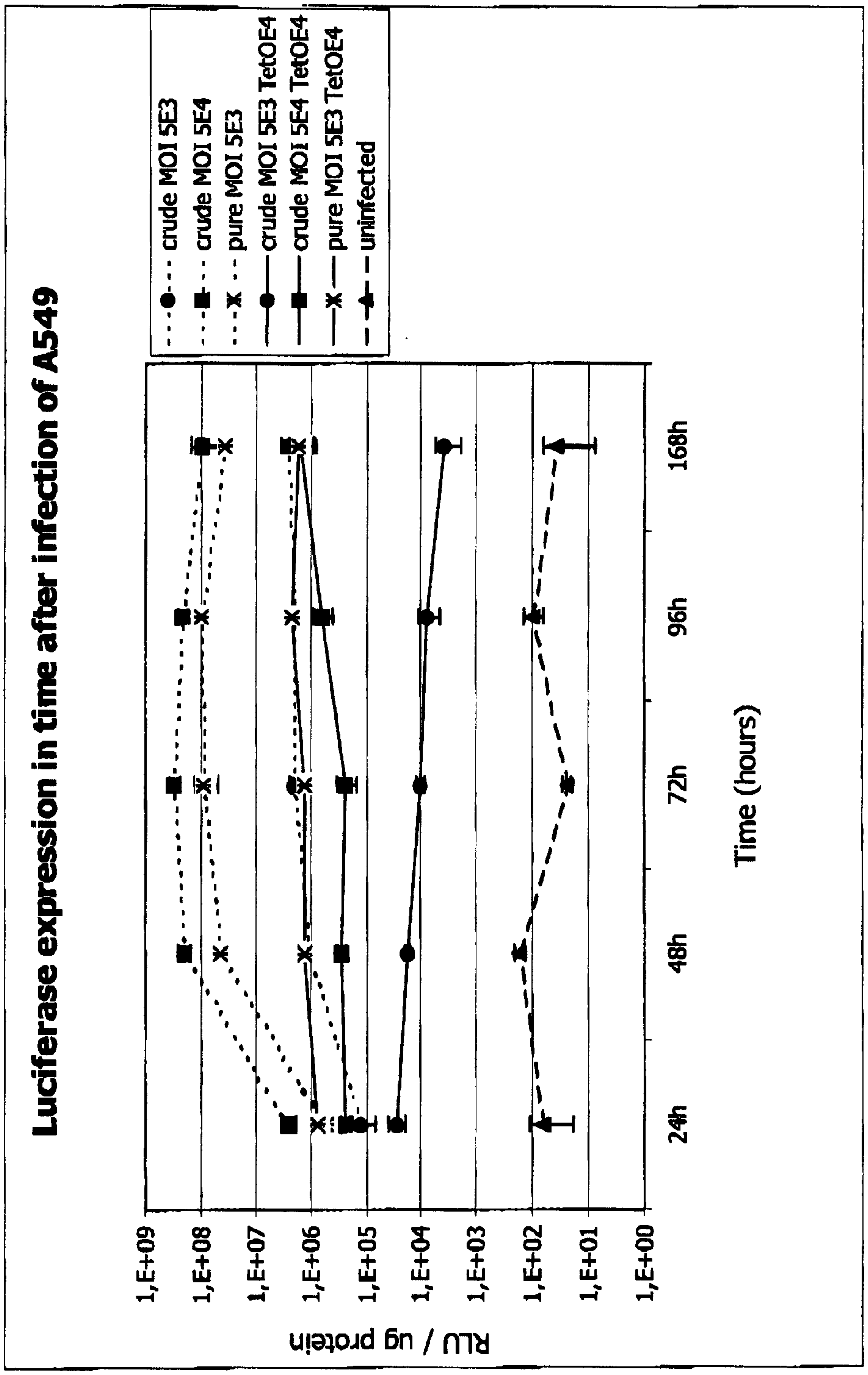

# Figure 17. Longevity of expression in HepG2 cells

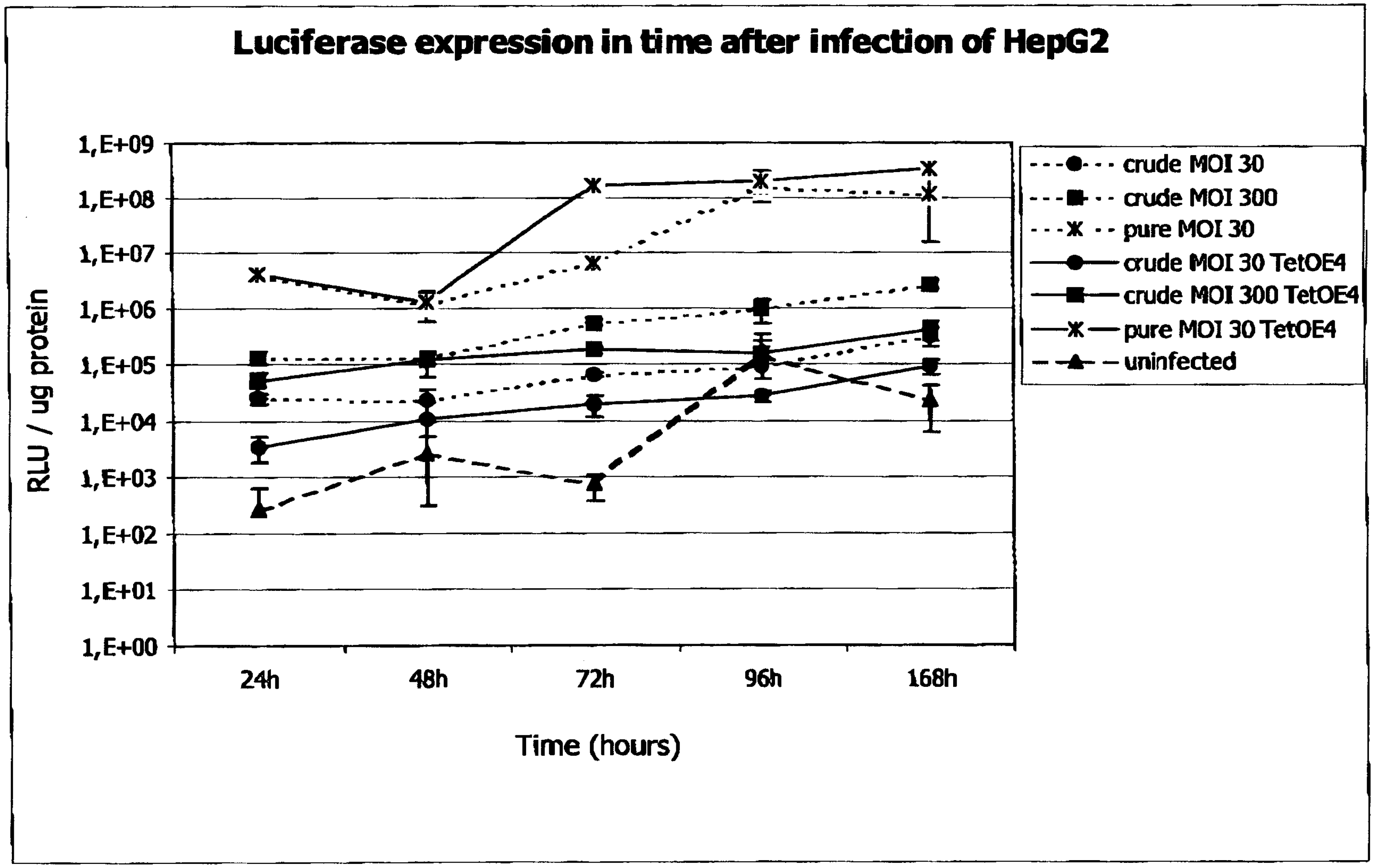

# Figure 18. Longevity of expression in MCF-7 cells

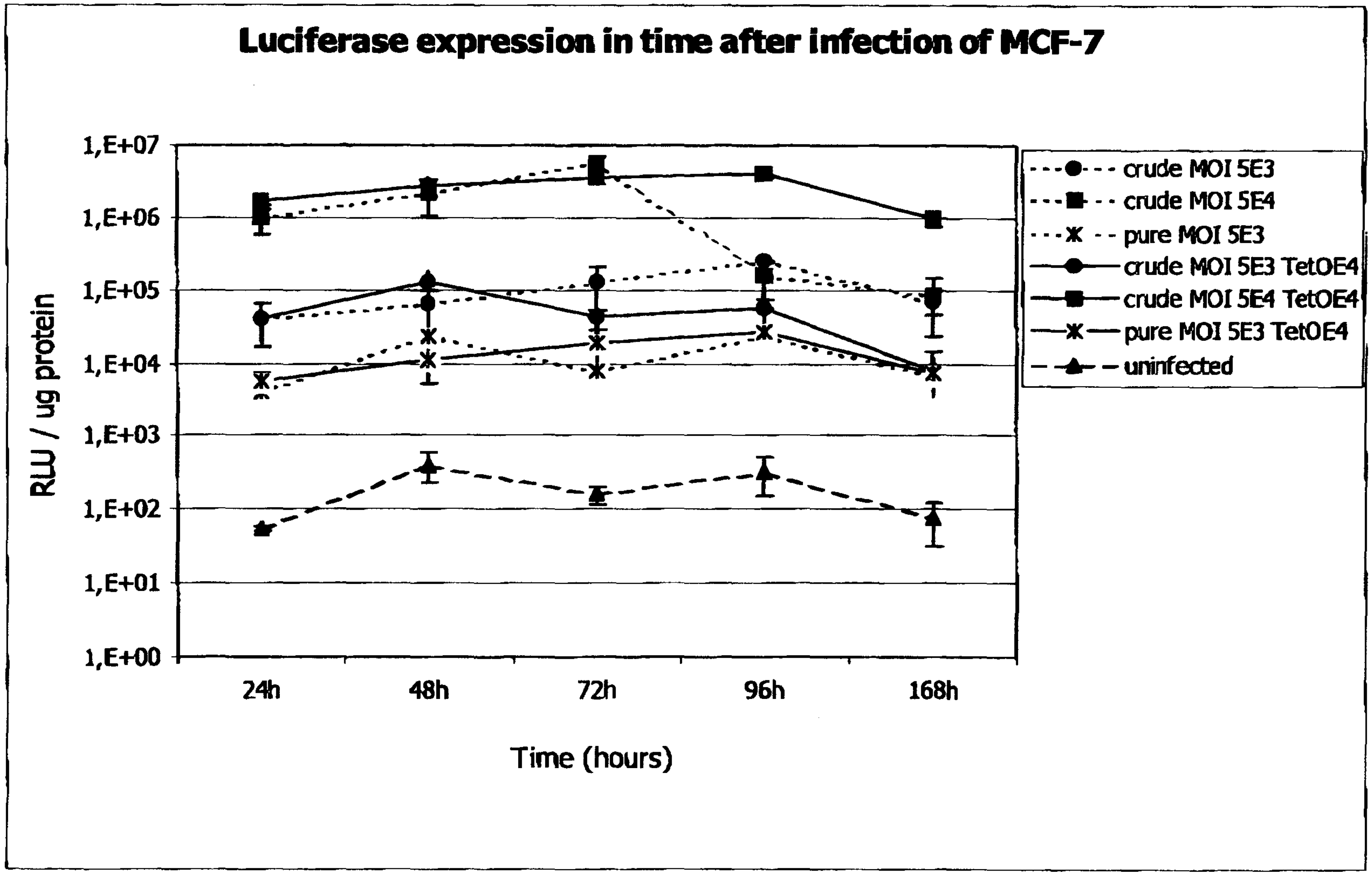

Figure 19. Vector Genome Analysis

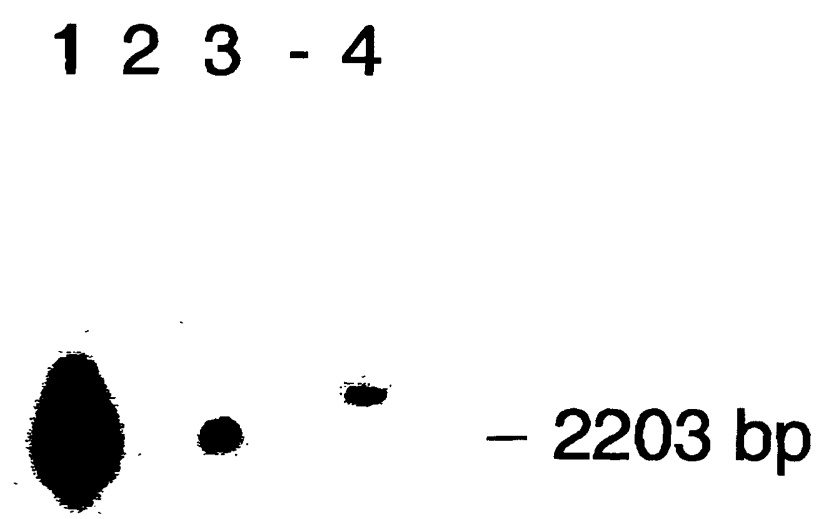


1) Ad.dE2A.tetO-E4 x PstI
2) Ad.dE2A x PstI
3) pWE/Ad.AflII-rITR.tetO-E4 x PacI x PstI
4) pNEB-PaSe.tetO x HindIII

Figure 20

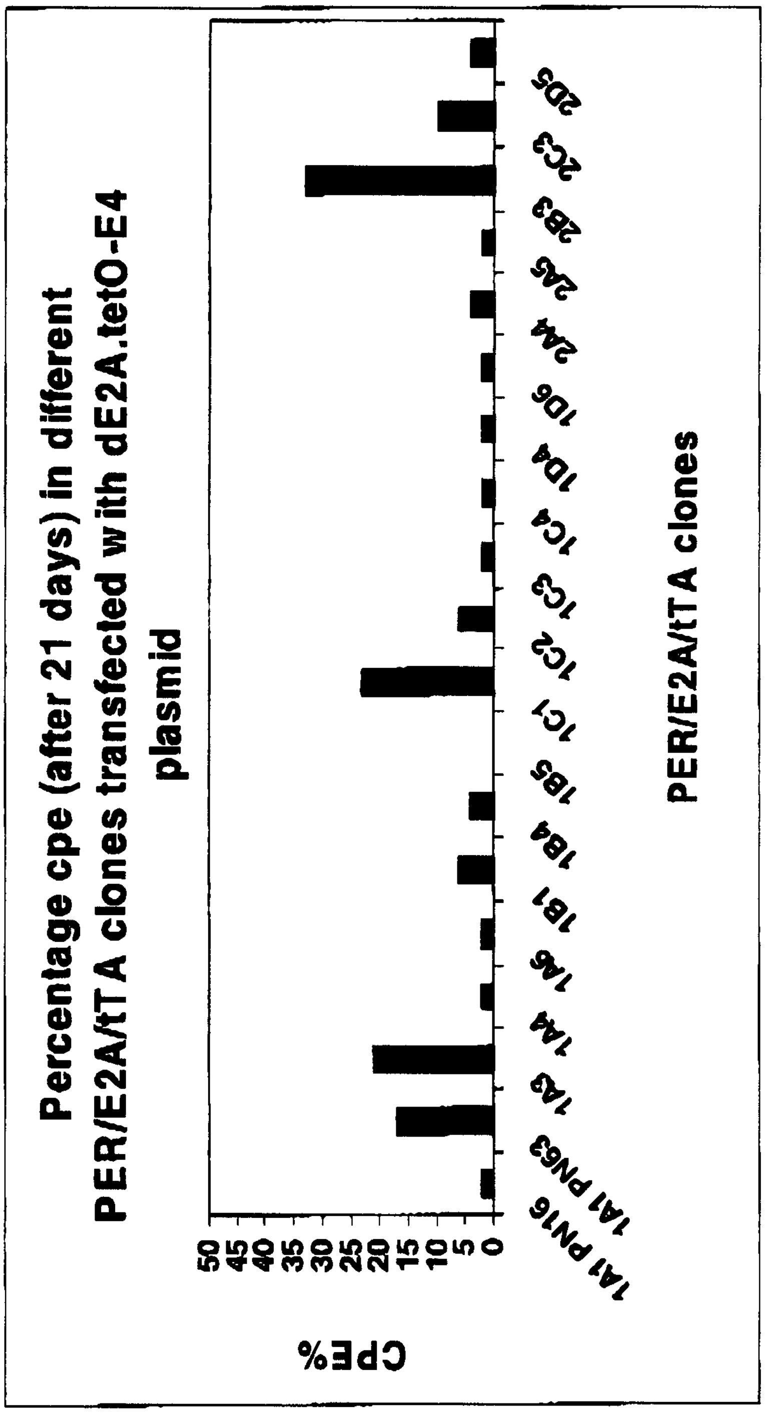
Percentage cpe (after 21 days) in different PER/E2A/tTA clones transfected with dE2A.tetO-E4 plasmid
CPE%
50
45
40
35
30
25
20
15
10
5
0
1A1 PN16
1A1 PN63
1A3
1A4
1A5
1B1
1B4
1B5
1C1
1C2
1C3
1C4
1D4
1D6
2A4
2A5
2B3
2C3
2D5
PER/E2A/tTA clones

Figure 21

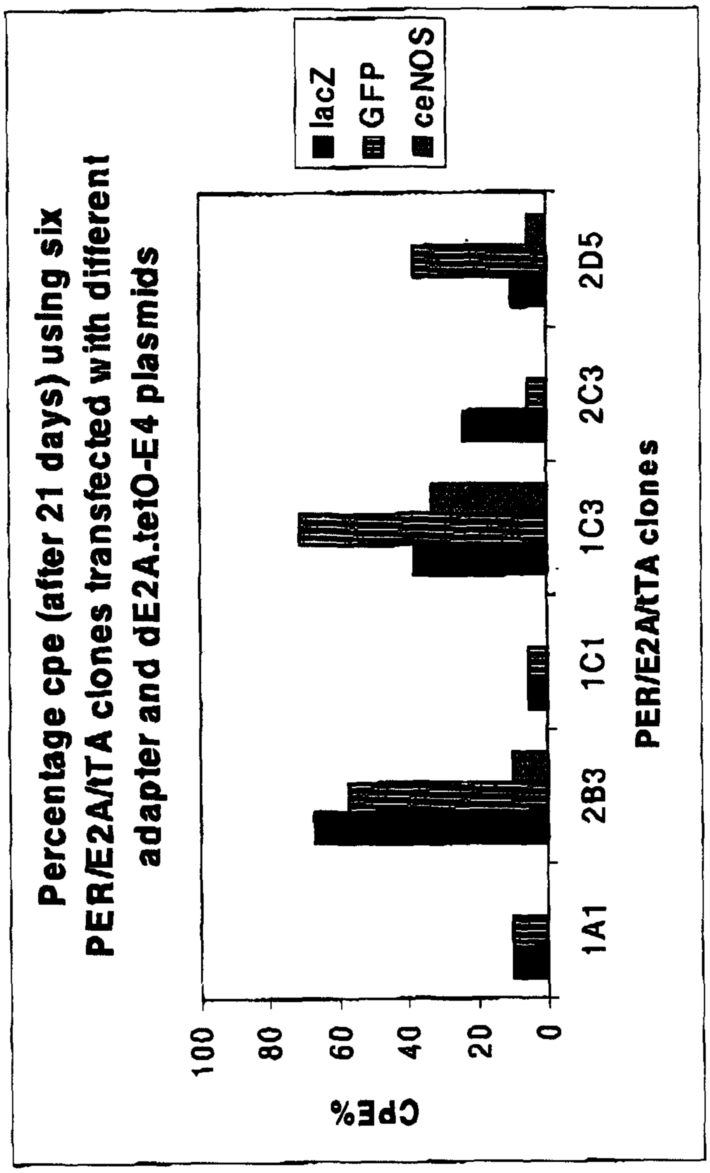
Percentage cpe (after 21 days) using six PER/E2A/tTA clones transfected with different adapter and dE2A.tetO-E4 plasmids
CPE%
100
80
60
40
20
0
1A1
2B3
1C1
1C3
2C3
2D5
PER/E2A/tTA clones
lacZ
GFP
ceNOS

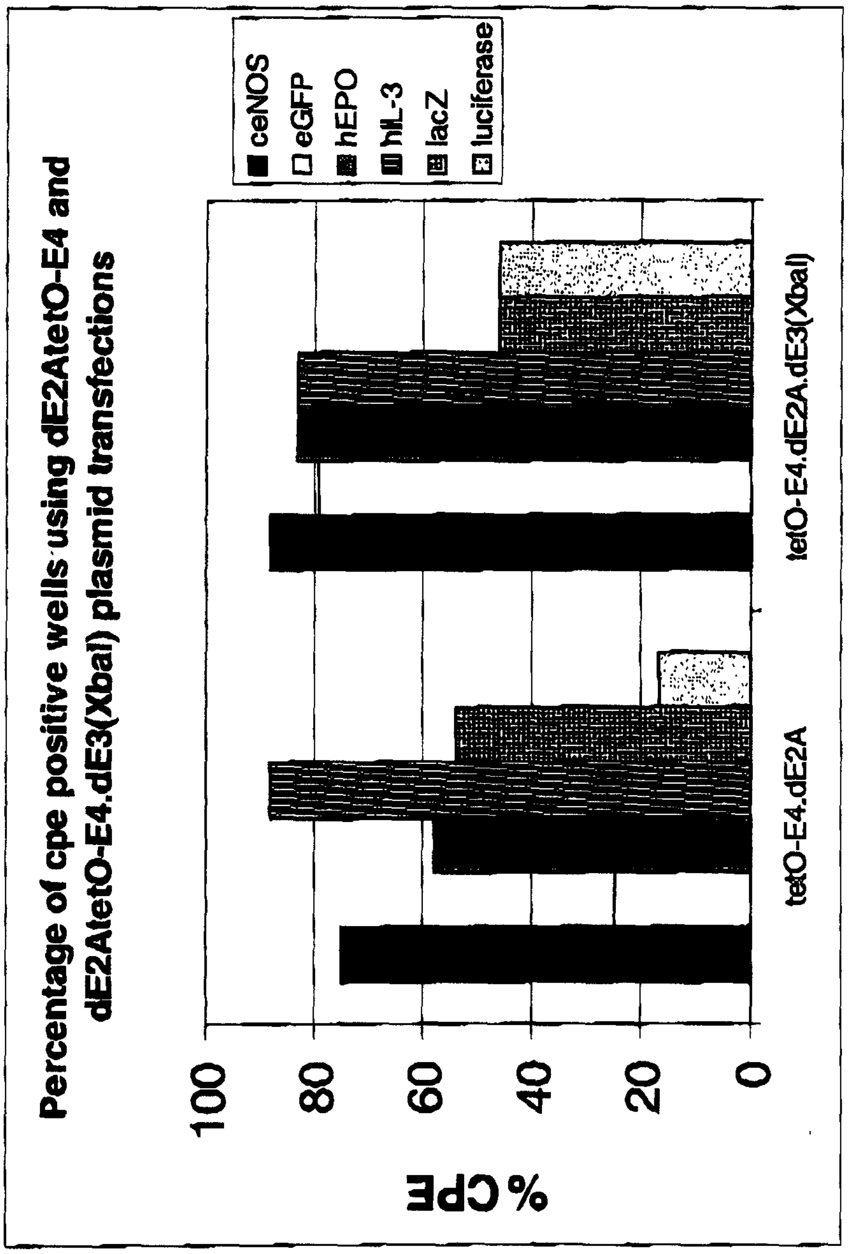
Percentage of cpe positive wells using dE2AtetO-E4 and dE2AtetO-E4.dE3(Xbal) plasmid transfections
% CPE
100
80
60
40
20
0
tetO-E4.dE2A
tetO-E4.dE2A.dE3(Xbal)
ceNOS
eGFP
hEPO
hIL-3
lacZ
luciferase

Figure 22

Figure 23

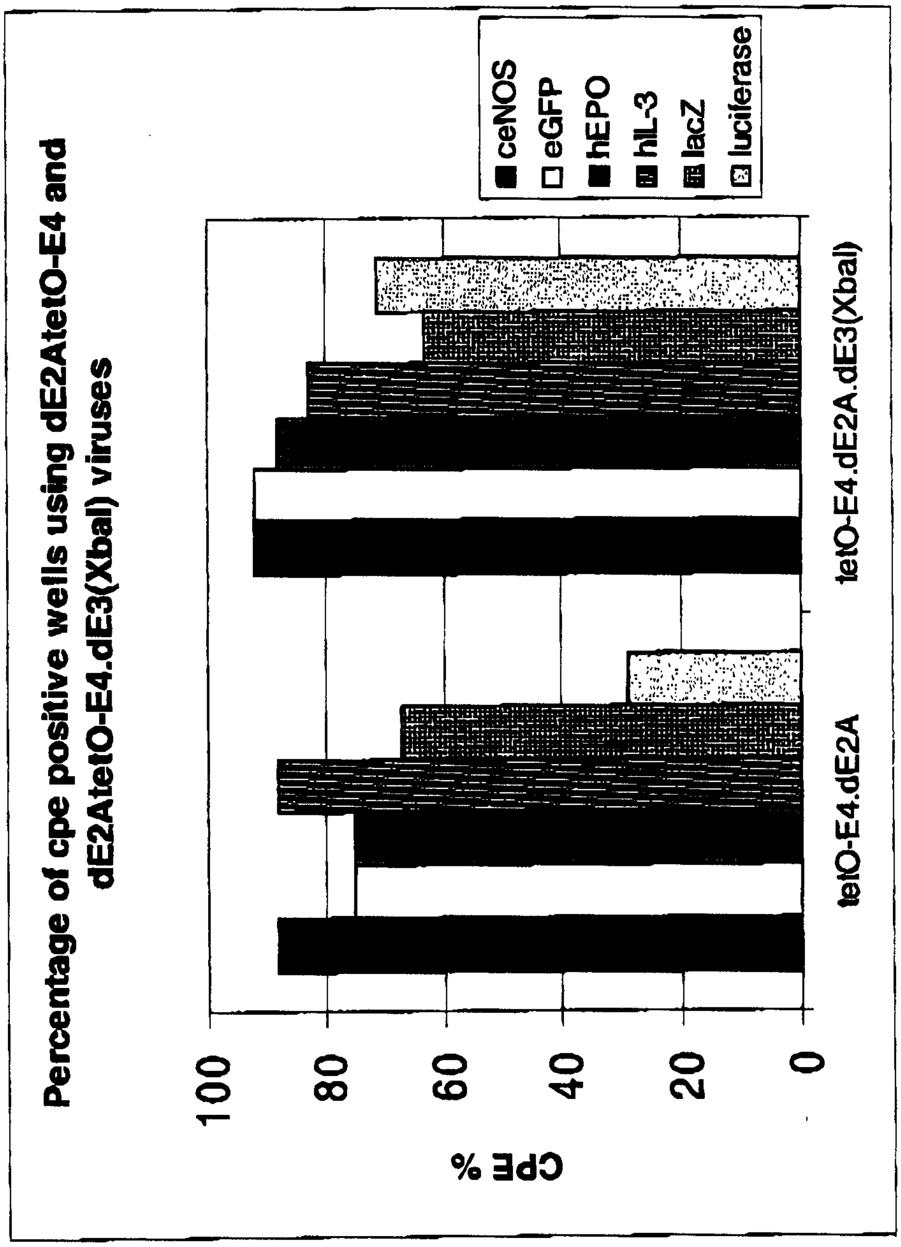
Percentage of cpe positive wells using dE2AtetO-E4 and dE2AtetO-E4.dE3(Xbal) viruses
CPE %
100
80
60
40
20
0
tetO-E4.dE2A
tetO-E4.dE2A.dE3(Xbal)
ceNOS
eGFP
hEPO
hIL-3
lacZ
luciferase

Figure 24

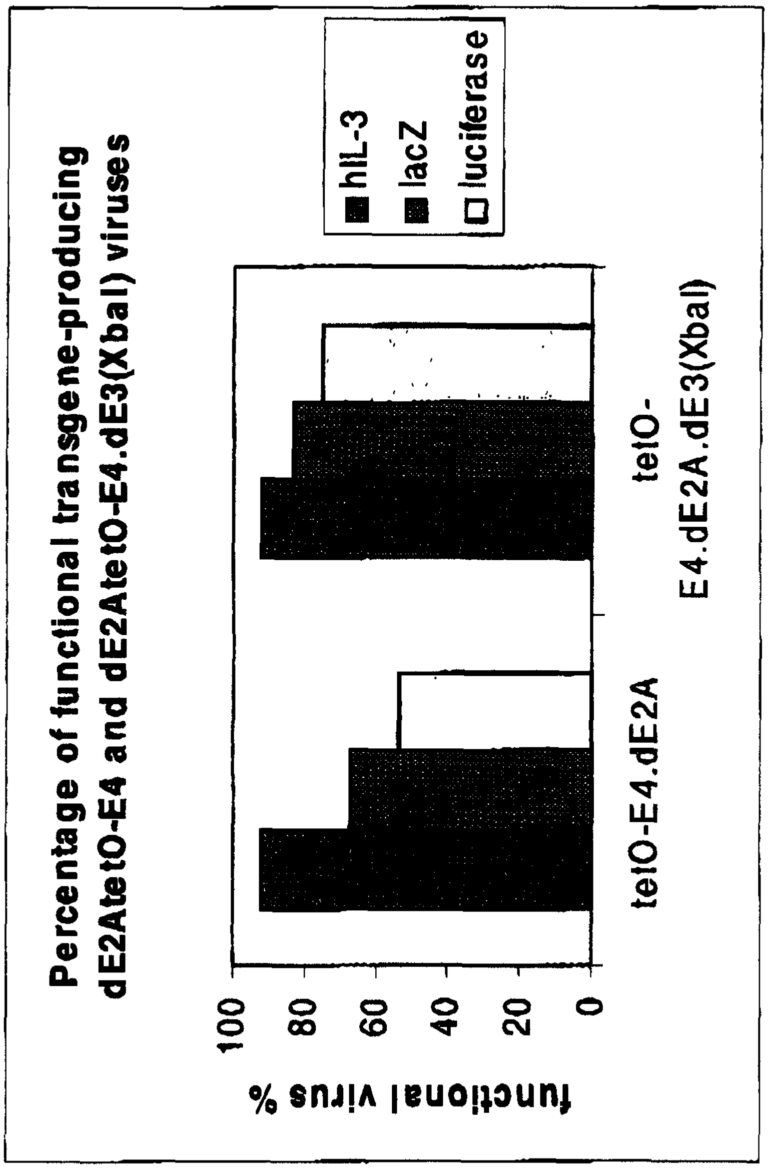
Percentage of functional transgene-producing dE2AtetO-E4 and dE2AtetO-E4.dE3(Xbal) viruses
functional virus %
100
80
60
40
20
0
tetO-E4.dE2A
tetO-E4.dE2A.dE3(Xbal)
hIL-3
lacZ
luciferase

European Patent Office

# EUROPEAN SEARCH REPORT

Application Number
EP 00 20 3121

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br>Y | WO 98 21350 A (ROTH JACK A ;FANG BINGLIANG (US); UNIV TEXAS (US))<br>22 May 1998 (1998-05-22)<br>* the whole document *<br>--- | 1,2,4-7,<br>9-17,<br>20-22<br>18,19 | C12N15/86<br>C12N5/10<br>C12N7/01<br>A61K48/00 |
| Y | WO 97 45550 A (BAXTER INT ;ZHANG WEI WEI (US); ALEMANY RAMON (US); DAI YIFAN (US))<br>4 December 1997 (1997-12-04)<br>* the whole document *<br>--- | 18 | |
| Y | WO 98 22609 A (ARMENTANO DONNA E ;GREGORY RICHARD J (US); GENZYME CORP (US); SMIT)<br>28 May 1998 (1998-05-28)<br>* the whole document *<br>--- | 19 | |
| D,X | FANG B. ET AL.: "DIMINISHING ADENOVIRUS GENE EXPRESSION AND VIRAL REPLICATION BY PROMOTER REPLACEMENT"<br>GENE,<br>vol. 71, 1 June 1997 (1997-06-01), pages 4798-4803, XP002062030<br>ISSN: 0378-1119<br>* the whole document *<br>--- | 1,2,4,7,<br>9-17,<br>20-22 | TECHNICAL FIELDS SEARCHED (Int.Cl.7)<br>C12N<br>A61K |
| X | JI L. ET AL.: " Reduced toxicity, attenuated immunogenicity and efficient mediation of human p53 gene expression in vivo by an adenovirus vector with dleted E1-E3 and inactivated E4 by GAL4-TATA promoter replacement."<br>GENE THERAPY,<br>vol. 6, March 1999 (1999-03), pages 393-402, XP000876528<br>* the whole document *<br>---<br>-/-- | 1,2,4,7,<br>9-17,<br>20-22 | |

The present search report has been drawn up for all claims

| Place of search | Date of completion of the search | Examiner |
|---|---|---|
| THE HAGUE | 8 December 2000 | Mandl, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 20 3121

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | RITTNER K. ET AL.: "Conditional repression of the E2 transcription unit in E1-E3-deleted adenovirus vectors is correleated with a strong reduction in viral DNA replication and late gene expression in vitro." JOURNAL OF VIROLOGY, vol. 71, no. 4, April 1997 (1997-04), pages 3307-331, XP002130029 * the whole document * --- | 1-22 | |
| D,A | WO 97 00326 A (FALLAUX FRITS JACOBUS ;UNIV LEIDEN (NL); BOUT ABRAHAM (NL); EB ALE) 3 January 1997 (1997-01-03) * the whole document * ----- | 1-22 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 8 December 2000 | Mandl, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 3121

This annex lists the patent family membersrelating to the patent documents cited in the above-mentioned European search report.
The members are as contained in the European Patent Office EDP file on
The European Patent Office is in no way liable for these particulars which are merely given for the purpose of information.

08-12-2000

| Patent document cited in search report | Publication date | Patent family member(s) | Publication date |
|---|---|---|---|
| WO 9821350 A | 22-05-1998 | AU 5253998 A | 03-06-1998 |
| | | US 6110744 A | 29-08-2000 |
| WO 9745550 A | 04-12-1997 | EP 0954591 A | 10-11-1999 |
| WO 9822609 A | 28-05-1998 | US 5877011 A | 02-03-1999 |
| | | AU 5455298 A | 10-06-1998 |
| | | EP 0946742 A | 06-10-1999 |
| WO 9700326 A | 03-01-1997 | AU 6018296 A | 15-01-1997 |
| | | CA 2222140 A | 03-01-1997 |
| | | EP 0833934 A | 08-04-1998 |
| | | JP 11507552 T | 06-07-1999 |
| | | US 5994128 A | 30-11-1999 |
| | | US 6033908 A | 07-03-2000 |

EPO FORM P0459

For more details about this annex : see Official Journal of the European Patent Office, No. 12/82